# EUROPEAN PATENT APPLICATION

(11) **EP 4 751 775 A1**
(43) Date of publication of application: **03.06.2026**
(21) Application number: 24215924.2
(22) Date of filing: 27.11.2024
(51) Int. Cl.: A61P 31/12, C07D 271/04, C07D 413/10, C07D 413/14, C07D 487/04, A61K 31/4245

(54) **N-CARBAMOYL SYDNONE IMINES FOR USE IN THE TREATMENT OF FLAVIVIRUS INFECTIONS**

(71) Applicant: IRBM S.P.A., 00071 Pomezia (RM) (IT); C.N.C.C.S. S.c.a.r.l. Collezione Nazionale Dei Composti Chimici e Centro Screening, 00071 Pomezia (RM) (IT)
(72) Inventor: Torrente De Haro, Esther, 00071 Pomezia (RM) (IT); Paonessa, Giacomo, 00071 Pomezia (RM) (IT); Ontoria Ontoria, Jesus Maria, 00071 Pomezia (RM) (IT); Montalbetti, Christian, 00071 Pomezia (RM) (IT); Corio, Alessandra, 00071 Pomezia (RM) (IT); Ievoli, Giovanni, 00071 Pomezia (RM) (IT)
(74) Representative: Pace Napoleone, Maria

(57) **Abstract**

The present invention pertains to compounds that inhibit Flavivirus NS2B-NS3 proteases and are potent and selective inhibitors of the West Nile virus and Zika virus. The compounds of the invention can be used alone or in combination with other agents to treat infections caused by these viruses. Additionally, the invention relates to pharmaceutical compositions containing these compounds.

## Description

### FIELD OF THE INVENTION

The present invention pertains to compounds that inhibit Flavivirus NS2B-NS3 proteases and prevent the replication of flaviviruses in cells. Specifically, these compounds are potent and selective inhibitors of the West Nile virus and Zika virus and can be used alone or in combination with other agents to treat infections caused by these viruses. Additionally, the invention relates to pharmaceutical compositions containing these compounds.

### BACKGROUND

The Flavivirus genus of the Flaviviridae family includes more than 70 related arthropodbome viruses. The most common and representative members are the dengue virus (DENV) with four serotypes (DENV-1, -2, -3, and -4), Zika (ZIKV), West Nile (WNV), Japanese-encephalitis (JEV), Yellow Fever (YFV), and tick-borne encephalitis (TBEV) viruses. These are the causative agents for viral haemorrhagic fever and encephalitis in human beings. These viruses are transmitted primarily by *Aedes* mosquitos. Infections with flaviviruses are a continuing public health threat. There are no approved drugs for an infection caused by West Nile virus (West Nile fever), and physicians typically recommend intensive support therapy, which may involve hospitalization, intravenous fluids, use of a ventilator to assist breathing, medications to control seizures, brain swelling, nausea and vomiting, as well as the use of antibiotics to prevent secondary bacterial infections. The medical state of the art is similar for Zika virus disease. There is no vaccine or specific therapeutic treatment available. The focus is on relieving symptoms, including rest, rehydration and acetaminophen for fever and pain.

The need for Flavivirus treatments is increasing as Flaviviruses are expected to continue spreading into uninfected areas of the world and mutate under drug pressure. The medical need is particularly strong for a safe, effective, and well-tolerated anti-viral treatment, as higher viremia levels are associated with more severe disease. Further, additional treatments are necessary due to the anticipated need for combination therapies to avoid drug resistance.

The Flavivirus genome consists of a positive-sense single-stranded RNA which is ~11 kb in length, consisting of a single long open reading frame (ORF). The single ORF encodes a polyprotein that is further processed by proteases from the host and the viral NS2B-NS3 complex. The flavivirus protease is highly conserved and essential for virus replication. The viral polyprotein is processed to three structural proteins (Capsid, pr-Membrane, and Envelope) and seven non-structural (NS) proteins (NS1, NS2A, NS2B, NS3, NS4A, NS4B, and NS5). Three structural proteins form the virus shell, whereas seven NS proteins participate in the membrane-bound replication complex. Among these NS proteins, only NS3 and NS5 bear enzymatic function (Pathogens 2022, 11 293). Flaviviral replication depends on the NS3 protease, which is a 69 kDa protein with two domains that have different enzymatic functions: a trypsin-like serine protease domain located within the *N*-terminal region; while the C-terminal region has the activities of an RNA-helicase and an RNA-stimulated NTPase. Moreover, it is only entirely activated when associated with its cofactor, namely NS2B; forming an enzymatic complex, NS2B-NS3. Considering this, NS2B-NS3 proteases represent a valuable target for the development of new antiviral compounds, which act inhibiting their replication complex and leading to the flaviviral death.

However, the success of various drug discovery attempts during the last decade has been limited by the nature of the viral enzyme as well as a lack of robust structural templates. Small-molecule, structurally diverse protease inhibitors have been reported to reach affinities in the lower micromolar range. Peptide-based, substrate-derived compounds are often nanomolar inhibitors, however, with highly compromised drug-likeness. With some exceptions, the antiviral cellular activity of most of the reported compounds has been patchy and insufficient for further development. Recent progress has been made in the elucidation of inhibitor binding using different structural methods. This will hopefully lead to more rational attempts for the identification of various lead compounds that may be successful in cellular assays, animal models and ultimately translated to patients.

The present invention yields a novel series of drug-like, broadly active inhibitors of flavivirus proteases, more specifically of West Nile and Zika viruses proteases provide new treatments and pharmaceutical compositions to treat infections thereof. The compounds of the invention are sydnone imine derivatives having a mesoionic heterocyclic core wherein the proper selection of substituents resulted in potent, selective and brain penetrant WNV and ZIKV NS2B-NS3 protease inhibitors. The compounds of the invention are capable of inhibiting replication of the above viruses in cells.

### SUMMARY OF THE INVENTION

It is therefore an object of the invention a compound of general formula (I): 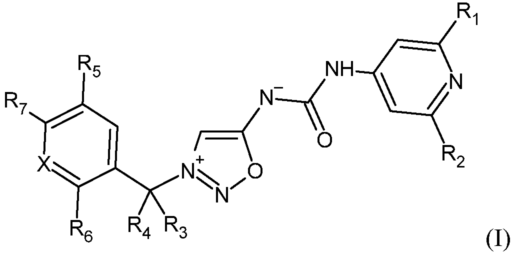 wherein
- XisCorN;
- R₁ is: CF₃, OCF₃ or cyclopropyl optionally substituted with fluorine;
- R₂ is: H, CH₃, OCH₃, Cl, NHCH₃, NHC(=O)R₁₂ wherein R₁₂ is a C₁₋₃alkyl optionally substituted with an aromatic ring ;
- R₃ is H or CH₃ and R₄ is H or CH₂OH; or R₃ and R₄ are linked together to form a spirocyclopropyl ring;
- R₅ and R₆ are each independently selected from: H, CH₃ and Cl;
- R₇ is selected from:
   a) pyridine, optionally substituted with one or more substitents independently selected from C₁₋₃alkyl, C₃₋₆cycloalkyl, C₁₋₃haloalkyl, C₁₋₃alkoxyl, halogen, hydroxy, C₁₋₃haloalkyl further substituted with an hydroxy group, C(O)OC₁₋₃alkyl, C(O)OH, C(O)NHC₁₋₃alkyl, NHC(=O)C₁₋₃alkyl, SO₂NHC₁₋₃alkyl, SO₂C₁₋₃alkyl, CN with the proviso that said optionally substituted pyridine is not 2-methylpyridin-3-yl and 2,4-dimethylpyridine-3-yl; or
   b) pyrazole of formula (II) or (III): 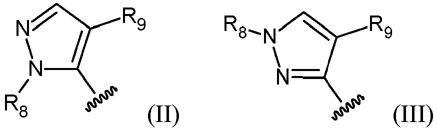 wherein:
      R₈ is selected from H, C₁₋₄alkyl optionally substituted with OH, C₃₋₆cycloalkyl, C₃₋₆heterocycloalkyl and C₁₋₃haloalkyl optionally substituted with OH;
      R₉ is selected from C₁₋₃alkyl, C₃₋₆cycloalkyl, C₁₋₃haloalkyl, C₁₋₃alkoxyl, CN and halogen;
      and wherein if R₉ is CH₃ then R₈ is not CH₃ or an oxetane ring; or
   c) pyridazine optionally substituted with halogen, C₁₋₃alkyl, C₁₋₃alkoxyl, C₁₋₃haloalkyl, C₃₋₆cycloalkyl, CN, with the proviso that said pyridazine is a not 3-methylpyridazin-4-yl; or
   d) pyrimidine of formula (IV): 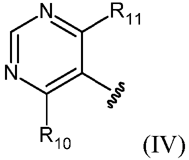 wherein R₁₀ and R₁₁ are each independently selected from C₁₋₃alkyl, C₁₋₃haloalkyl, C₃₋₆cycloalkyl, and wherein if R₁₀ and R₁₁ are both CH₃ then in general formula (I):
      ▪ X is N; and/or
      ▪ R₁ is cyclopropyl; and/or
      ▪ R₂ is CH₃, OCH₃, Cl, NHCH₃; and/or
      ▪ R₅ is Cl;
   e) phenyl or 5 or 6 membered heteroaromatic ring selected from pyrazine, 6-oxo-1,6-dihydropyridine, imidazole and oxazole, each of said ring being optionally substituted with one or more substitents independently selected from C₁₋₃alkyl, C₁₋₃haloalkyl, C₁₋₃alkoxyl, halogen, hydroxy, C₁₋₃haloalkyl further substituted with an hydroxy group, C(O)OC₁₋₃alkyl, C(O)OH, C(O)NHC₁₋₃alkyl; or
or a pharmaceutically acceptable salt, tautomer, solvate, or stereoisomer thereof. Preferably R₁ is CF₃ and R₂ is H.

Preferably R₃ and R₄ are H.

Still preferably R₇ is a pyridine ring selected from: 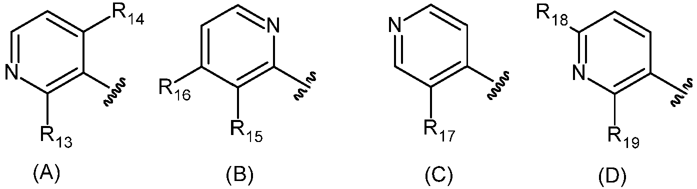 wherein:
- in structure (A) R₁₃ and R₁₄ are independently selected from H, C₁₋₃alkyl, C₁₋₃haloalkyl, C₁₋₃alkoxyl, halogen, and wherein one of R₁₃ and R₁₄ is C₁₋₃haloalkyl, preferably one of R₁₃ and R₁₄ is trifluoromethyl or difluoromethyl;
- in structure (B) R₁₅ and R₁₆ are independently selected from H, C₁₋₃haloalkyl and hydroxy, preferably the C₁₋₃haloalkyl is trifluoromethyl or difluoromethyl; more preferably R₁₅ is trifluoromethyl or difluoromethyl and R₁₆ is H;
- in structure (C) R₁₇ is C₁₋₃haloalkyl, preferably trifluoromethyl or difluoromethyl;
- in structure (D) R₁₈ is selected from C₁₋₃alkyl and C(=O)OH, C(=O)=CH₃ and C(=O)NHCH₃ and R₁₉ is C₁₋₃alkyl or C₁₋₃haloalkyl.

Still preferably R₇ is a pyrazole of formula (II) wherein:
- R₈ is H, methyl, hydroxy-2-methyl-propyl, 1,1-difluoro-2-hydroxyethyl, cyclopropyl, cyclobutyl, oxetane, tetrahydropyrane, tetrahydrofurane; and
- R₉ is methoxy, halogen, trifluoromethyl;
or wherein:
- R₈ is H, hydroxy-2-methyl-propyl, cyclopropyl, cyclobutyl, tetrahydropyrane, tetrahydrofurane; and
- R₉ is methyl.

Still preferably R₇ is a pyrazole of formula (II) wherein R₈ is CH₃ and R₉ is halogen, preferably R₉ is chlorine.

Still preferably R₇ is a pyrazole of formula (III) wherein R₈ is C₁₋₃alkyl or C₁₋₃haloalkyl or and R₉ is halogen.

Still preferably R₇ is selected from: 2-(trifluoromethyl)pyridin-3-yl, 2-(difluoromethyl)pyridin-3-yl, 3-(trifluoromethyl)pyridin-2-yl, 4-chloro-2-(trifluoromethyl)pyridin-3-yl, 4-(trifluoromethyl)pyridin-3-yl, 2-methoxy-4-(trifluoromethyl)pyridin-3-yl, 4-(trifluoromethyl)pyridin-2-yl, 2-trifluoromethylphenyl, 2-(difluoromethyl)-4-methylpyridin-3-yl, 3-(difluoromethyl)pyridin-2-yl, 2-(difluoromethyl)pyridin-3-yl, 3-(trifluoromethyl)pyridin-4-yl, 3-hydroxypyridin-2-yl, 1,1-difluoro-2-hydroxyethyl)pyridin-3-yl, 4-(difluoromethyl)pyridin-3-yl, 3-(difluoromethyl)pyridin-4-yl, 6-(methoxycarbonyl)-2-methylpyridin-3-yl, 6-carboxy-2-methylpyridin-3-yl, 2-methyl-6-(methylcarbamoyl)pyridin-3-yl.

Still preferably R₇ is selected from: 1-cyclopropyl-4-methoxy-1H-pyrazol-5-yl, 4-methoxy-1-methyl-1H-pyrazol-5-yl, 1-cyclopropyl-4-methyl-1H-pyrazol-5-yl, 1-cyclobutyl-4-methyl-1H-pyrazol-5-yl, 1-cyclobutyl-4-methoxy-1H-pyrazol-5-yl, 4-chloro-1-methyl-1H-pyrazol-5-yl, 4-(4-chloro-1-(oxetan-3-yl)-1H-pyrazol-5-yl, 1-methyl-4-(trifluoromethyl)-1H-pyrazol-5-yl, 1-(oxetan-3-yl)-4-(trifluoromethyl)-1H-pyrazol-5-yl, 4-fluoro-1-(oxetan-3-yl)-1H-pyrazol-5-yl, 4-chloro-1-methyl-1H-pyrazol-5-yl, 4-methyl-1-(tetrahydro-2H-pyran-4-yl)-1H-pyrazol-5-yl, 4-chloro-1-methyl-1H-pyrazol-5-yl, 4-chloro-1-methyl-1H-pyrazol-5-yl, 4-chloro-1-(2-hydroxy-2-methylpropyl)-1H-pyrazol-5-yl, 4-chloro-1-(tetrahydro-2H-pyran-4-yl)-1H-pyrazol-5-yl, 4-chloro-1-(tetrahydrofuran-3-yl)-1H-pyrazol-5-yl, 4-chloro-1H-pyrazol-5-yl, 4-chloro-1-(2,2,2-trifluoroethyl)-1H-pyrazol-3-yl.

Still preferably R₇ is selected from: 1,4-dimethyl-1H-imidazol-5-yl, 3-methoxypyridazin-4-yl, 1-cyclobutyl-4-methyl-1H-imidazol-5-yl, 2-trifluoromethylphenyl, 2-difluoromethylphenyl, 4,6-dimethylpyrimidin-5-yl, 4-cyclopropyl-6-(trifluoromethyl)pyrimidin-5-yl, 3-(trifluoromethyl)pyrazin-2-yl.

It is a further object of the invention a compound of general formula (I) selected from:
- (3-(4-(2-(trifluoromethyl)pyridin-3-yl)benzyl)-1,2,3-oxadiazol-3-ium-5-yl)((2-(trifluoromethyl)pyridin-4-yl)carbamoyl)amide
- (3-(4-(1,4-dimethyl-1H-imidazol-5-yl)benzyl)-1,2,3-oxadiazol-3-ium-5-yl)((2-(trifluoromethyl)pyridin-4-yl)carbamoyl)amide
- (3-(4-(1-cyclopropyl-4-methoxy-1H-pyrazol-5-yl)benzyl)-1,2,3-oxadiazol-3-ium-5-yl)((2-(trifluoromethyl)pyridin-4-yl)carbamoyl)amide
- (3-(4-(4-methoxy-1-methyl-1H-pyrazol-5-yl)benzyl)-1,2,3-oxadiazol-3-ium-5-yl)((2-(trifluoromethyl)pyridin-4-yl)carbamoyl)amide
- (3-(4-(1-cyclopropyl-4-methyl-1H-pyrazol-5-yl)benzyl)-1,2,3-oxadiazol-3-ium-5-yl)((2-(trifluoromethyl)pyridin-4-yl)carbamoyl)amide
- (3-(4-(3-methoxypyridazin-4-yl)benzyl)-1,2,3-oxadiazol-3-ium-5-yl)((2-(trifluoromethyl)pyridin-4-yl)carbamoyl)amide
- (3-(4-(1-cyclobutyl-4-methyl-1H-imidazol-5-yl)benzyl)-1,2,3-oxadiazol-3-ium-5-yl)((2-(trifluoromethyl)pyridin-4-yl)carbamoyl)amide
- (3-(4-(1-cyclobutyl-4-methyl-1H-pyrazol-5-yl)benzyl)-1,2,3-oxadiazol-3-ium-5-yl)((2-(trifluoromethyl)pyridin-4-yl)carbamoyl)amide
- (3-(4-(1-cyclobutyl-4-methoxy-1H-pyrazol-5-yl)benzyl)-1,2,3-oxadiazol-3-ium-5-yl)((2-(trifluoromethyl)pyridin-4-yl)carbamoyl)amide
- (3-(4-(2-(difluoromethyl)pyridin-3-yl)benzyl)-1,2,3-oxadiazol-3-ium-5-yl)((2-(trifluoromethyl)pyridin-4-yl)carbamoyl)amide
- (3-(4-(4-chloro-1-methyl-1H-pyrazol-5-yl)benzyl)-1,2,3-oxadiazol-3-ium-5-yl)((2-(trifluoromethyl)pyridin-4-yl)carbamoyl)amide
- (3-(4-(3-(trifluoromethyl)pyridin-2-yl)benzyl)-1,2,3-oxadiazol-3-ium-5-yl)((2-(trifluoromethyl)pyridin-4-yl)carbamoyl)amide
- (3-(4-(4-chloro-2-(trifluoromethyl)pyridin-3-yl)benzyl)-1,2,3-oxadiazol-3-ium-5-yl)((2-(trifluoromethyl)pyridin-4-yl)carbamoyl)amide
- (3-(4-(4-chloro-1-(oxetan-3-yl)-1H-pyrazol-5-yl)benzyl)-1,2,3-oxadiazol-3-ium-5-yl)((2-(trifluoromethyl)pyridin-4-yl)carbamoyl)amide
- (3-(4-(4-(trifluoromethyl)pyridin-3-yl)benzyl)-1,2,3-oxadiazol-3-ium-5-yl)((2-(trifluoromethyl)pyridin-4-yl)carbamoyl)amide
- (3-(4-(1-methyl-4-(trifluoromethyl)-1H-pyrazol-5-yl)benzyl)-1,2,3-oxadiazol-3-ium-5-yl)((2-(trifluoromethyl)pyridin-4-yl)carbamoyl)amide
- (3-(4-(1-(oxetan-3-yl)-4-(trifluoromethyl)-1H-pyrazol-5-yl)benzyl)-1,2,3-oxadiazol-3-ium-5-yl)((2-(trifluoromethyl)pyridin-4-yl)carbamoyl)amide
- (3-(4-(6-oxo-2-(trifluoromethyl)-1,6-dihydropyridin-3-yl)benzyl)-1,2,3-oxadiazol-3-ium-5-yl)((2-(trifluoromethyl)pyridin-4-yl)carbamoyl)amide
- (3-(4-(2-methoxy-4-(trifluoromethyl)pyridin-3-yl)benzyl)-1,2,3-oxadiazol-3-ium-5-yl)((2-(trifluoromethyl)pyridin-4-yl)carbamoyl)amide
- (3-(4-(4-(trifluoromethyl)pyridin-2-yl)benzyl)-1,2,3-oxadiazol-3-ium-5-yl)((2-(trifluoromethyl)pyridin-4-yl)carbamoyl)amide
- (3-((2'-(trifluoromethyl)-[1,1'-biphenyl]-4-yl)methyl)-1,2,3-oxadiazol-3-ium-5-yl)((2-(trifluoromethyl)pyridin-4-yl)carbamoyl)amide
- (3-(4-(4-fluoro-1-(oxetan-3-yl)-1H-pyrazol-5-yl)benzyl)-1,2,3-oxadiazol-3-ium-5-yl)((2-(trifluoromethyl)pyridin-4-yl)carbamoyl)amide
- (3-((2'-(difluoromethyl)-[1,1'-biphenyl]-4-yl)methyl)-1,2,3-oxadiazol-3 -ium-5-yl)((2-(trifluoromethyl)pyridin-4-yl)carbamoyl)amide
- (3-(4-(2-(difluoromethyl)-4-methylpyridin-3-yl)benzyl)-1,2,3-oxadiazol-3-ium-5-yl)((2-(trifluoromethyl)pyridin-4-yl)carbamoyl)amide
- (3-(4-(4-chloro-1-methyl-1H-pyrazol-5-yl)-3-methylbenzyl)-1,2,3-oxadiazol-3-ium-5-yl)((2-(trifluoromethyl)pyridin-4-yl)carbamoyl)amide
- (3-(4-(4-methyl-1-(tetrahydro-2H-pyran-4-yl)-1H-pyrazol-5-yl)benzyl)-1,2,3-oxadiazol-3-ium-5-yl)((2-(trifluoromethyl)pyridin-4-yl)carbamoyl)amide
- (3-(1-(4-(4-chloro-1-methyl-1H-pyrazol-5-yl)phenyl)cyclopropyl)-1,2,3-oxadiazol-3-ium-5-yl)((2-(trifluoromethyl)pyridin-4-yl)carbamoyl)amide
- (3-(1-(4-(2-(difluoromethyl)pyridin-3-yl)phenyl)cyclopropyl)-1,2,3-oxadiazol-3-ium-5-yl)((2-(trifluoromethyl)pyridin-4-yl)carbamoyl)amide
- (3-(4-(3-(difluoromethyl)pyridin-2-yl)benzyl)-1,2,3-oxadiazol-3-ium-5-yl)((2-(trifluoromethyl)pyridin-4-yl)carbamoyl)amide
- (3-(2-chloro-4-(2-(difluoromethyl)pyridin-3-yl)benzyl)-1,2,3-oxadiazol-3-ium-5-yl)((2-(trifluoromethyl)pyridin-4-yl)carbamoyl)amide
- (3-(3-chloro-4-(4,6-dimethylpyrimidin-5-yl)benzyl)-1,2,3-oxadiazol-3-ium-5-yl)((2-(trifluoromethyl)pyridin-4-yl)carbamoyl)amide
- (3-(1-(4-(2-(difluoromethyl)pyridin-3-yl)phenyl)-2-hydroxyethyl)-1,2,3-oxadiazol-3-ium-5-yl)((2-(trifluoromethyl)pyridin-4-yl)carbamoyl)amide
- (3-(1-(4-(4-chloro-1-methyl-1H-pyrazol-5-yl)phenyl)-2-hydroxyethyl)-1,2,3-oxadiazol-3-ium-5-yl)((2-(trifluoromethyl)pyridin-4-yl)carbamoyl)amide
- (3-(4-(3-(trifluoromethyl)pyridin-4-yl)benzyl)-1,2,3-oxadiazol-3-ium-5-yl)((2-(trifluoromethyl)pyridin-4-yl)carbamoyl)amide
- (3-((6-(4,6-dimethylpyrimidin-5-yl)pyridin-3-yl)methyl)-1,2,3-oxadiazol-3-ium-5-yl)((2-(trifluoromethyl)pyridin-4-yl)carbamoyl)amide
- (3-((6-(4-chloro-1-methyl-1H-pyrazol-5-yl)pyridin-3-yl)methyl)-1,2,3-oxadiazol-3-ium-5-yl)((2-(trifluoromethyl)pyridin-4-yl)carbamoyl)amide
- (3-((2'-(difluoromethyl)-[2,3'-bipyridin]-5-yl)methyl)-1,2,3-oxadiazol-3-ium-5-yl)((2-(trifluoromethyl)pyridin-4-yl)carbamoyl)amide
- (3-(4-(3-hydroxypyridin-2-yl)benzyl)-1,2,3-oxadiazol-3-ium-5-yl)((2-(trifluoromethyl)pyridin-4-yl)carbamoyl)amide
- (3-(4-(4-chloro-1-(2-hydroxy-2-methylpropyl)-1H-pyrazol-5-yl)benzyl)-1,2,3-oxadiazol-3-ium-5-yl)((2-(trifluoromethyl)pyridin-4-yl)carbamoyl)amide
- (3-(4-(4-cyclopropyl-6-(trifluoromethyl)pyrimidin-5-yl)benzyl)-1,2,3-oxadiazol-3-ium-5-yl)((2-(trifluoromethyl)pyridin-4-yl)carbamoyl)amide
- (3-(4-(4-chloro-1-(tetrahydro-2H-pyran-4-yl)-1H-pyrazol-5-yl)benzyl)-1,2,3-oxadiazol-3-ium-5-yl)((2-(trifluoromethyl)pyridin-4-yl)carbamoyl)amide
- (3-(4-(4-chloro-1-(tetrahydrofuran-3-yl)-1H-pyrazol-5-yl)benzyl)-1,2,3-oxadiazol-3-ium-5-yl)((2-(trifluoromethyl)pyridin-4-yl)carbamoyl)amide
- (3-(4-(3-(trifluoromethyl)pyrazin-2-yl)benzyl)-1,2,3-oxadiazol-3-ium-5-yl)((2-(trifluoromethyl)pyridin-4-yl)carbamoyl)amide
- (3-(4-(2-(1,1-difluoro-2-hydroxyethyl)pyridin-3-yl)benzyl)-1,2,3-oxadiazol-3-ium-5-yl)((2-(trifluoromethyl)pyridin-4-yl)carbamoyl)amide
- (3-(4-(4-(difluoromethyl)pyridin-3-yl)benzyl)-1,2,3-oxadiazol-3-ium-5-yl)((2-(trifluoromethyl)pyridin-4-yl)carbamoyl)amide
- (3-(4-(4-chloro-1H-pyrazol-5-yl)benzyl)-1,2,3-oxadiazol-3-ium-5-yl)((2-(trifluoromethyl)pyridin-4-yl)carbamoyl)amide
- (3-(4-(3-(difluoromethyl)pyridin-4-yl)benzyl)-1,2,3-oxadiazol-3-ium-5-yl)((2-(trifluoromethyl)pyridin-4-yl)carbamoyl)amide
- (3-(4-(6-(methoxycarbonyl)-2-methylpyridin-3-yl)benzyl)-1,2,3-oxadiazol-3-ium-5-yl)((2-(trifluoromethyl)pyridin-4-yl)carbamoyl)amide
- (3-(4-(6-carboxy-2-methylpyridin-3-yl)benzyl)-1,2,3-oxadiazol-3-ium-5-yl)((2-(trifluoromethyl)pyridin-4-yl)carbamoyl)amide
- (3-(4-(4-chloro-1-(2,2,2-trifluoroethyl)-1H-pyrazol-3-yl)benzyl)-1,2,3-oxadiazol-3-ium-5-yl)((2-(trifluoromethyl)pyridin-4-yl)carbamoyl)amide
- (3-(4-(2-methyl-6-(methylcarbamoyl)pyridin-3-yl)benzyl)-1,2,3-oxadiazol-3-ium-5-yl)((2-(trifluoromethyl)pyridin-4-yl)carbamoyl)amide
- (3-(4-(3-Methoxypyridazin-4-yl)benzyl)-1,2,3-oxadiazol-3-ium-5-yl)((2-(trifluoromethyl)pyridin-4-yl)carbamoyl)amide
- ((2-cyclopropylpyridin-4-yl)carbamoyl)(3-(4-(4,6-dimethylpyrimidin-5-yl)benzyl)-1,2,3-oxadiazol-3-ium-5-yl)amide
- (3-(4-(4-chloro-1-methyl-1H-pyrazol-5-yl)benzyl)-1,2,3-oxadiazol-3-ium-5-yl)((2-cyclopropylpyridin-4-yl)carbamoyl)amide
- ((2-cyclopropylpyridin-4-yl)carbamoyl)(3-(4-(2-(difluoromethyl)pyridin-3-yl)benzyl)-1,2,3-oxadiazol-3-ium-5-yl)amide
- (3-(4-(4,6-Dimethylpyrimidin-5-yl)benzyl)-1,2,3-oxadiazol-3-ium-5-yl)((2-methoxy-6-(trifluoromethyl)pyridin-4-yl)carbamoyl)amide
- (3-(4-(4,6-Dimethylpyrimidin-5-yl)benzyl)-1,2,3-oxadiazol-3-ium-5-yl)((2-(trifluoromethoxy)pyridin-4-yl)carbamoyl)amide
- (3-(4-(4,6-Dimethylpyrimidin-5-yl)benzyl)-1,2,3-oxadiazol-3-ium-5-yl)((2-methyl-6-(trifluoromethyl)pyridin-4-yl)carbamoyl)amide
- ((2-Chloro-6-(trifluoromethyl)pyridin-4-yl)carbamoyl)(3-(4-(4,6-dimethylpyrimidin-5-yl)benzyl)-1,2,3-oxadiazol-3-ium-5-yl)amide
- (3-(4-(4,6-Dimethylpyrimidin-5-yl)benzyl)-1,2,3-oxadiazol-3-ium-5-yl)((2-(methylamino)-6-(trifluoromethyl)pyridin-4-yl)carbamoyl)amide.

The compound of the invention as above defined is an inhibitor of NS2B-NS3 protease of a Flavivirus, preferably an inhibitor of the NS2B-NS3 protease of West Nile and/or Zika virus.

It is a further object of the invention the compound as above defined for medical use, preferably said compound is for use in treatment and/or prevention of a Flavivirus infection, more preferably for use in the treatment and/or prevention of an infection from West Nile and Zika virus.

It is a further object of the invention a pharmaceutical composition comprising the compound of the invention, either alone or in combination with one further therapeutic agent, and at least one pharmaceutically acceptable excipient.

It is a further object of the invention said pharmaceutical composition for use in the treatment and/or prevention of a Flavivirus infection, preferably wherein the Flavivirus is selected from West Nile and Zika and virus.

### DETAILED DESCRIPTION OF THE INVENTION

The present invention provides inhibitors of NS2B-NS3 protease of a Flavivirus, preferably inhibitors of the NS2B-NS3 protease of Zika and/or West Nile virus, thus inhibiting replication of the above viruses in cells. More specifically, the present invention provides a specific chemotypes obtained by fine tuning the substituents on the two side of the molecule of general formula (I): the proper balance of the substituted pyridine on the urea moiety and of the aromatic/heteroaromatic ring in the bis-aromatic system provided compounds with significant selectivity versus the NS2B-NS3 proteases of West Nile virus and of Zika virus with respect to the other Flaviviruses' proteases. Moreover, the compounds of the invention are able to penetrate the blood brain barrier to reach their target and treat the infection at the central nervous system level.

In one embodiment the invention provides a compound of general formula (I):

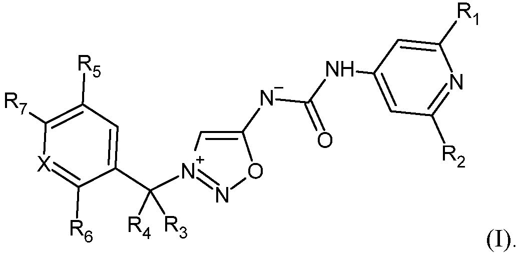

In the above formula (I), X is C or N.

Preferably in formula (I) R₁ is a fluoroalkyl or a fluoroalkoxy or a cycloalkyl. More specifically said R₁ can be any of CF₃, OCF₃ or a substituted cyclopropyl. Said cyclopropyl can opionally be substituted with fluorine atoms. When the cyclopropyl is substituted with fluorine, said fluorine can be on any carbon atom of the cyclopropyl ring; preferably said fluorine is linked to the carbon atom linked to the pyridine ring.

Preferably in formula (I) R₂ is: H, CH₃, OCH₃, Cl, NHCH₃, NHC(=O)R₁₂ wherein R₁₂ is a C₁₋₃alkyl optionally substituted with an aromatic ring. The definition of NHC(=O)R₁₂ encompasses methylamide, ethylamide, propylamide, isopropylamide, benzylamide, phenylethylamide and the like. It is also intended that the aromatic ring can be an heteroaromatic ring and that it can be substituted by, for example, a methyl or an halogen. More preferably, R₂ is H, methyl, chlorine or NHCH₃; even more preferably R₂ is H.

Preferably in formula (I) R₃ is H or CH₃ and R₄ is H or CH₂OH; or R₃ and R₄ are linked together to form a spirocyclopropyl ring; or R₃ and R₄ are methyl.

Preferably in formula (I) R₅ and R₆ are each independently selected from: H, CH₃ and Cl.

Preferably in formula (I) R₇ is a pyridine ring. Said pyridine ring in R₇ is optionally substituted with one or more substitents independently selected from C₁₋₃alkyl, C₁₋₃haloalkyl, C₁₋₃alkoxyl, halogen, hydroxy, C₁₋₃haloalkyl further substituted with an hydroxy group, C(O)OC₁₋₃alkyl, C(O)OH, C(O)NHC₁₋₃alkyl, NHC(=O)C₁₋₃alkyl, SO₂NHC₁₋₃alkyl, SO₂C₁₋₃alkyl, CN. Said optionally substituted pyridine is not 2-methylpyridin-3-yl and 2,4-dimethylpyridine-3-yl, as depicted below:

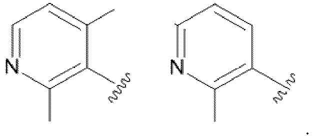

More preferably R₇ is a pyridine ring selected from: 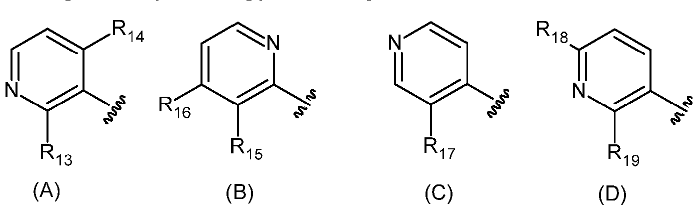 wherein:
- in structure (A) R₁₃ and R₁₄ are independently selected from H, C₁₋₃alkyl, C₁₋₃haloalkyl, C₁₋₃alkoxyl, halogen, and wherein one of R₁₃ and R₁₄ is C₁₋₃haloalkyl, preferably one of R₁₃ and R₁₄ is trifluoromethyl or difluoromethyl; as indicated above, in structure (A) R₁₃ and R₁₄ cannot be both CH₃ and/or if R₁₃ is CH₃ then R₁₄ is not H and/or if R₁₄ is CH₃ then R₁₃ is not H;
- in structure (B) R₁₅ and R₁₆ are independently selected from H, C₁₋₃haloalkyl and hydroxy, preferably the C₁₋₃haloalkyl is trifluoromethyl or difluoromethyl;
- in structure (C) R₁₇ is C₁₋₃haloalkyl, preferably trifluoromethyl or difluoromethyl;
- in structure (D) R₁₈ is selected from C₁₋₃alkyl and C(=O)OH, C(=O)=CH₃ and C(=O)NHCH₃ and R₁₉ is C₁₋₃alkyl or C₁₋₃haloalkyl.

Still preferably R₇ is a substituted pyridine selected from: 2-(trifluoromethyl)pyridin-3-yl, 2-(difluoromethyl)pyridin-3-yl, 3-(trifluoromethyl)pyridin-2-yl, 4-chloro-2-(trifluoromethyl)pyridin-3-yl, 4-(trifluoromethyl)pyridin-3-yl, 2-methoxy-4-(trifluoromethyl)pyridin-3-yl, 4-(trifluoromethyl)pyridin-2-yl, 2-trifluoromethylphenyl, 2-(difluoromethyl)-4-methylpyridin-3-yl, 3-(difluoromethyl)pyridin-2-yl, 2-(difluoromethyl)pyridin-3-yl, 3-(trifluoromethyl)pyridin-4-yl, 3-hydroxypyridin-2-yl, 1,1-difluoro-2-hydroxyethyl)pyridin-3-yl, 4-(difluoromethyl)pyridin-3-yl, 3-(difluoromethyl)pyridin-4-yl, 6-(methoxycarbonyl)-2-methylpyridin-3-yl, 6-carboxy-2-methylpyridin-3-yl, 2-methyl-6-(methylcarbamoyl)pyridin-3-yl.

Still preferably in formula (I) R₇ is a pyrazole of formula (II) or (III):

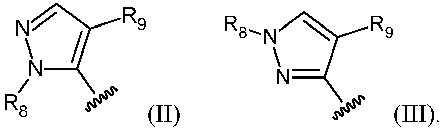

In the above pyrazoles (II) and (III) R₈ can be any of H, C₁₋₄alkyl optionally substituted with OH, C₃₋₆cycloalkyl, C₃₋₆heterocycloalkyl and C₁₋₃haloalkyl; R₉ can be any of C₁₋₃alkyl, C₁₋₃haloalkyl, C₁₋₃alkoxyl, CN and halogen.

In the above pyrazoles (II) and (III), if R₉ is CH₃ then R₈ is not CH₃ or an oxetane ring. With this proviso, preferably R₇ is a pyrazole of formula (II) wherein:
- R₈ is H, methyl, hydroxy-2-methyl-propyl, cyclopropyl, cyclobutyl, oxetane, tetrahydropyrane, tetrahydrofurane; and
- R₉ is methoxy, halogen, trifluoromethyl;
or wherein:
- R₈ is H, hydroxy-2-methyl-propyl, cyclopropyl, cyclobutyl, tetrahydropyrane, tetrahydrofurane; and
- R₉ is methyl.

Still preferably R₇ is a pyrazole of formula (III) wherein R₈ is C₁₋₃alkyl or C₁₋₃haloalkyl or and R₉ is halogen.

Still preferably R₇ is a substituted pyrazole selected from: 1-cyclopropyl-4-methoxy-1H-pyrazol-5-yl, 4-methoxy-1-methyl-1H-pyrazol-5-yl, 1-cyclopropyl-4-methyl-1H-pyrazol-5-yl, 1-cyclobutyl-4-methyl-1H-pyrazol-5-yl, 1-cyclobutyl-4-methoxy-1H-pyrazol-5-yl, 4-chloro-1-methyl-1H-pyrazol-5-yl, 4-(4-chloro-1-(oxetan-3-yl)-1H-pyrazol-5-yl, 1-methyl-4-(trifluoromethyl)-1H-pyrazol-5-yl, 1-(oxetan-3-yl)-4-(trifluoromethyl)-1H-pyrazol-5-yl, 4-fluoro-1-(oxetan-3-yl)-1H-pyrazol-5-yl, 4-chloro-1-methyl-1H-pyrazol-5-yl, 4-methyl-1-(tetrahydro-2H-pyran-4-yl)-1H-pyrazol-5-yl, 4-chloro-1-methyl-1H-pyrazol-5-yl, 4-chloro-1-methyl-1H-pyrazol-5-yl, 4-chloro-1-(2-hydroxy-2-methylpropyl)-1H-pyrazol-5-yl, 4-chloro-1-(tetrahydro-2H-pyran-4-yl)-1H-pyrazol-5-yl, 4-chloro-1-(tetrahydrofuran-3-yl)-1H-pyrazol-5-yl, 4-chloro-1H-pyrazol-5-yl, 4-chloro-1-(2,2,2-trifluoroethyl)-1H-pyrazol-3-yl.

Still preferably in formula (I) R₇ is a pyridazine optionally substituted with halogen, C₁₋₃alkoxyl, C₁₋₃haloalkyl, C₃₋₆cycloalkyl, CN. Said pyridazine in R₇ can be substituted with one, two or three of the above indicated substituents.

Still preferably in formula (I) R₇ is a pyrimidine of formula (IV):

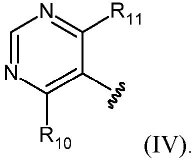

In the above pyrimidine R₁₀ and R₁₁ can be any of C₁₋₃alkyl, C₁₋₃haloalkyl, C₃₋₆cycloalkyl. Additionally, if R₁₀ and R₁₁ are both CH₃ in the above pyrimidine of formula (IV), then the following conditions must be verified by general formula (I):
- X is N; and/or
- R₁ is cyclopropyl;
- R₂ is CH₃, OCH₃, Cl, NHCH₃; and/or
- R₃ is H; and/or
- R₅ is Cl.

Still preferably in formula (I) R₇ is a phenyl, a pyrazine, a 6-oxo-1,6-dihydropyridine, an imidazole or an oxazole and each of said ring is optionally substituted with one or more substitents independently selected from C₁₋₃alkyl, C₁₋₃haloalkyl, C₁₋₃alkoxyl, halogen, hydroxy, C₁₋₃haloalkyl further substituted with an hydroxy group, C(O)OC₁₋₃alkyl, C(O)OH, C(O)NHC₁₋₃alkyl, CN, NH(CO)C₁₋₃alkyl.

Still preferably R₇ is selected from: 1,4-dimethyl-1H-imidazol-5-yl, 3-methoxypyridazin-4-yl, 1-cyclobutyl-4-methyl-1H-imidazol-5-yl, 2-trifluoromethylphenyl, 2-difluoromethylphenyl, 4,6-dimethylpyrimidin-5-yl, 4-cyclopropyl-6-(trifluoromethyl)pyrimidin-5-yl, 3-(trifluoromethyl)pyrazin-2-yl.

In a preferred embodiment of the invention, in the compound of formula (I) R₁ is CF₃ and R₂ is H. In a further embodiment, R₁ is cyclopropyl and R₂ is H. In a further embodyment R₁ is 1-fluoro-1-cyclopropyl and R₂ is H. Still preferably, R₁ is CF₃ and R₂ is OCH₃; R₁ is CF₃ and R₂ is CH₃; R₁ is CF₃ and R₂ is NHCH₃; R₁ is CF₃ and R₂ is Cl; R₁ is CF₃ and R₂ is NHCH₃.

In a preferred embodiment of the invention, in the compound of formula (I) R₃ and R₄ are H.

As explained above, the compounds of the invention can pass the blood brain barrier and this property is very important in the prevention and/or treatment of the mosquito-borne encephalitis. In an embodiment the invention provides compound according to general formula (I) characterized by a specific selection of the substituent(s) on the pyridine on the urea mojety and on the biaromatic/heteroaromatic mojety linked to the central sydnone through a methylene carbon. Said selection of substituents provided compounds able to inhibit the NS2B-NS3 protease of West Nile virus and of Zika virus with efficacy significantly higher than for the other Flaviviruses proteases. In a further embodiment the invention provides a compound as defined above for medical use, preferably for use in treatment and/or prevention of a Flavivirus infection, preferably wherein the Flavivirus is West Nile and Zika virus. In one embodiment, the Flavivirus infection is West Nile fever. In one embodiment, the Flavivirus infection is from the Zika virus.

Zika virus (ZIKV), the causative pathogen of Zika fever, is a small enveloped ss (+) RNA virus with a genome of around 11 kilobases in length that encodes a single polyprotein that is cleaved to produce 10 viral proteins. ZIKV infections in humans were sporadic before emerging in the Pacific and the Americas in the last decade. Indeed, ZIKV infection was associated with only mild illness prior to the large French Polynesian outbreak in 2013 and 2014, when severe neurological complications were reported, and the emergence in Brazil of a dramatic increase in severe congenital malformations (microcephaly) associated with ZIKV infection during pregnancy. The majority of cases (around 80%) are asymptomatic. When symptoms occur, they are typically mild, self-limiting, and similar to other arbovirus infections (e.g., DENV and CHIKV). Commonly reported symptoms include rash, fever, arthralgia and headache. Rare deaths have been described in patients infected with Zika virus. ZIKV is now known to cause fetal infection and congenital Zika syndrome, which includes microcephaly, cerebral malformations, ophthalmological and hearing defects, and arthrogryposis. Mild elevations in inflammatory markers have been described during ZIKV. Similarly to what happens in DENV infection, in ZIKV infection the interferon system is the central mediator of host defense and target of viral counterattack. A polyfunctional immune activation was seen during the acute phase of ZIKV infection, with elevated cytokine profiles associated with Thl (IL-2), Th2 (IL-4, IL-13), Thl7 (IL-17), and also Th9 (IL-9) responses (16). Increased IL-4, IL- 6, IL-8, IL-10, and IP-10 levels are also observed in ZIKV infected patients. Although no "cytokine storm" is observed in ZIKV patients, in ZIKV-infected placentas a massive inflammation has been observed, that may hamper the success of pregnancy. ZIKV perturbs the pro-/anti-inflammatory equilibrium of the placenta leading to tissue damage and massive infiltration of the villous core by inflammatory Hofbauer cells.

West Nile virus (WNV) is an important emerging neurotropic virus, responsible for increasingly severe encephalitis outbreaks in humans and horses worldwide. WNV is a member of the Flaviviridae family and is encoded by a ~ 11 kb positive-sense single-stranded RNA (ssRNA) genome. The genome is translated as a single polyprotein, and subsequent cleavage of this polyprotein by viral and host proteases generates 10 viral proteins. WNV pathogenesis follow three phases, the early phase initial infection and spread (the early phase), peripheral viral amplification (the visceral-organ dissemination phase) and neuroinvasion (the central nervous system (CNS) phase). The innate immune response, including type I interferon (IFN) and innate cell-mediated responses is responsible for the early control of WNV, whereas the adaptive immune response, including humoral and adaptive immune cell mediated responses (CD4+, CD8+ and regulatory T cells), is essential for WNV clearance and limiting possible immune response-mediated damage in the later stages of infection. The early phase after subcutaneous infection is defined by WNV replication in keratinocytes and skin-resident DCs, followed by viral amplification within the draining lymph node, which results in viremia and spread to visceral organs. The specific target cells for WNV infection are not well defined, but are thought to be subsets of DCs, macrophages and possibly neutrophils. WNV invasion of the CNS tissues (for example, the brain and spinal cord) constitutes the third phase of the infection. WNV may enter the brain through a combination of mechanisms that facilitate viral neuroinvasion, such as direct infection with or without a breakdown of the blood-brain barrier (BBB) and/or virus transport along peripheral neurons. Innate antiviral defenses are essential for the control of WNV infection, including the production of type I IFNs and pro-inflammatory cytokines, the expression of antiviral genes and the subsequent activation of the adaptive immune response. In WNV infection the innate immune activity is mainly triggered by RIG-1 like receptor (RLR) signaling, although Toll-like receptors (TLRs) could also contribute to NF-KB activation and the production of type I interferons and pro-inflammatory cytokines. DCs and macrophages, both of which are innate immune sentinel cells and target cells of WNV infection, are pivotal in linking innate and adaptive immune responses. Macrophages and DCs are readily activated by WNV, releasing pro-inflammatory cytokines and chemokines such as type IIFN, TNF, IL-Ib, CCL2, CCL3, CCL5 and IL-8. These cytokines are important in regulating innate cell-mediated responses (involving NK cells, neutrophils and gd T cells) as well as in developing adaptive immune responses. A major hallmark of WNV pathogenesis is neuroinflammation, which is caused by exaggerated innate and acquired immune response. Accumulation of inflammatory monocytes into the brain and their differentiation to macrophages and microglia can also worsen neuroinflammation and CNS injury, as demonstrated in a murine model of nonlethal WNV infection. Recognition of WNV nucleic acid in monocytes/microglia by TLRs may lead to the production of TNF-a, which results in a loss of tight junctions, allowing the entry of WNV and immune cells into the perivascular space of the brain in mice. Thus, activation of cells of the monocyte/macrophage system by WNV appears to result in important neuropathological consequences, and exaggerated innate responses may cause inflammation, altering the blood brain barrier permeability and allowing the virus to enter the CNS. Indeed, treatment of infected neuronal cells with antibodies blocking TNF-a and other pro-inflammatory mediators results in a significant reduction of WNV-mediated neuronal death, suggesting that such mediators play a major role in the pathogenesis of WNV infection in the CNS In a further embodiment the invention provides selective inhibitors of the West Nile virus and of the Zika virus protease, wherein said compounds are also able to pass the blood brain barrier. Therefore, the compounds of the invention are particularly suited for use in the treatment of the infection by West Nile virus in the central nervous system (CNS) phase.

It is a further object of the invention of a pharmaceutical composition comprising the compound as defined above and at least one pharmaceutically acceptable excipient.

Preferably the pharmaceutical composition is for use in the treatment and/or prevention of a flavivirus infection, preferably wherein the flavivirus is West Nile and Zika virus.

In a preferred embodiment, said pharmaceutical composition comprises at least one further active compound selected from the group consisting of: antivirals, antibacterials, anti-inflammatory agents, anti-pain agents and antipyretic agents.

It is a further object of the invention of a method for the synthesis of the compound of general formula (I) as defined above.

The present invention includes within its scope prodrugs of the compound of formula (I). In general, such prodrugs will be functional derivatives of the compound of formula (I) which are readily convertible *in vivo* into the required compound of formula (I). Conventional procedures for the selection and preparation of suitable prodrug derivatives are described, for example, in "Design of Prodrugs", ed. H. Bundgaard, Elsevier, 1985.

A prodrug may be a pharmacologically inactive derivative of a biologically active substance (the "parent drug" or "parent molecule") that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The present invention includes within its scope solvates of the compound of formula (I) and salts thereof, for example, hydrates.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention. In addition, the compounds disclosed herein may exist as tautomers and all tautomeric forms are intended to be encompassed by the scope of the invention, even though only one tautomeric structure is depicted. Specific tautomeric forms of compounds of the invention are depicted below:

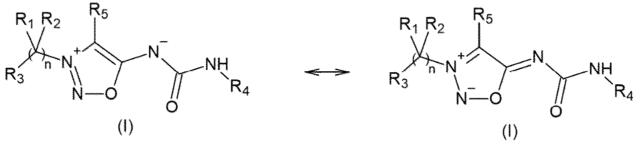

The compounds may exist in different isomeric forms, all of which are encompassed by the present invention.

When any variable occurs more than one time in any constituent, its definition on each occurrence is independent of every other occurrence. Also, combinations of substituents and variables are permissible only if such combinations result in stable compounds.

It is understood that substituents and substitution patterns on the compounds of the instant invention can be selected by one of ordinary skill in the art to provide compounds that are chemically stable and that can be readily synthesized from readily available starting materials by techniques known in the art, as well as those methods set forth below. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents. A substituent on a saturated, partially saturated or unsaturated heterocycle can be attached at any substitutable position.

As used herein, "alkyl" is intended to include both branched and straight-chain saturated aliphatic hydrocarbon groups having the specified number of carbon atoms. For example, "C₁-C₆ alkyl" is defined to include groups having 1, 2, 3, 4, 5 or 6 carbons in a linear or branched arrangement. For example, "C₁-C₆ alkyl" specifically includes methyl, ethyl, n-propyl, i-propyl, n-butyl, t-butyl, i-butyl, pentyl, hexyl, and so on.

"Cycloalkyl" refers to a non-aromatic hydrocarbon ring system (monocyclic, bicyclic, or polycyclic), including cyclized alkyl and alkenyl groups. The term "Cn-m cycloalkyl" or "(Cn-Cm) cycloalkyl" refers to a cycloalkyl that has n to m ring member carbon atoms. Cycloalkyl groups can include mono- or polycyclic (e.g., having 2, 3, or 4 fused rings) groups and spirocycles. Cycloalkyl groups can have 3, 4, 5, 6, 7, 8, 9, 10, 11, 12, 13, or 14 ring-forming carbons (C₃₋₁₄). In some embodiments, the cycloalkyl group has 3 to 14 members, 3 to 10 members, 3 to 6 ring members, 3 to 5 ring members, or 3 to 4 ring members. In some embodiments, the cycloalkyl group is monocyclic. In some embodiments, the cycloalkyl group is monocyclic or bicyclic. In some embodiments, the cycloalkyl group is a C₃₋₆ monocyclic cycloalkyl group. Ring-forming carbon atoms of a cycloalkyl group can be optionally oxidized to form an oxo or sulfido group. Cycloalkyl groups also include cycloalkylidenes. In some embodiments, cycloalkyl is cyclopropyl, cyclobutyl, cyclopentyl, or cyclohexyl. Examples of cycloalkyl groups include cyclopropyl, cyclobutyl, cyclopentyl, cyclohexyl, cycloheptyl, cyclopentenyl, cyclohexenyl, cyclohexadienyl, cycloheptatrienyl, norbornyl, norpinyl, norcamyl, bicyclo[1.1.1]pentanyl, bicyclo[2.1.1]hexanyl, and the like. In some embodiments, the cycloalkyl group is cyclopropyl, cyclobutyl, cyclopentyl, or cyclohexyl. In some embodiments, cycloalkyl includes a single saturated carbocyclic ring of three to eight ring carbons, such as cyclopropyl, cyclobutyl, cyclopentyl, and cyclohexyl. Cycloalkyl may optionally be substituted with one or more substituents, such as one, two, or three substituents. In some embodiments, the cycloalkyl substituent is selected from the group consisting of (C₁-C₆)alkyl, hydroxy, (C₁-C₆)alkoxy, halo(C₁-C₆)alkyl, halo(C₁-C₆)alkoxy, halo, amino, mono- and di(C₁-C₆)alkylamino, hetero(C₁-C₆)alkyl, acyl, aryl, and heteroaryl. A cycloalkyl group can be unsubstituted or optionally substituted. When optionally substituted, one or more hydrogen atoms of the cycloalkyl group (e.g., from 1 to 4, from 1 to 2, or 1) may be replaced with a moiety such as halogen, OH, cyano, methyl, trifluoromethyl, amino and the like. In some aspects, a substituted cycloalkyl group can incorporate an exo- or endocyclic alkene (e.g., cyclohex-2-en-1-yl). In some aspects, a cycloalkyl group is unsubstituted or not optionally substituted.

"Alkoxy" represents an alkyl group of indicated number of carbon atoms attached through an oxygen bridge. "Alkoxy" therefore encompasses the definitions of alkyl above. Examples of suitable alkoxy groups include methoxy, ethoxy, *n*-propoxy, *i*-propoxy, *n*-butoxy, *s*-butoxy and *t-*butoxy. The preferred alkoxy group is methoxy.

The terms "haloC₁₋₆alkyl" and "haloC₁₋₆alkoxy" mean a C₁₋₆alkyl or C₁₋₆alkoxy group in which one or more (in particular, 1 to 3) hydrogen atoms have been replaced by halogen atoms, especially fluorine or chlorine atoms. Preferred are fluoroC₁₋₆alkyl and fluoroC₁₋₆alkoxy groups, in particular fluoroC₁₋₃alkyl and fluoroC₁₋₃alkoxy groups, for example, CF₃, CHF₂, CH₂F, CH₂CH₂F, CH₂CHF₂, CH₂CF₃, OCF₃, OCHF₂, OCH₂F, OCH₂CH₂F, OCH₂CHF₂ or OCH₂CF₃, and most especially CF₃, OCF₃ and OCHF₂.

The term "hydroxyC₁₋₆alkyl" means a C₁₋₆alkyl group in which one or more (in particular, 1 to 3) hydrogen atoms have been replaced by hydroxy groups. Preferred are CH₂OH, CH₂CHOH and CHOHCH₃.

As used herein, "aryl" or "aromatic ring" is intended to mean any stable monocyclic or bicyclic carbon ring of 6 to 10 atoms, wherein at least one ring is aromatic. Examples of such aryl elements include phenyl, naphthyl, tetrahydronaphthyl, indanyl and tetrahydrobenzo[7]annulene. The preferred aryl group is phenyl or naphthyl, especially phenyl.

As used herein, "heteroaryl" or "heteroaromatic ring" is intended to mean any stable monocyclic or bicyclic ring containing 1, 2, 3 or 4 heteroatoms independently selected from N, O and S, wherein at least one ring is aromatic. In particular the term heteroaryl includes 5 membered aromatic heterocycles containing 1, 2, 3 or 4 heteroatoms independently selected from N, O and S , but not more than one of which is O or S; 6 membered aromatic heterocycles containing 1, 2 or 3 nitrogen atoms; or a 7-13 membered aromatic heterocycle containing heteroatoms independently selected from N, O or S of 5 to 10 atoms, wherein at least one ring is aromatic. Heteroaryl ring also includes partially saturated bicyclic systems, such as indoline, isoindoline, 4,5,6,7-tetrahydropyrazolo[1,5-a]pyrazine; further included are tautomeric structures, such as for example hydroxypyrimidine and hydroxypyrimidones.

The terms "heterocyclic ring", "heterocycloalkyl ring" and "cycloheteroalkyl ring" are used interchangeably in the present invention in the present invention and refer to a non-aromatic ring radical, which consists of carbon atoms and at least one heteroatom of nitrogen, phosphorus, oxygen or sulfurExamples of particular heterocyclic rings of the invention are tetrahydrofuranyl, tetrahydropyranyl, tetrahydrothiopyranyl, tetrahydroisoquinolinyl, azetidinyl, 1,4-dioxanyl, hexahydroazepinyl, piperazinyl, piperidyl, pyridin-2-onyl, pyrrolidinyl, imidazolinyl, pyrazolinyl, pyrrolinyl, morpholinyl, thiomorpholinyl, dihydrobenzoimidazolyl, dihydrobenzofuranyl, dihydrobenzothiophenyl, dihydrobenzoxazolyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, dihydroisochromenyl, dihydroimidazolonyl, dihydrotriazolonyl, dihydrobenzodioxinyl, dihydrothiazolopyrimidinyl, methylenedioxybenzoyl, tetrahydrofuranyl, tetrahydrothienyl, tetrahydroquinolinyl, thiazolidinonyl, imidazolonyl, isoindolinonyl, octahydroquinolizinyl, octahydroisoindolyl, azabicycloheptanyl, chromenonyl, triazolopyrimidinyl, dihydrobenzoxazinyl, thiazolotriazolyl, azoniabicycloheptanyl, azoniabicyclooctanyl. Attachment of a heterocyclyl substituent can occur via a carbon atom or via a heteroatom.

A preferred 4-member saturated heterocycle is azetidine. A further preferred 4-membered saturated heterocycle is oxetane.

A preferred 5-member saturated heterocycle is tetrahydrofurane.

Preferred 6 membered saturated or partially saturated heterocycles are pyrrolidinyl, piperidyl, piperazinyl, morpholinyl, thiomorpholinyl and thiazolidinyl.

Preferred 5 membered heteroaryls are thienyl, thiazolyl, pyrazolyl, isoxazolyl, imidazolyl, thiadiazolyl, oxazolyl, triazolyl, tetrazolyl, furyl and oxadiazolyl.

The preferred 6 membered heteroaryls are pyridinyl and pyrymidinyl.

Preferred 8-10 membered heteroaryls are benzothienyl, indolyl, benzothiadiazolyl, benzoxadiazolyl, thiazolotriazolyl, dihydrobenzodioxinyl, dihydrothiazolopyrimidinyl, dihydrobenzoxazinyl, dihydrobenzofuranyl, benzothiazolyl, quinolinyl, isoquinolinyl, benzimidazolyl, benzofuranyl, dihydrobenzoxazolyl, dihydroindolyl, dihydroquinazolinyl, dihydrophthalazinyl, indazolyl, benzisoxazolyl, benzotriazolyl, dihydroisoindolyl, tetrahydronaphthyridinyl, triazolopyrimidinyl and tetrahydroquinolinyl.

As used herein, the term 'halogen' refers to fluorine, chlorine, bromine and iodine, of which fluorine, chlorine and bromine are preferred.

As used herein, the term "amine" refers to linear and cyclic amines, such as methylamine, dimethylamine, diethylamine, isopropylamine, pyrrolidine, pyperidine and the like. A substutuent referred generically as "amine" indicates that an amine radical is linked through its nitrogen to the specific residue. In some instances, it is herein used the term "C₁₋₆alkylamine" which refers to an alkyl group substituted with an amino group, such as -CH₂NH₂, -CH₂NHCH₃, CH₂NHCH₂CH₃, CH₂-pyrrolidine, CH₂-piperidine and the like.

As used herein, an optionally substituted aryl, heteroaryl, cycloalkyl, heterocycloalkyl refer to any of said ring systems as above defined being optionally substituted with groups such as halogen, hydroxy, methyl, trifluoromethyl, amino, methylamino, and the like.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reaction of the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base.

Thus, pharmaceutically acceptable salts of the compounds of this invention include the conventional non-toxic salts of the compounds of this invention as formed by reaction of a basic instant compound with an inorganic or organic acid. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like. Preferably, a pharmaceutically acceptable salt of this invention contains one equivalent of a compound of formula (I) and 1, 2 or 3 equivalents of an inorganic or organic acid. More particularly, pharmaceutically acceptable salts of this invention are the tartrate, trifluoroacetate or the chloride salts.

When the compound of the present invention is acidic, suitable "pharmaceutically acceptable salts" refers to salts prepared form pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, *N*,*N*¹-dibenzylethylenediamine, diethylamine, 2-diethylaminoethanol, 2-dimethylaminoethanol, ethanolamine, ethylenediamine, *N-*ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described above, and other typical pharmaceutically acceptable salts is more fully described by Berg et al., "Pharmaceutical Salts," J. Pharm. Sci., 1977:66:1-19.

It will also be noted that the compounds of the present invention are potentially internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

The compounds of the invention find use in a variety of applications for human and animal health. The compounds of the invention are flavivirus inhibitors and can be used in the treatment and/or prevention of flavivirus infections.

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

The invention also provides pharmaceutical compositions comprising one or more compounds of this invention and a pharmaceutically acceptable carrier. The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water-soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or *n*-propyl *p*-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsion. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solution. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution may be then introduced into a water and glycerol mixture and processed to form a microemulsion. The injectable solutions or microemulsions may be introduced into a patient's blood stream by local bolus injection.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of inj ectables.

Compounds of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound of the invention are employed. The compounds of the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol.

When a compound according to this invention is administered into a human subject, the daily dosage regimen will normally be determined by the prescribing physician with the dosage generally varying according to the age, weight, sex and response of the individual patient, as well as the severity of the patient's symptoms.

The instant compounds are also useful in combination with known therapeutic agents for simultaneous, separate or sequential administration.

The term "administration" and variants thereof (e.g., "administering" a compound) in reference to a compound of the invention means introducing the compound or a prodrug of the compound into the system of the subject in need of treatment. When a compound of the invention or prodrug thereof is provided in combination with one or more other active agents (e.g., a cytotoxic agent, etc.), "administration" and its variants are each understood to include concurrent and sequential introduction of the compound or prodrug thereof and other agents.

As used herein, the term "composition" is intended to encompass a product comprising the specified ingredients in the specified amounts, as well as any product which results, directly or indirectly, from combination of the specified ingredients in the specified amounts.

The term "therapeutically effective amount" as used herein means that amount of active compound or pharmaceutical agent that elicits the biological or medicinal response in a tissue, system, animal or human that is being sought by a researcher, veterinarian, medical doctor or other clinician.

When a compound of the present invention is administered into a human subject, the daily dosage regimen will normally be determined by the prescribing physician, with the dosage generally varying according to the age, weight, and response of the individual patient, as well as the severity of the patient's symptoms. In one exemplary application, oral dosages of the present invention will range between about 0.01 mg per kg of body weight per day (mg/kg/day) to about 100 mg/kg/day, preferably 0.01 to 10 mg/kg/day, and most preferably 0.1 to 5.0 mg/kg/day. In embodiments of the invention, the compounds of the present invention may be administered according to a dosing regimen of a daily dose. In embodiments of the invention, the compounds of the present invention may be administered according to a dosing regimen of a once daily dose, more preferably according to a twice a day (or b.i.d.) dosing regimen. In further embodiments, the compounds of the present invention may be administered according to a dosing regimen of a daily dose for 10 days, 9 days, 8 days, 7 days, 6 days, 5 days, 4 days, 3 days, 2 days or 1 day. Preferred regimen is 2-5 days, or 3-5 days, or 3, 4 or 5 days, most preferably 3 days or 5 days. The dose may be a dose of 5 mg a day or less, 4.5 mg a day or less, 4 mg a day or less, 3.5 mg a day or less, 3 mg a day or less, 2.5 mg a day or less or 2 mg a day or less.

It is a further object of the invention a method treating, ameliorating or preventing a Flavivirus infection and related conditions in a subject, preferably wherein the Flavivirus is selected from the group consisting of West Nile and Zika virus, comprising administering to said subject a therapeutically effective amount of the compound as defined above.

These and other aspects of the invention will be apparent from the teachings contained herein. The invention will be illustrated by reference to the following non-limited examples.

### EXAMPLES

### Chemistry

### General

The compounds, or pharmaceutically acceptable salts thereof, compositions, and methods described herein are further illustrated by the following non-limiting examples.

As used herein, the following abbreviations have the following meanings. If an abbreviation is not defined, it has its generally accepted meaning.

Boc₂O: Di-tert-butyl dicarbonate; DCM: Dichloromethane; DHP: 3,4-Dihydropyran; DIPEA: *N*,*N*-Diisopropylethylamine; DMF: Dimethylformamide; DMSO: Dimethylsulfoxide; ES⁺: Electrospray Positive Ionisation; EtOAc: Ethyl acetate; EtOH: Ethanol; h: Hour; HATU: Hexafluorophosphate Azabenzotriazole Tetramethyl Uronium; HPLC: High Performance Liquid Chromatography; LiBH₄: Lithium borohydride; LiOH: Lithium hydroxide; LCMS: Liquid Chromatography Mass Spectrometry; KOAc: Potassium acetate; K₂CO₃: Potassium carbonate; K₃PO₄: tripotassium phosphate; MeCN: Acetonitrile; MeOH: Methanol; min: Minute; NaH: Sodium Hydride; NH₃: ammonium; NMP: N-Methylpyrrolidone; NMR: Nuclear Magnetic Resonance; NH₂NH₂: Hydrazine; NaCNBH₃: Sodium cyanoborohydride; Pd(dppf)Cl₂: (1,1'-Bis(diphenylphosphino)ferrocene)-palladium(II) dichloride; Pd(OAc)₂: Palladium(II) acetate; PCy₃: triclyclohexylphosphine; Py: pyridine; RP: Reverse Phase; t_{R}: Retention time; rt: room temperature; ^{t}BuONO: *tert*-Butyl nitrite; TBDMS: Tert-Butyldimethylsilyl; TEA: Triethylamine; TIPS: Triisopropylsilyl ether; TFA: Trifluoroacetic acid; THF: Tetrahydrofurane; UPLC: Ultra High Performance Liquid Chromatography.

### General experimental details

Solvents and reagents were obtained from commercial suppliers and were used without further purification. Flash chromatography purifications were performed on prepacked cartridges on a Biotage system. Purity of final compounds was determined using MS and UPLC. UPLC-MS analyses were performed on a Waters Acquity UPLC^{™}, equipped with a diode array and a ZQ mass spectrometer, using an X-Terra C18 column (5 µm, 4.6 x 50 mm) or a BEH C18 column (1.7 mm, 2.1 x 50 mm). Mobile phase comprised a linear gradient of binary mixtures of H₂O containing 0.1% formic acid (A), and MeCN containing 0.1% formic acid (B). The linear gradient used is: (A): 90% (0.1 min), 90%-0% (2.6 min), 0% (0.3 min), 0%-90% (0.1 min) with a 0.5 mL/min flow. The purity of final compounds was ≥95%. All ¹H spectra were recorded on Bruker AV400 spectrometer at 400 MHz except where indicated. Chemical shift (δ) are reported in parts per million relative to TMS using CDCl₃ as a solvent or relative to the residual solvent signal using DMSO-d₆. Coupling costants (*J*) are reported in Hertz (Hz). Multiplicities are reported as singlet (s), broad (br), doublet (d), doublet of doublet (dd), doublet of doublet of doublet (ddd), triplet (t), doublet of triplet (dt), doublet of doublet of triplet (ddt), triplet of triplet (tt), quartet (q), doublet of quartets (dq) or multiplet (m). Unless indicated, spectra were acquired at 300 K. Temperatures are expressed in degrees Celsius (°C) and are uncorrected. Unless otherwise indicated, commercially available reagents and solvents (HPLC grade) were used without further purification. Where the synthesis of intermediates and starting materials is not described, these compounds are commercially available or can be made from commercially available compounds by standard methods or by extension of the Examples herein. During any of the synthetic sequences described herein it may be necessary and/or desirable to protect sensitive or reactive groups or any of the molecules concerned, this may be achieved by means of conventional protecting groups, such as those described in Protecting Groups in Organic Synthesis (3rd Edition, Greene, T.W. and Wuts, P.G.M.; Wiley Interscience, 1999) and Protecting Groups (Kocienski, P.J.; Thieme, 1994).

Intermediates **1-24** were synthetized following the synthetic procedures described below.

### Intermediates 2 & 4:1-Cyclobutyl-4-methyl-5-(4,4,5,5-tetramethyl-1,3,2-dioxaborolan-2-yl)-1H-pyrazole & 4-chloro-1-methyl-5-(4,4,5,5-tetramethyl-1,3,2-dioxaborolan-2-yl)-1H-pyrazole

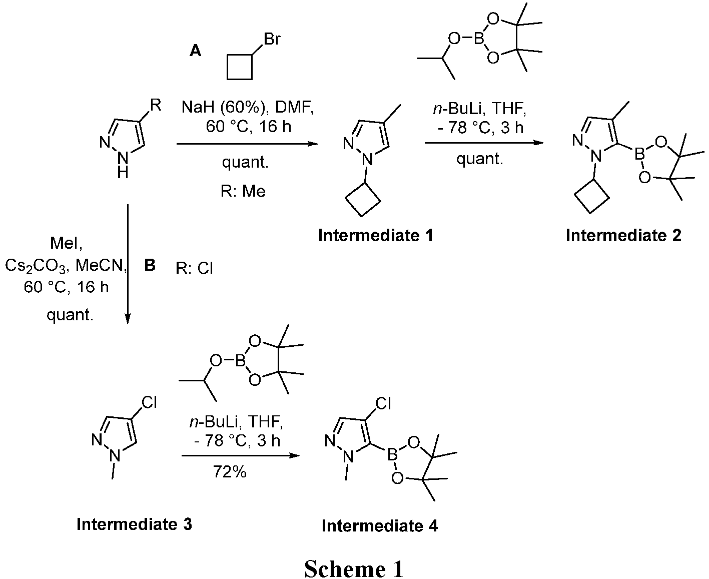

### Step 1A: 1-Cyclobutyl-4-methyl-1H-pyrazole (Intermediate 1)

A solution of 4-methyl-1*H*-pyrazole (493 mg, 6 mmol) in DMF (2 mL) at 0 °C, NaH (60%, 600 mg, 15.01 mmol) was added, the resulted suspension was stirred at 0 °C for 30 min. Then bromocyclobutane (1.2 g, 9.01 mmol) was added at 0 °C and then the reaction was heated at 60 °C for 16 h. After consumption of starting material, the reaction mixture was cooled at 0 °C and subsequently quenched with water. The crude reaction was extracted with EtOAc (2x). The collected organic layers were washed with brine, dried over Na₂SO₄, filtered and concentrated *in vacuo* to give the crude compound as a colourless oil (910 mg, quant.) which was used in next step without further purification.

### Step 2A: 1-Cyclobutyl-4-methyl-5-(4, 4, 5, 5-tetramethyl-1,3, 2-dioxaborolan-2-yl)-1H-pyrazole (Intermediate 2)

A solution of 1-cyclobutyl-4-methyl-1*H*-pyrazole (200 mg, 1.32 mmol) in THF (4.5 mL) at - 78 °C, was treated with n-BuLi (2.5 M in hexanes, 687 µL, 1.72 mmoL) and stirred at - 78 °C for 1 h. Then 2-isopropoxy-4,4,5,5-tetramethyl-1,3,2-dioxoborolane (320 mg, 1.72 mmol) was added and the reaction was stirred for 1 h at - 78 °C. After checking the effective formation of the desire product, the mixture was warmed at 0 °C and subsequent quenched with NH₄Cl sat. sol. followed by HCl (1 N) until pH = 7. The aqueous phase was then extracted with EtOAc (2x) and the resulting organic phase was washed with brine, dried over Na₂SO₄, filtered and concentrated *in vacuo* to give the Intermediate **2** as a yellow oil (350 mg, quant.) as a mixture of boronic ester and boronic acid (50:50 calculated by UPLC). The resulting crude compound was used in the next step without further purification. LCMS (ES⁺) *m*/*z* calculated for C₁₄H₂₃BN₂O₂ 262.16, found 263 (M+H)⁺ HPLC t_{R} 2.20 min (boronic ester); 180 (M+H)⁺ HPLC t_{R} 0.80 min (boronic acid)

### Step 1B: 4-Chloro-1-methyl-pyrazole (Intermediate 3)

A suspension of 4-methyl-1*H*-pyrazole (300 mg, 2.93 mmol) and Cs₂CO₃ (1430 mg, 4.39 mmol) in acetonitrile (5.8 mL) at rt, iodomethane (830 mg, 5.85 mmol) was added. The reaction mixture was then heated to 30 °C and stirred for 16 h. The mixture was cooled to rt and then quenched with water and EtOAc. The extracted organic phase was washed with brine, dried over Na₂SO₄, filtered and concentrated *in vacuo* to give the crude product as a colorless oil (170 mg, 50%) which was used in the next step without further purification. LCMS (ES⁺) *m*/*z* calculated for C₄H₅ClN₂ 116.55, found 196 (M+DMSO+H)⁺ HPLC t_{R} 1.13 min.

### Step 2B: 4-Chloro-1-methyl-S-(4, 4, 5, 5-tetramethyl-1,3, 2-dioxaborolan-2-yl)-1H-pyrazole (Intermediate 4)

A solution of 4-chloro-1-methyl-pyrazole (Intermediate **3**, 70 mg, 0.6 mmol) in THF (2 mL) at - 78 °C was treated with n-BuLi (2.5 M in hexanes, 312 µL, 0.78 mmoL) and stirred at - 78 °C for 1 h. Then 2-isopropoxy-4,4,5,5-tetramethyl-1,3,2-dioxoborolane (145 mg, 0.78 mmol) was added and the reaction was stirred for 1 h at - 78 °C. After checking the effective formation of the desire product, the mixture was warmed at 0 °C and quenched with NH₄Cl sat. sol. followed by HCl (1 N) until pH = 7. The aqueous phase was then extracted with EtOAc (2x) and the resulting organic phase was washed with brine, dried over Na₂SO₄, filtered and concentrated *in vacuo* to give the crude product which was purified by flash chromatography on silica gel (eluting with 0-100% EtOAc in petroleum ether) to get the title compound as a white solid (162 mg, 72%) as mixture of boronic ester and boronic acid (5:95 calculated by UPLC). The resulting crude compound was used in the next step without further purification. ¹H NMR (400 MHz, DMSO-d₆) δ 7.56 (s, 1H), 3.96 (s, 3H), 1.32 (s, 12H). LCMS (ES⁺) m/z calculated for C₁₀H₁₆BClN₂O₂ 242.51, found 243 (M+H)⁺. HPLC t_{R} 1.88 min (boronic ester); found 161 (M+H)⁺. HPLC *t*_{R} 0.65 min (boronic acid).

Intermediates **5-10** were prepared using the synthetic procedure described for the synthesis of Intermediate **2** or Intermediate **4** in Scheme 1 using the appropriate starting materials and Cs₂CO₃ (1.5 equiv.) or NaH 60% dispersion in mineral oil (2.5 equiv.) as specified.

### Intermediate 5: 1-Cyclobutyl-4-methoxy-5-(4,4,5,5-tetramethyl-1,3,2-dioxaborolan-2-yl)-1H-pyrazole

The title compound was obtained as a yellow oil (49 mg, 79% yield). The title compound was obtained as a mixture of boronic ester and boronic acid (65:35 calculated by UPLC). LCMS (ES⁺) *m*/*z* calculated for C₁₄H₂₃BN₂O₃ 278.16, found 279 (M+H)⁺. HPLC *t*_{R} 2.01 min (boronic ester); 197 (M+H)⁺. HPLC t_{R} 1.10 min (boronic acid).

### Intermediate 6: 4-Chloro-1-(oxetan-3-yl)-5-(4,4,5,5-tetramethyl-1,3,2-dioxaborolan-2-yl)-1H-pyrazole

The title compound was obtained as a white solid (108 mg, quant.) which was used as a crude in the next step. LCMS (ES⁺) *m*/*z* calculated for C₁₂H₁₈BClN₂O₃ 284.55, found 203 (M+H-pinacol)⁺. HPLC *t*_{R} 0.71 min (boronic acid).

### Intermediate 7: 1-Methyl-5-(4,4,5,5-tetramethyl-1,3,2-dioxaborolan-2-yl)-4-(trifluoromethyl)-1H-pyrazole

The title compound was obtained as a yellow oil (130 mg, quant.) and used as a crude in the next step. The title compound was obtained as a mixture of boronic ester and boronic acid (1:1 calculated by UPLC). LCMS (ES⁺) *m*/*z* calculated for C₁₁H₁₆BF₃N₂O₂ 276.07, found 277 (M+H)⁺. HPLC *t*_{R} 1.99 min (boronic ester); found 195 (M+H)⁺. HPLC t_{R} 0.84 min (boronic acid).

### Intermediate 8: 1-(Oxetan-3-yl)-5-(4,4,5,5-tetramethyl-1,3,2-dioxaborolan-2-yl)-4-(trifluoromethyl)-1H-pyrazole

The title compound was obtained as a yellow oil (162 mg, 80%) and used as a crude in the next step. The title compound was obtained as a mixture of boronic ester and boronic acid (1:1 calculated by UPLC). LCMS (ES⁺) *m*/*z* calculated for C₁₃H₁₈BF₃N₂O₃ 318.10, found 319 (M+H)⁺. HPLC *t*_{R} 1.68 min (boronic ester); found 237 (M+H)⁺. HPLC *t*_{R} 0.90 min (boronic acid).

### Intermediate 9: 4-Fluoro-1-(oxetan-3-yl)-5-(4,4,5,5-tetramethyl-1,3,2-dioxaborolan-2-yl)-1H-pyrazole

The title compound was obtained as a yellow oil (162 mg, 86%) as mixture of boronic ester and acid (1:1 calculated by UPLC). LCMS (ES⁺) *m*/*z* calculated for C₁₂H₁₈BFN₂O₃ 268.10, found 187 (M+H-pinacol)⁺. HPLC *t*_{R} 0.54 min (boronic acid).

### Intermediate 10: 4-Chloro-1-tetrahydrofuran-3-yl-5-(4,4,5,5-tetramethyl-1,3,2-dioxaborolan-2-yl)pyrazole

The title compound was obtained as a colorless oil (68 mg, 29%). ¹H NMR (400 MHz, DMSO-*d₆*) δ 7.63 (s, 1H), 5.44-5.37 (m, 1H), 4.03-3.91 (m, 2H), 3.86-3.75 (m, 2H), 2.40-2.26 (m, 2H), 1.32 (s, 12H). LCMS (ES⁺) *m*/*z* calculated for C₁₃H₂₀BClN₂O₃ 298.57-300.57, found 299-301 (M+H)⁺. HPLC *t*_{R} 2.05 min.

### Intermediate 11: 2-(Difluoromethyl)-3-(4,4,5,5-tetramethyl-1,3,2-dioxaborolan-2-yl)pyridine

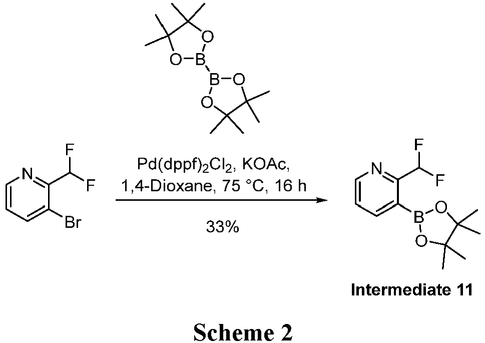

A suspension of 3-bromo-2-(difluoromethyl)pyridine (245 mg, 1.18 mmol), bis(pinacolato)diboron (598 mg, 2.36 mmol), Pd(dppf)Cl₂ (86.2 mg, 0.12 mmol) and KOAc (347 mg, 3.53 mmol) in anhydrous 1,4-dioxane (7.8 mL) was heated at 75 °C for 3 h. Then the reaction mixture was diluted with EtOAc (2x), washed with water and brine. The collected organic layer was dried over Na₂SO₄, filtered, and concentrated *in vacuo* to get a residue which was purified by flash chromatography on silica gel (eluting with 0-100% EtOAc in petroleum ether) to give the title compound as a colorless oil (100 mg, 33%). ¹H NMR (400 MHz, DMSO-d₆) δ 8.78 (dd, *J =* 1.8, 4.8 Hz, 1H), 8.13 (td, *J* = 0.9, 7.6 Hz, 1H), 7.61-7.57 (m, 1H), 7.21 (t, *J* = 54.7 Hz, 1H), 1.34 (s, 12H). ¹⁹F NMR (377 MHz, DMSO-d₆) δ -114.05 (s, 2F). LCMS (ES⁺) *m*/*z* calculated for C₁₂H₁₆BF₂NO₂ 255.07, found 174 (M+H-pinacol)⁺. HPLC *t*_{R} 0.82 min.

Intermediate **12** was prepared using the synthetic procedure described for the synthesis of Intermediate **11** in Scheme 2 using the appropriate starting materials.

### Intermediate 12: 4,6-Dimethyl-5-(4,4,5,5-tetramethyl-1,3,2-dioxaborolan-2-yl)pyrimidine

The title compound was obtained as a brown oil (3.0 g, 49%). ¹H NMR (400 MHz, DMSO-d₆) δ 8.88 (s, 1H), 2.49-2.48 (m, 6H), 1.36 (s, 12H). LCMS (ES⁺) *m*/*z* calculated for C₁₂H₁₉BN₂O₂ 234.10, found 235 (M+H)⁺. HPLC *t*_{R} 1.41 min.

### Intermediate 13: 3-Bromo-4-(difluoromethyl)pyridine

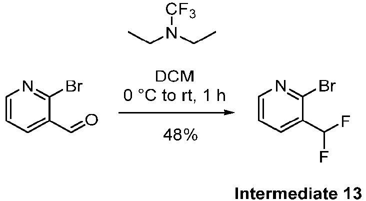

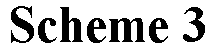

A solution of diethyl(trifluorosulfuranyl)amine (0.39 mL, 2.96 mmol) in dry DCM (0.3 mL) at rt, was added dropwise to a stirring solution of 3-bromoisonicotinaldehyde (100 mg, 0.54 mmol) in dry DCM (0.7 mL) at 0 °C. The reaction was allowed to warm up to rt and stirred for 1 h before being poured in ice-cold water. A saturated aqueous solution of NaHCO₃ was added until pH = 8 and the solution was diluted with DCM (2x). The organic layer was dried over Na₂SO₄, filtered, and concentrated *in vacuo* to get a residue which was purified by flash chromatography on silica gel (eluting with 0-100% EtOAc in petroleum ether) to get the title compound as a colorless oil (54 mg, 48%). ¹H NMR (400 MHz, DMSO-d₆) δ 8.92 (s, 1H), 8.75 (d, *J=* 5.0 Hz, 1H), 7.70 (d, *J* = 4.9 Hz, 1H), 7.19 (t, *J=* 53.5 Hz, 1H). ¹⁹F NMR (376 MHz, DMSO-d₆) δ -118.68 (s, 2F). LCMS (ES⁺) *m*/*z* calculated for C₆H₄BrF₂N 206.95-208.95, found 208-210 (M+H)⁺. HPLC *t*_{R} 1.46 min

Intermediates **14** and **15** were prepared using the synthetic procedure described for the synthesis of Intermediate **13** in Scheme 3 using the appropriate starting materials.

### Intermediate 14: 2-Bromo-3-(difluoromethyl)pyridine

The title compound was obtained as an orange oil (34 mg, 20%). ¹H NMR (400 MHz, DMSO-d₆) δ 8.60-8.55 (m, 1H), 8.14-8.09 (m, 1H), 7.64 (dd, *J=* 4.8, 7.7 Hz, 1H), 7.16 (t, *J=* 53.9 Hz, 1H). ¹⁹F NMR (376 MHz, DMSO-d₆) δ -115.92 (s, 2F). LCMS (ES⁺) *m*/*z* calculated for C₆H₄BrF₂N 206.95-208.95, found 208-210 (M+H)⁺. HPLC *t*_{R} 1.38 min.

### Intermediate 15: 4-Bromo-3-(difluoromethyl)pyridine

The title compound was obtained as a colourless oil (125 mg, quant.). ¹H NMR (400 MHz, DMSO-*d₆*) δ 8.79 (s, 1H), 8.61 (d, *J* = 5.3 Hz, 1H), 7.90 (d, *J* = 5.4 Hz, 1H), 7.24 (t, *J* = 52 Hz, 1H). ¹⁹F NMR (376 MHz, DMSO-*d₆*) δ -116.00 (s, 2F). LCMS (ES⁺) *m*/*z* calculated for C₆H₄BrF₂N 206.95-208.95, found 208, 210 (M+H)⁺. HPLC *t*_{R} 1.37 min.

### Intermediate 18: 4-Methyl-1-tetrahydropyran-4-yl-5-(4,4,5,5-tetramethyl-1,3,2-dioxaborolan-2-yl)pyrazole

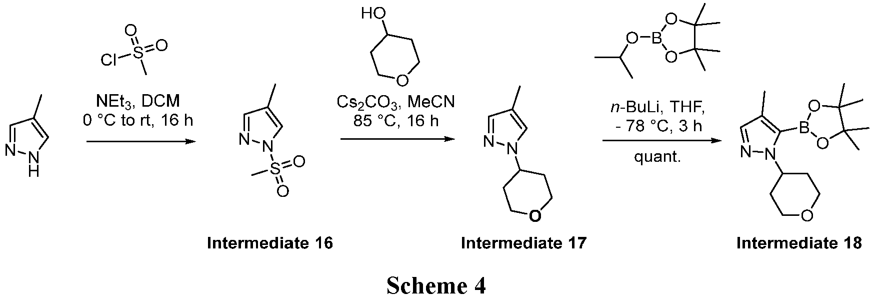

### Step 1: 4-Methyl-1-methylsulfonyl-pyrazole (Intermediate 16)

A suspension of 4-methyl-1*H*-pyrazole (300 mg, 3.65 mmol) and *N*,*N*-diethylethanamine (0.65 mL, 4.75 mmol) in dry DCM (12 mL) at 0 °C, methanesulfonyl chloride (460 mg, 4.02 mmol) was added. The resulting reaction mixture was allowed to warm up to rt and was stirred for 16 h before being quenched with water. The layer was separated, and the collected organic phase was dried over Na₂SO₄, filtered, and concentrated *in vacuo* to give a residue which was used without further purification in the next step. LCMS (ES⁺) *m*/*z* calculated for C₅H₈N₂O₂S 160.19, found 161 (M+H)⁺. HPLC *t*_{R} 0.85 min.

### Step 2: 4-Methyl-1-tetrahydropyran-4-yl-pyrazolepyrazole (Intermediate 17)

A suspension of 4-methyl-1-methylsulfonyl-pyrazole (Intermediate **16**, 99 mg, 0.59 mmol), tetrahydropyran-4-ol (50 mg, 0.49 mmol) and Cs₂CO₃ (287 mg, 0.88 mmol) in dry acetonitrile (1.6 mL) was stirred at 85 °C for 16 h, before being diluted with water and EtOAc. The organic layer was dried over Na₂SO₄, filtered, and concentrated *in vacuo* to give a residue which was used as such in step 3. LCMS (ES⁺) *m*/*z* calculated for C₉H₁₄N₂O 166.22, found 167 (M+H)⁺. HPLC *t*_{R} 0.96 min.

### Step 3: 4-Methyl-1-tetrahydropyran-4-yl-5-(4,4,5,5-tetramethyl-1,3,2-dioxaborolan-2-yl)pyrazole (Intermediate 18)

The title compound was prepared following the procedure reported for the synthesis of Intermediate **2** (in Scheme 1, Step 2) and was obtained as a colorless oil (21.0 mg, 68%). LCMS (ES⁺) *m*/*z* calculated for C₁₅H₂₅BN₂O₃ 292.18, found 293 (M+H)⁺. HPLC *t*_{R} 1.91 min.

Intermediate **19** was prepared using the synthetic procedure described for the synthesis of Intermediate **18** in Scheme 4 using the appropriate starting materials

### Intermediate 19: 4-Chloro-1-tetrahydropyran-4-yl-5-(4,4,5,5-tetramethyl-1,3,2-dioxaborolan-2-yl)pyrazole

The title compound was obtained as a white solid (48 mg, 14%). ¹H NMR (400 MHz, DMSO-*d₆*) δ 7.62 (s, 1H), 4.86-4.77 (m, 1H), 3.98 (br dd, *J=* 3.8, 11.4 Hz, 2H), 3.45-3.36 (m, 2H), 2.05-1.95 (m, 2H), 1.83 (br dd, *J =* 2.1, 12.4 Hz, 2H), 1.32 (s, 12H). LCMS (ES⁺) *m*/*z* calculated for C₁₄H₂₂BClN₂O₃ 312.60, found 313 (M+H)⁺. HPLC *t*_{R} 2.09 min.

### Intermediate 22: 4-Chloro-1-tetrahydrofuran-3-yl-5-(4,4,5,5-tetramethyl-1,3,2-dioxaborolan-2-yl)pyrazole

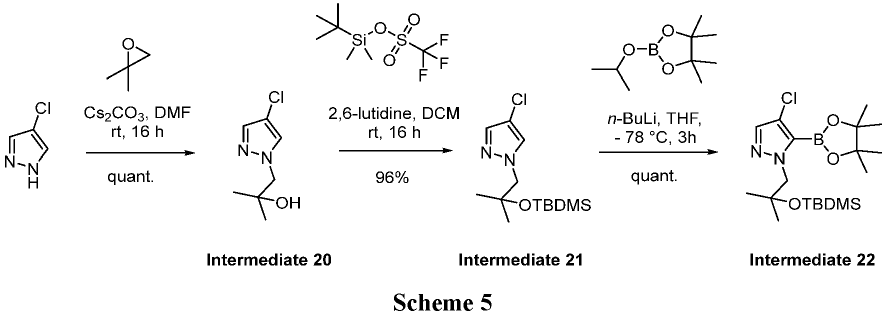

### Step 1: 1-(4-Chloropyrazol-1-yl)-2-methyl-propan-2-ol (Intermediate 20)

A suspension of 4-chloro-1*H*-pyrazole (300 mg, 2.93 mmol) and Cs₂CO₃ (846 mg, 4.39 mmol) in dry DMF (3 mL) at rt, 2,2-dimethyloxirane (632 mg, 8.78 mmol) was added. The resulting reaction mixture was stirred at rt for 16 h before being quenched with water and diluted with EtOAc. The organic layer was dried over Na₂SO₄, filtered, and concentrated *in vacuo* to get a residue which was purified by flash chromatography on silica gel (eluting with 0-100% EtOAc in petroleum ether) to give the title compound as a colorless oil (524 mg, quant.). ¹H NMR (400 MHz, DMSO-*d₆*) δ 7.84 (s, 1H), 7.51 (s, 1H), 4.72 (s, 1H), 3.98 (s, 2H), 1.05 (s, 6H). LCMS (ES⁺) *m*/*z* calculated for C₇H₁₁ClN₂O 174.63, found 175 (M+H)⁺. HPLC *t*_{R} 1.01 min.

### Step 2: Tert-butyl-[2-(4-chloropyrazol-1-yl)-1,1-dimethyl-ethoxy]-dimethyl-silane (Intermediate 21)

A stirred suspension of 1-(4-chloropyrazol-1-yl)-2-methyl-propan-2-ol (Intermediate **20**, 524 mg, 3.00 mmol) in dry DCM (5.8 mL) at 0 °C, was treated with 2,6-lutidine (0.5 mL, 4.39 mmol) and trifluoromethanesulfonic acid (0.8 mL, 3.51 mmol). The resulting reaction mixture was allowed to warm up to rt and stirred at rt for 16 h before being quenched with H₂O and diluted with EtOAc. The organic layer was dried over Na₂SO₄, filtered, and concentrated *in vacuo* to get a residue which was purified by flash chromatography on silica gel (eluting with 0-100% EtOAc in petroleum ether) to give the title compound as a colorless oil (811 mg, 96%). ¹H NMR (400 MHz, DMSO-d₆) δ 7.78 (s, 1H), 7.53 (s, 1H), 4.01 (s, 2H), 1.18 (s, 6H), 0.83 (s, 9H), 0.02 (s, 6H). LCMS (ES⁺) *m*/*z* calculated for C₁₃H₂₅ClN₂OSi 288.89, found 289 (M+H)⁺. HPLC *t*_{R} 2.70 min.

### Step 3: Tert-butyl-[2-[4-chloro-5-(4,4,5,5-tetramethyl-1,3,2-dioxaborolan-2yl)pyrazol-1-yl]-1,1-dimethyl-ethoxy]-dimethyl-silane (Intermediate 22)

The title compound was prepared following the procedure reported for the synthesis of Intermediate **2** (in Scheme 1, Step 2) and was obtained as a colorless oil (46 mg, 16%). ¹H NMR (400 MHz, DMSO-d₆) δ 7.62 (s, 1H), 4.31 (s, 2H), 1.31 (s, 12H), 1.20 (s, 6H), 0.79 (s, 9H), 0.05 (s, 6H). LCMS (ES⁺) *m*/*z* calculated for C₁₉H₃₆BClN₂O₃Si 414.85, found 201 (M+H-pin-OTBDMS)⁺. HPLC *t*_{R} 1.05 min.

### Intermediates 24: 4-Chloro-1-(tetrahydro-2H-pyran-2-yl)-5-(4,4,5,5-tetramethyl-1,3,2-dioxaborolan-2-yl)-1H-pyrazole

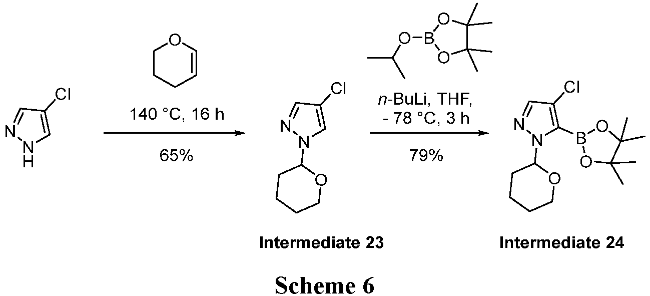

### Step 1: 4-Chloro-1-(tetrahydro-2H-pyran-2-yl)-1H-pyrazole (Intermediate 23)

DHP (3.2 g, 38.04 mmol) was added to 4-chloro-1*H*-pyrazole (3 g, 29.26 mmol) and the resulting mixture was stirred at 140 °C for 16 h. The reaction was allowed to warm up to rt and the residue was purified by flash chromatography on silica gel (eluting with 0-100% EtOAc in petroleum ether) to give the title compound as a colorless oil (3.5 g, 65%). LCMS (ES⁺) *m*/*z* calculated for C₈H₁₁ClN₂O 186.64, found 209 (M+Na)⁺. HPLC *t*_{R} 1.60 min.

### Step 2: 4-Chloro-1-(tetrahydro-2H-pyran-2-yl)-5-(4, 4,5, 5-tetramethyl-1,3,2-dioxaborolan-2-yl)-1H-pyrazole (Intermediate 24)

A solution of 4-chloro-1-(tetrahydro-2*H*-pyran-2-yl)-1*H*-pyrazole (Intermediate **23**, 2.4 g, 12.71 mmol) in THF (42 mL) at -78 °C was treated with n-BuLi (2.5 M in hexanes, 11.9 mL, 19.07 mmoL) and the resulted mixture reaction was stirred at -78 °C for 1 h. Then 2-isopropoxy-4,4,5,5-tetramethyl-1,3,2-dioxoborolane (3.1 mg, 16.52 mmol) was added and the reaction was stirred for 1h at -78 °C. After checking the effective formation of the desire product, the mixture was allowed to heat to 0 °C and quenched with NH₄Clss followed by HCl (1 N) until pH = 7. The mixture was then extracted with EtOAc (2x) and the resulting organic phase was washed with brine, dried over Na₂SO₄, filtered and concentrated *in vacuo* to get the residue which was purified by flash chromatography on silica gel (eluting with 0-35% EtOAc in petroleum ether) to give the title compound Intermediate **24** as a yellow oil (3.6 g, 79%). LCMS (ES⁺) *m*/*z* calculated for C₁₄H₂₂BClN₂O₃ 312.60, found 229 (M-pinacol-H)⁻. HPLC *t*_{R} 1.20 min.

### Examples 1 & 8:(3-(4-(2-(Trifluoromethyl)pyridin-3-yl)benzyl)-1,2,3-oxadiazol-3-ium-5-yl)((2-(trifluoromethyl)pyridin-4-yl)carbamoyl)amide & (3-(4-(1-Cyclobutyl-4-methyl-1H-pyrazol-5-yl)benzyl)-1,2,3-oxadiazol-3-ium-5-yl)((2-(trifluoromethyl)pyridin-4-yl)carbamoyl)amide.

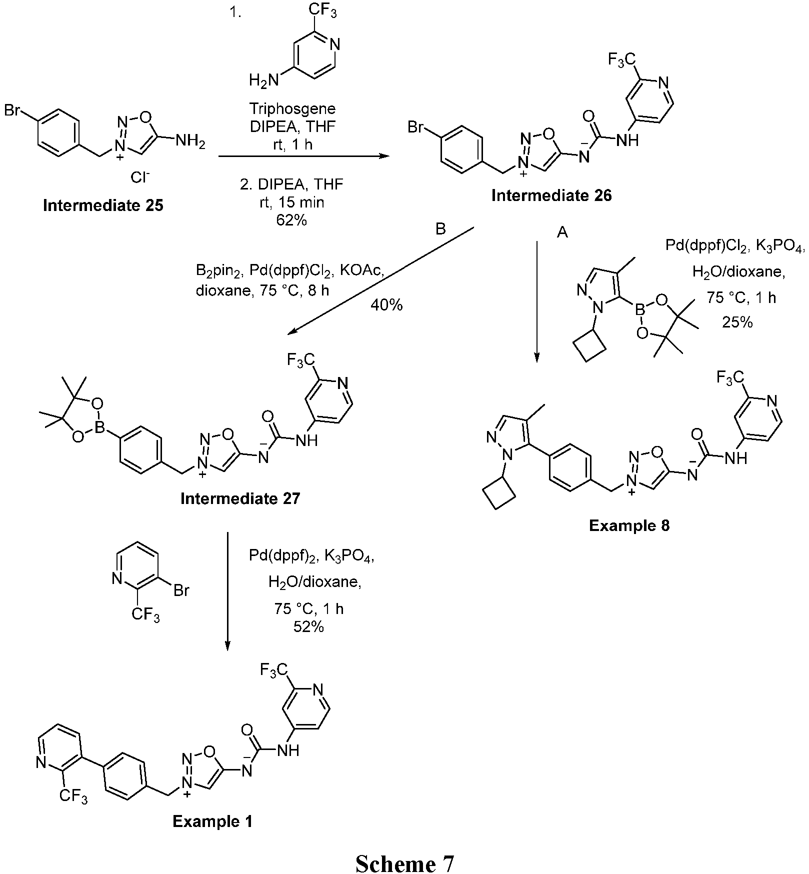

### Step 1: (3-(4-Bromobenzyl)-1,2, 3-oxadiazol-3-ium-5-yl) ((2-(trifluoromethyl)pyridin-4-yl)carbamoyl)amide (Intermediate 26)

A solution of 2-(trifluoromethyl)pyridin-4-amine (222 mg, 1.41 mmol) and DIPEA (0.48 mL, 2.77 mmol) in dry THF (1.3 mL) was treated with triphosgene (204 mg, 0.69 mmol) and stirred at rt for 1 h. This solution was added dropwise to a stirring suspension of 5-amino-3-(4-bromobenzyl)-1,2,3-oxadiazol-3-ium chloride (Intermediate **25**, 200 mg, 0.69 mmol, prepared as described in WO2023/227734) in dry THF (5.0 mL) followed by the addition of DIPEA (0.48 mL, 2.77 mmol) at 0 °C. The mixture was stirred at this temperature for 5 minutes before being diluted with H₂O and extracted with EtOAc (2x). The collected organic layers were washed with brine, dried over Na₂SO₄, filtered, and concentrated *in vacuo* to get a residue which was purified by flash chromatography on silica gel (eluting with 0-100% EtOAc in petroleum ether) to give the title compound as a yellow solid (190 mg, 62%). ¹H NMR (400 MHz, DMSO-*d*₆) δ 10.17 (s, 1H), 8.46 (d, *J* = 5.6 Hz, 1H), 8.31 (s, 1H), 8.20 (d, *J* = 1.8 Hz, 1H), 7.70-7.67 (m, 3H), 7.57-7.53 (m, 2H), 5.81 (s, 2H). LCMS (ES⁺) *m*/*z* calculated for C₁₆H₁₁BrF₃N₅O₂ 441.00, 443.00 found 440, 442 (M-H)⁻. HPLC *t*_{R} 1.81 min.

### Step 2A: (3-(4-(1-Cyclobutyl-4-methyl-1H-pyrazol-5-yl)benzyl)-1,2,3-oxadiazol-3-ium-5-yl)((2-(trifluoromethyl)pyridin-4-yl)carbamoyl)amide (Example 8)

A suspension of (3-(4-bromobenzyl)-1,2,3-oxadiazol-3-ium-5-yl)((2-(trifluoromethyl)pyridin-4-yl)carbamoyl)amide (Intermediate **26**, 30 mg, 0.07 mmol), Intermediate **2** (25.4 mg, 0.12 mmol), Pd(dppf)Cl₂ (5 mg, 0.01 mmol) and K₃PO₄ (43.2 mg, 0.2 mmol) in a mixture of 1,4-dioxane (0.8 mL) and H₂O (0.1 mL) was heated at 75 °C for 1 h before being diluted with EtOAc and washed with H₂O and brine. The organic layer was dried over Na₂SO₄, filtered, and concentrated *in vacuo* to get a residue which was purified by flash chromatography on C18 RP (eluting with 0-100% CH₃CN in H₂O) to give the title compound as a white powder (14.0 mg, 25%). ¹H NMR (400 MHz, DMSO-*d*₆) δ 10.19 (s, 1H), 8.47 (d, *J=* 5.6 Hz, 1H), 8.41 (s, 1H), 8.21 (d, *J=* 1.9 Hz, 1H), 7.73-7.68 (m, 3H), 7.45-7.39 (m, 3H), 5.91 (s, 2H), 4.59 (t, *J=* 8.3 Hz, 1H), 3.31-3.29 (m, 1H), 2.60-2.53 (m, 2H), 2.27-2.18 (m, 2H), 1.94 (s, 3H), 1.77-1.62 (m, 2H). ¹⁹F NMR (377 MHz, DMSO-d₆) δ -66.93 (s, 3F). LCMS (ES⁺) *m*/*z* calculated for C₂₄H₂₂F₃N₇O₂ 497.47, found 498 (M+H)⁺. HPLC *t*_{R} 1.78 min.

### Step 2B: (3-(4-(4, 4, 5, 5-Tetramethyl-1,3, 2-dioxaborolan-2-yl) benzyl)-1,2, 3-oxadiazol-3-ium-5-yl)((2-(trifluoromethyl)pyridin-4-yl)carbamoyl)amide (Intermediate 27)

A suspension of (3-(4-bromobenzyl)-1,2,3-oxadiazol-3-ium-5-yl)((2-(trifluoromethyl)pyridin-4-yl)carbamoyl)amide (Intermediate **26**, 192 mg, 0.43 mmol), bis(pinacolate)diboron (221 mg, 0.87 mmol), Pd(dppf)Cl₂ (31.8 mg, 0.04 mmol) and KOAc (128 mg, 1.3 mmol) in 1,4-dioxane (4.0 mL) was heated at 75 °C for 8 h before being diluted with EtOAc and washed with H₂O and brine. The organic layer was dried over Na₂SO₄, filtered, and concentrated *in vacuo* to get a residue which was purified by flash chromatography on silica gel (eluting with 0-100% EtOAc in petroleum ether) to give the title compound as an orange powder (112 mg, 53%). ¹H NMR (400 MHz, DMSO-d₆) δ 10.17 (s, 1H), 8.46 (d, *J* = 5.6 Hz, 1H), 8.28 (s, 1H), 8.19 (d, *J* = 1.8 Hz, 1H), 7.75 (d, *J=* 8.1 Hz, 2H), 7.69 (dd, *J=* 5.6, 1.9 Hz, 1H), 7.57 (d, *J =* 8.1 Hz, 2H), 5.86 (s, 2H), 1.30 (s, 12H). LCMS (ES⁺) *m*/*z* calculated for C₂₂H₂₃BF₃N₅O₄ 489.18, found 490 (M+H)⁺. HPLC *t*_{R} 2.02 min.

### Step 3: (3-(4-(2-(Trifluoromethyl)pyridin-3-yl)benzyl)-1,2,3-oxadiazol-3-ium-5-yl)((2-(trifluoromethyl)pyridin-4-yl)carbamoyl)amide (Example 1)

The title compound was prepared following the conditions reported for the synthesis of **Example 2** in Step 2A using (3-(4-(4,4,5,5-tetramethyl-1,3,2-dioxaborolan-2-yl)benzyl)-1,2,3-oxadiazol-3-ium-5-yl)((2-(trifluoromethyl)pyridin-4-yl)carbamoyl)amide (Intermediate **27**) and 5-bromo-4-trifluoromehylpyrimidine as starting materials. The title compound was obtained as a white powder (26.4 mg, 52%). ¹H NMR (400 MHz, DMSO-d₆) δ 10.19 (s, 1H), 8.80 (dd, *J* = 1.1, 4.6 Hz, 1H), 8.47 (d, *J =* 5.6 Hz, 1H), 8.38 (s, 1H), 8.21 (d, *J =* 1.8 Hz, 1H), 7.96 (d, *J* = 8.0 Hz, 1H), 7.80 (dd, *J* = 4.6, 7.9 Hz, 1H), 7.72-7.67 (m, 3H), 7.48 (d, *J* = 8.1 Hz, 2H), 5.92 (s, 2H). ¹⁹F NMR (377 MHz, DMSO-d₆) δ -59.80 (s, 3F), -66.93 (s, 3F). LCMS (ES⁺) *m*/*z* calculated for C₂₂H₁₄F₆N₆O₂ 508.38, found 509 (M+H)⁺. HPLC *t*_{R} 1.74 min.

**Examples 6, 9, 11, 14, 16-17, 22, 25-28, 31-33, 39** and **42-43** were prepared using the synthetic procedure described for the synthesis of **Example 8** in Step 2A Scheme 7 using the appropriate starting materials.

### Example 6: (3-(4-(3-Methoxypyridazin-4-yl)benzyl)-1,2,3-oxadiazol-3-ium-5-yl)((2-(trifluoromethyl)pyridin-4-yl)carbamoyl)amide (trifluoroacetate salt).

The title compound was obtained as a white powder (19.5 mg, 40%). ¹H NMR (400 MHz, DMSO-*d₆*) δ 10.19 (s, 1H), 9.17 (d, *J* = 6.3 Hz, 1H), 8.46 (d, *J* = 5.6 Hz, 1H), 8.36 (s, 1H), 8.20 (d, *J* = 1.8 Hz, 1H), 7.94 (d, *J=* 8.3 Hz, 2H), 7.74-7.69 (m, 3H), 7.58 (d, *J=* 6.3 Hz, 1H), 5.92 (s, 2H), 3.99 (s, 3H). ¹⁹F NMR (377 MHz, DMSO-d₆) δ -66.92 (s, 3F) and -74.68 (s, 6F). LCMS (ES⁺) *m*/*z* calculated for C₂₁H₁₆F₃N₇O₃ 471.39, found 472 (M+H)⁺. HPLC *t*_{R} 1.14 min.

### Example 9: ((3-(4-(1-Cyclobutyl-4-methoxy-1H-pyrazol-5-yl)benzyl)-1,2,3-oxadiazol-3-ium-5-yl)((2-(trifluoromethyl)pyridin-4-yl)carbamoyl)amide.

The title compound obtained as a white powder (11.5 mg, 19%). ¹H NMR (400 MHz, DMSO-d₆) δ 10.19 (s, 1H), 8.46 (d, *J* = 5.6 Hz, 1H), 8.37 (s, 1H), 8.21 (d, *J =* 1.9 Hz, 1H), 7.70-7.68 (m, 3H), 7.53 (s, 1H), 7.45 (d, *J=* 8.3 Hz, 2H), 5.89 (s, 2H), 4.75-4.67 (m, 1H), 3.70 (s, 3H), 2.61-2.54 (m, 2H), 2.31-2.21 (m, 2H), 1.77-1.66 (m, 2H). ¹⁹F NMR (377 MHz, DMSO-d₆) δ -66.92 (s, 3F). LCMS (ES⁺) *m*/*z* calculated for C₂₄H₂₂F₃N₇O₃ 513.47, found 514 (M+H)⁺. HPLC *t*_{R} 1.74 min.

### Example 11: (3-(4-(4-Chloro-1-methyl-1H-pyrazol-5-yl)benzyl)-1,2,3-oxadiazol-3-ium-5-yl)((2-(trifluoromethyl)pyridin-4-yl)carbamoyl)amide.

The title compound was obtained as a white powder (3.6 mg, 4%). ¹H NMR (400 MHz, DMSO-*d₆*) δ 10.19 (s, 1H), 8.46 (d, *J=* 5.6 Hz, 1H), 8.41 (s, 1H), 8.21 (d, *J=* 1.9 Hz, 1H), 7.78-7.74 (m, *J=* 8.4 Hz, 2H), 7.70 (dd, *J=* 1.9, 5.6 Hz, 1H), 7.68 (s, 1H), 7.65-7.60 (m, 2H), 5.92 (s, 2H), 3.78 (s, 3H). ¹⁹F NMR (377 MHz, DMSO-d₆) δ -66.93 (s, 3F). LCMS (ES⁺) *m*/*z* calculated for C₂₀H₁₅F₃N₇O₂ 477.83, found 478 (M+H)⁺. HPLC *t*_{R} 1.61 min.

### Example 14: (3-(4-(4-Chloro-1-(oxetan-3-yl)-1H-pyrazol-5-yl)benzyl)-1,2,3-oxadiazol-3-ium-5-yl)((2-(trifluoromethyl)pyridin-4-yl)carbamoyl)amide.

The title compound was obtained as a white powder (5.3 mg, 8%). ¹H NMR (400 MHz, DMSO-*d₆*) δ 10.19 (s, 1H), 8.47 (d, *J* = 5.6 Hz, 1H), 8.42 (s, 1H), 8.21 (d, *J=* 1.8 Hz, 1H), 7.90 (s, 1H), 7.77-7.73 (m, *J* = 8.4 Hz, 2H), 7.70 (dd, *J* = 1.9, 5.6 Hz, 1H), 7.51-7.47 (m, 2H), 5.92 (s, 2H), 5.46-5.38 (m, 1H), 4.92 (t, *J=* 6.4 Hz, 2H), 4.83-4.77 (m, 2H). ¹⁹F NMR (377 MHz, DMSO-d₆) δ -66.93 (s, 3F). LCMS (ES⁺) *m*/*z* calculated for C₂₂H₁₇F₃N₇O₃ 519.86, found 520 (M+H)⁺. HPLC *t*_{R} 1.61 min.

### Example 16: (3-(4-(1-Methyl-4-(trifluoromethyl)-1H-pyrazol-5-yl)benzyl)-1,2,3-oxadiazol-3-ium-5-yl)((2-(trifluoromethyl)pyridin-4-yl)carbamoyl)amide.

The title compound was obtained as a white powder (3.2 mg, 6%). ¹H NMR (400 MHz, DMSO-*d₆*) δ 10.19 (s, 1H), 8.47 (d, *J=* 5.6 Hz, 1H), 8.42 (s, 1H), 8.21 (d, *J=* 1.8 Hz, 1H), 7.90 (s, 1H), 7.77-7.73 (m, *J* = 8.4 Hz, 2H), 7.70 (dd, *J* = 1.9, 5.6 Hz, 1H), 7.51-7.47 (m, 2H), 5.92 (s, 2H), 5.46-5.38 (m, 1H), 4.92 (t, *J=* 6.4 Hz, 2H), 4.83-4.77 (m, 2H). ¹⁹F NMR (377 MHz, DMSO-d₆) δ -53.29 (s, 3F), -66.93 (s, 3F). LCMS (ES⁺) *m*/*z* calculated for C₂₁H₁₅F₆N₇O 511.38, found 512 (M+H)⁺. HPLC *t*_{R} 1.70 min.

### Example 17: (3-(4-(1-(Oxetan-3-yl)-4-(trifluoromethyl)-1H-pyrazol-5-yl)benzyl)-1,2,3-oxadiazol-3-ium-5-yl)((2-(trifluoromethyl)pyridin-4-yl)carbamoyl)amide.

The title compound was obtained as a yellow powder (6.1 mg, 10%). ¹H NMR (400 MHz, DMSO-*d₆*) δ 10.20 (s, 1H), 8.47 (d, *J*= 5.8 Hz, 1H), 8.43 (s, 1H), 8.20 (d, *J*= 11.5 Hz, 2H), 7.76-7.68 (m, 3H), 7.49 (d, *J=* 8.3 Hz, 2H), 5.93 (s, 2H), 5.30 (s, 1H), 4.93 (t, *J=* 6.4 Hz, 2H), 4.77 (t, *J=* 7.2 Hz, 2H). ¹⁹F NMR (377 MHz, DMSO-d₆) δ -55.38 (s, 3F), -66.93 (s, 3F). LCMS (ES⁺) m/z calculated for C₂₃H₁₇F₆N₇O₃ 553.42, found 554 (M+H)⁺. HPLC *t*_{R} 1.70 min.

### Example 22: (3-(4-(4-Fluoro-1-(oxetan-3-yl)-1H-pyrazol-5-yl)benzyl)-1,2,3-oxadiazol-3-ium-5-yl)((2-(trifluoromethyl)pyridin-4-yl)carbamoyl).

The title compound was obtained as a white powder (2.1 mg, 4%). ¹H NMR (400 MHz, DMSO-*d₆*) δ 10.19 (s, 1H), 8.48-8.44 (m, 1H), 8.40 (s, 1H), 8.21 (d, *J=* 1.9 Hz, 1H), 7.84 (d, *J=* 4.5 Hz, 1H), 7.75 (d, *J* = 8.4 Hz, 2H), 7.70 (dd, *J =* 1.8, 5.6 Hz, 1H), 7.50 (d, *J* = 8.0 Hz, 2H), 5.92 (s, 2H), 5.53-5.45 (m, 1H), 4.93 (t, *J =* 6.4 Hz, 2H), 4.86-4.79 (m, 2H). ¹⁹F NMR (377 MHz, DMSO-*d*₆) δ -66.93 (s, 3F), -177.01 (s, 1F). LCMS (ES⁺) *m*/*z* calculated for C₂₂H₁₇F₄N₇O₃ 533.41, found 534 (M+H)⁺. HPLC *t*_{R} 1.54 min.

### Example 25: (3-(4-(4-Chloro-1-methyl-1H-pyrazol-5-yl)-3-methylbenzyl)-1,2,3-oxadiazol-3-ium-5-yl)((2-(trifluoromethyl)pyridin-4-yl)carbamoyl)amide (trifluoroacetate salt).

The title compound was obtained as a white powder (5.6 mg, 13%). ¹H NMR (400 MHz, DMSO-*d₆*) δ 10.19 (s, 1H), 8.46 (d, *J* = 5.4 Hz, 1H), 8.40 (s, 1H), 8.21 (d, *J =* 1.8 Hz, 1H), 7.70 (dd, *J=* 1.9, 5.6 Hz, 1H), 7.68 (s, 1H), 7.61 (s, 1H), 7.54 (dd, *J =* 1.5, 7.8 Hz, 1H), 7.39 (d, *J* = 7.8 Hz, 1H), 5.88 (s, 2H), 3.58 (s, 3H), 2.14 (s, 3H). ¹⁹F NMR (377 MHz, DMSO-*d*₆) δ -66.92 (s, 3F), - 73.63 (s, 6F). LCMS (ES⁺) *m*/*z* calculated for C₂₁H₁₇ClF₃N₇O₂ 491.85, found 492 (M+H)⁺. HPLC *t*_{R} 1.75 min.

### Example 26: (3-(4-(4-Methyl-1-(tetrahydro-2H-pyran-4-yl)-1H-pyrazol-5-yl)benzyl)-1,2,3-oxadiazol-3-ium-5-yl)((2-(trifluoromethyl)pyridin-4-yl)carbamoyl)amide.

The title compound was obtained as a white powder (4.5 mg, 19%). ¹H NMR (400 MHz, DMSO-*d₆*) δ 10.19 (s, 1H), 8.47 (d, *J=* 5.6 Hz, 1H), 8.42 (s, 1H), 8.21-8.21 (m, 1H), 8.21 (d, *J=* 1.9 Hz, 1H), 7.72 (d, J = 8.3 Hz, 2H), 7.69 (d, *J* = 1.9 Hz, 1H), 7.48 (d, *J*= 8.3 Hz, 2H), 7.42 (s, 1H), 5.91 (s, 2H), 4.15 (br d, *J=* 11.4 Hz, 1H), 3.84-3.92 (m, 2H), 3.24-3.30 (m, 2H), 2.08 (br dd, J = 11.9, 4.6 Hz, 2H), 1.92 (s, 3H), 1.73 (br dd, *J* = 12.6, 2.6 Hz, 2H). ¹⁹F NMR (377 MHz, DMSO-d₆) δ - 66.93 (s, 3F). LCMS (ES⁺) *m*/*z* calculated for C₂₅H₂₄F₃N₇O₃ 527.50, found 528 (M+H)⁺. HPLC *t*_{R} 1.62 min.

### Example 27: (3-(1-(4-(4-Chloro-1-methyl-1H-pyrazol-5-yl)phenyl)-cyclopropyl)-1,2,3-oxadiazol-3-ium-5-yl)((2-(trifluoromethyl)pyridin-4-yl)carbamoyl) amide.

The title compound was obtained as a white powder (20.8 mg, 60%).¹H NMR (400 MHz, DMSO-*d₆*) δ 10.21 (s, 1H), 8.50 (s, 1H), 8.46 (d, *J =* 5.6 Hz, 1H), 8.24 (d, *J =* 1.9 Hz, 1H), 7.71-7.62 (m, 4H), 7.62-7.56 (m, 2H), 3.78 (s, 3H), 2.11-2.03 (m, 2H), 1.86-1.78 ppm (m, 2H). ¹⁹F NMR (377 MHz, DMSO-*d*₆) δ -66.91 (s, 3F). LCMS (ES⁺) *m*/*z* calculated for C₂₂H₁₇ClF₃N₇O₂ 503.86, found 504 (M+H)⁺. HPLC *t*_{R} 1.85 min.

### Example 28: (3-(1-(4-(2-(Difluoromethyl)pyridin-3-yl)phenyl)cyclopropyl)-1,2,3-oxadiazol-3-ium-5-yl)((2-(trifluoromethyl)pyridin-4-yl)carbamoyl)amide.

The title compound was obtained as a white powder (28.2 mg, 60%).¹H NMR (400 MHz, DMSO-*d₆*) δ 10.21 (s, 1H), 8.77-8.73 (m, 1H), 8.49-8.45 (m, 2H), 8.24 (d, *J=* 1.8 Hz, 1H), 7.91-7.85 (m, 1H), 7.71-7.62 (m, 4H), 7.52-7.45 (m, 2H), 6.83 (t, *J* = 53.7 Hz, 1H), 2.11-2.04 (m, 2H), 1.84-1.76 ppm (m, 2H). ¹⁹F NMR (377 MHz, DMSO-d₆) δ -66.91 (s, 3F), -111.54 (s, 2F). LCMS (ES⁺) *m*/*z* calculated for C₂₄H₁₇F₅N₆O₂ 516.42, found 517 (M+H)⁺. HPLC *t*_{R} 1.80 min.

### Example 31: (3-(3-Chloro-4-(4,6-dimethylpyrimidin-5-yl)benzyl)-1,2,3-oxadiazol-3-ium-5-yl)((2-(trifluoromethyl)pyridin-4-yl)carbamoyl)amide.

The title compound was obtained as a white powder (3.3 mg, 8%). ¹H NMR (400 MHz, DMSO-*d₆*) δ 10.20 (s, 1H), 8.96 (s, 1H), 8.48-8.45 (m, 2H), 8.22 (d, *J =* 1.8 Hz, 1H), 7.96 (d, *J =* 1.5 Hz, 1H), 7.72-7.68 (m, 2H), 7.53 (d, *J =* 7.9 Hz, 1H), 5.92 (s, 2H), 2.14 (s, 6H). ¹⁹F NMR (377 MHz, DMSO-*d*₆) δ -66.93 (s, 3F). LCMS (ES⁺) *m*/*z* calculated for C₂₄H₁₇ClF₃N₇O₂ 503.86, found 504 (M+H)⁺. HPLC *t*_{R} 1.52 min.

### Example 32: (3-(1-(4-(2-(Difluoromethyl)pyridin-3-yl)phenyl)-2-hydroxyethyl)-1,2,3-oxadiazol-3-ium-5-yl)((2-(trifluoromethyl)pyridin-4-yl)carbamoyl)-amide.

The title compound was obtained as a white powder (3.4 mg, 8%). ¹H NMR (400 MHz, DMSO-*d₆*) δ 10.19 (s, 1H), 8.79-8.72 (m, 1H), 8.52 (s, 1H), 8.46 (d, *J =* 5.5 Hz, 1H), 8.24 (d, *J =* 1.8 Hz, 1H), 7.89 (dd, *J* = 0.9, 7.9 Hz, 1H), 7.77 (d, *J=* 8.3 Hz, 2H), 7.71-7.65 (m, 2H), 7.51 (d, *J* = 8.3 Hz, 2H), 6.99-6.67 (m, 1H), 6.17 (dd, *J* = 4.3, 9.4 Hz, 1H), 5.76 (t, *J* = 5.4 Hz, 1H), 4.58 (ddd, *J* = 5.7, 9.5, 12.0 Hz, 1H), 4.14 (td, *J =* 4.8, 12.1 Hz, 1H). ¹⁹F NMR (377 MHz, DMSO-*d*₆) δ -66.91 (s, 3F), -111.54 (s, 2F). LCMS (ES⁺) *m*/*z* calculated for C₂₃H₁₇F₅N₆O₃ 520.41, found 521 (M+H)⁺. HPLC *t*_{R} 1.55 min.

### Example 33: (3-(1-(4-(4-Chloro-1-methyl-1H-pyrazol-5-yl)phenyl)-2-hydroxyethyl)-1,2,3-oxadiazol-3-ium-5-yl)((2-(trifluoromethyl)pyridin-4-yl)carbamoyl)-amide.

The title compound was obtained as a white powder (1.1 mg, 2%). ¹H NMR (400 MHz, DMSO-*d₆*) δ 10.20 (br s, 1H), 8.52 (s, 1H), 8.46 (d, *J =* 5.6 Hz, 1H), 8.24 (d, *J =* 1.9 Hz, 1H), 7.80 (d, *J =* 8.4 Hz, 2H), 7.70 - 7.66 (m, 2H), 7.63 (d, *J* = 8.4 Hz, 2H), 6.18 (dd, *J* = 4.3, 9.2 Hz, 1H), 4.56 (dd, *J* = 9.4, 12.0 Hz, 1H), 4.16 (dd, J = 4.4, 12.1 Hz, 1H), 3.78 (s, 3H).¹⁹F NMR (377 MHz, DMSO-*d₆*) δ -66.91 (s, 3F). LCMS (ES⁺) *m*/*z* calculated for C₂₁H₁₇ClF₃N₇O₃ 507.85, found 508 (M+H)⁺. HPLC *t*_{R} 1.60 min.

### Example 39: (3-(4-(1-(2-((tert-Butyldimethylsilyl)oxy)-2-methylpropyl)-4-chloro-1H-pyrazol-5-yl)benzyl)-1,2,3-oxadiazol-3-ium-5-yl)((2-(trifluoromethyl)pyridin-4-yl)carbamoyl)amide.

The title compound was obtained as a white powder (7.2 mg, 19%). ¹H NMR (400 MHz, DMSO-*d₆*) δ 10.18 (s, 1H), 8.45 (d, *J =* 5.5 Hz, 1H), 8.34 (s, 1H), 8.22 (d, *J =* 1.6 Hz, 1H), 7.79-7.73 (m, 3H), 7.68-7.59 (m, 3H), 5.91 (s, 2H), 4.03 (s, 2H), 1.05 (s, 6H), 0.63 (s, 9H), -0.16 (s, 6H). ¹⁹F NMR (377 MHz, DMSO-d₆) δ -66.94 (s, 3F). LCMS (ES⁺) *m*/*z* calculated for C₂₉H₃₅ClF₃N₇O₃Si 649.22, found 650 (M+H)⁺. HPLC *t*_{R} 1.61 min (Method 3).

### Example 42: (3-(4-(4-Chloro-1-(tetrahydro-2H-pyran-4-yl)-1H-pyrazol-5-yl)benzyl)-1,2,3-oxadiazol-3-ium-5-yl)((2-(trifluoromethyl)pyridin-4-yl)carbamoyl)amide.

The title compound was obtained as a white powder (11.0 mg, 18%). ¹H NMR (400 MHz, DMSO-*d₆*) δ 10.20 (s, 1H), 8.49-8.44 (m, 1H), 8.43 (s, 1H), 8.21 (d, *J=* 1.8 Hz, 1H), 7.78-7.75 (m, 2H), 7.70 (dd, *J* = 1.8, 5.6 Hz, 1H), 7.57 (d, *J* = 8.1 Hz, 2H), 7.41 (s, 1H), 5.93 (s, 2H), 4.29-4.20 (m, 1H), 3.88 (br dd, *J=* 3.8, 11.2 Hz, 2H), 3.28 (m, 2H), 2.12-1.98 (m, 2H), 1.79 (br d, *J=* 10.5 Hz, 2H). ¹⁹F NMR (377 MHz, DMSO-d₆) δ -66.93 (s, 3F). LCMS (ES⁺) *m*/*z* calculated for C₂₄H₂₁ClF₃N₇O₃ 547.92, found 548 (M+H)⁺. HPLC t_{R} 1.75 min.

### Example 43: (3-(4-(4-Chloro-1-(tetrahydrofuran-3-yl)-1H-pyrazol-5-yl)benzyl)-1,2,3-oxadiazol-3-ium-5-yl)((2-(trifluoromethyl)pyridin-4-yl)carbamoyl)amide.

The title compound was obtained as a white powder (16.8 mg, 24%). ¹H NMR (400 MHz, DMSO-*d₆*) δ 10.20 (s, 1H), 8.47 (d, *J=* 5.6 Hz, 1H), 8.43 (s, 1H), 8.21 (d, *J=* 1.8 Hz, 1H), 7.78-7.74 (m, 3H), 7.70 (dd, *J=* 1.7, 5.7 Hz, 1H), 7.56 (d, *J* = 8.1 Hz, 2H), 5.93 (s, 2H), 4.89-4.82 (m, 1H), 4.00-3.92 (m, 2H), 3.85-3.74 (m, 2H), 2.24 (q, *J* = 6.8 Hz, 2H). ¹⁹F NMR (377 MHz, DMSO-d₆) δ - 66.93 (s, 3F). LCMS (ES⁺) *m*/*z* calculated for C₂₃H₁₉ClF₃N₇O₃ 533.90, found 534 (M+H)⁺. HPLC t_{R} 1.73 min.

### Examples 2, 3-5, 7, 10, 12-13, 15, 18-21, 23-24, 29-30, 34, 38, 41, 44-46, 48-49 were prepared using the synthetic procedure described for the synthesis of Example 1 in Step 3 Scheme 7 using the appropriate starting materials.

### Example 2: (3-(4-(1,4-Dimethyl-1H-imidazol-5-yl)benzyl)-1,2,3-oxadiazol-3-ium-5-yl)((2-(trifluoromethyl)pyridin-4-yl)carbamoyl)amide (trifluoroacetate salt).

The title compound was obtained as a white powder (2.6 mg, 10%). ¹H NMR (400 MHz, DMSO-*d₆*) δ 10.19 (s, 1H), 9.08 (s, 1H), 8.47 (d, *J=* 5.6 Hz, 1H), 8.41 (s, 1H), 8.22 (d, *J =* 1.8 Hz, 1H), 7.81-7.77 (m, *J* = 8.3 Hz, 2H), 7.68 (dd, *J=* 1.9, 5.6 Hz, 1H), 7.66-7.62 (m, *J* = 8.4 Hz, 2H), 5.93 (s, 2H), 3.75 (s, 3H), 2.25 (s, 3H). ¹⁹F NMR (377 MHz, DMSO-d₆) δ -66.93 (s, 3F), -73.88 (s, 3F). LCMS (ES⁺) *m*/*z* calculated for C₂₁H₁₈F₃N₇O₂ 457.41, found 458 (M+H)⁺. HPLC *t*_{R} 1.02 min.

### Example 3: (3-(4-(1-Cyclopropyl-4-methoxy-1H-pyrazol-5-yl)benzyl)-1,2,3-oxadiazol-3-ium-5-yl)((2-(trifluoromethyl)pyridin-4-yl)carbamoyl)amide.

The title compound was obtained as a white powder (4.2 mg, 12%). ¹H NMR (400 MHz, DMSO-*d₆*) δ 10.18 (s, 1H), 8.46 (d, *J* = 5.5 Hz, 1H), 8.36 (s, 1H), 8.23-8.18 (m, 1H), 7.69 (s, 5H), 7.41 (s, 1H), 5.88 (s, 2H), 3.71 (s, 3H), 3.68 (br dd, *J =* 3.7, 7.3 Hz, 1H), 3.31-3.29 (m, 2H), 0.98-0.85 (m, 4H). ¹⁹F NMR (377 MHz, DMSO-d₆) δ -66.93 (s, 3F). LCMS (ES⁺) *m*/*z* calculated for C₂₃H₂₀F₃N₇O₃ 499.45, found 500 (M+H)⁺. HPLC *t*_{R} 1.66 min.

### Example 4: (3-(4-(4-Methoxy-1-methyl-1H-pyrazol-5-yl)benzyl)-1,2,3-oxadiazol-3-ium-5-yl)((2-(trifluoromethyl)pyridin-4-yl)carbamoyl)amide (trifluoroacetate salt).

The title compound was obtained as a white powder (4.0 mg, 11%). ¹H NMR (400 MHz, DMSO-*d₆*) δ 10.18 (s, 1H), 8.46 (d, *J* = 5.6 Hz, 1H), 8.36 (s, 1H), 8.20 (d, *J* = 1.9 Hz, 1H), 7.72-7.66 (m, 3H), 7.61-7.55 (m, 2H), 7.46-7.41 (m, 2H), 5.88 (s, 2H), 3.79 (s, 3H), 3.71 (s, 3H). ¹⁹F NMR (377 MHz, DMSO-*d*₆) δ -66.93 (s, 3F) -74.55 (s, 3F). LCMS (ES⁺) *m*/*z* calculated for C₂₁H₁₈F₃N₇O₃ 473.41, found 474 (M+H)⁺. HPLC t_{R} 1.51 min.

### Example 5: (3-(4-(1-Cyclopropyl-4-methyl-1H-pyrazol-5-yl)benzyl)-1,2,3-oxadiazol-3-ium-5-yl)((2-(trifluoromethyl)pyridin-4-yl)carbamoyl)amide (trifluoroacetate salt).

The title compound was obtained as a white powder (4.4 mg, 11%). ¹H NMR (400 MHz, DMSO-*d₆*) δ 10.19 (s, 1H), 8.46 (d, *J* = 5.6 Hz, 1H), 8.40 (s, 1H), 8.21 (d, *J* = 1.8 Hz, 1H), 7.74-7.67 (m, 3H), 7.59 (d, *J=* 7.7 Hz, 2H), 7.30 (s, 1H), 5.91 (s, 2H), 3.58-3.53 (m, 1H), 1.96 (s, 3H), 0.94-0.88 (m, 2H), 0.86-0.78 (m, 2H). ¹⁹F NMR (377 MHz, DMSO-d₆) δ -66.93 (s, 3F) -74.55 (s, 3F). LCMS (ES⁺) *m*/*z* calculated for C₂₃H₂₀F₃N₇O₂ 483.45, found 484 (M+H)⁺. HPLC *t*_{R} 1.69 min.

### Example 7: (3-(4-(1-Cyclobutyl-4-methyl-1H-imidazol-5-yl)benzyl)-1,2,3-oxadiazol-3-ium-5-yl)((2-(trifluoromethyl)pyridin-4-yl)carbamoyl)amide (trifluoroacetate salt).

The title compound was obtained as a white powder (3.0 mg, 12%). ¹H NMR (400 MHz, DMSO-*d₆*) δ 10.20 (s, 1H), 9.31 (s, 1H), 8.47 (d, *J =* 5.6 Hz, 1H), 8.39 (s, 1H), 8.22 (d, *J =* 1.8 Hz, 1H), 7.79-7.75 (m, *J* = 8.3 Hz, 2H), 7.68 (dd, *J =* 2.1, 5.7 Hz, 1H), 7.57-7.53 (m, *J* = 8.3 Hz, 2H), 5.93 (s, 2H), 4.71-4.67 (m, 1H), 2.41-2.31 (m, 2H), 2.24-2.21 (m, 2H), 2.20 (s, 3H), 1.75-1.72 (m, 2H). ¹⁹F NMR (377 MHz, DMSO-d₆) δ -66.93 (s, 3F) -73.61 (s, 6F). LCMS (ES⁺) *m*/*z* calculated for C₂₄H₂₂F₃N₇O₂ 497.47, found 498 (M+H)⁺. HPLC *t*_{R} 1.10 min.

### Example 10: (3-(4-(2-(Difluoromethyl)pyridin-3-yl)benzyl)-1,2,3-oxadiazol-3-ium-5-yl)((2-(trifluoromethyl)pyridin-4-yl)carbamoyl)amide.

The title compound was obtained as a white powder (21.0 mg, 56%). ¹H NMR (400 MHz, DMSO-*d₆*) δ 10.19 (s, 1H), 8.75 (dd, *J* = 1.4, 4.6 Hz, 1H), 8.47 (d, *J* = 5.6 Hz, 1H), 8.39 (s, 1H), 8.21 (d, *J=* 1.8 Hz, 1H), 7.90 (d, *J=* 7.8 Hz, 1H), 7.75-7.66 (m, 4H), 7.51 (d, *J =* 8.1 Hz, 2H), 7.01-6.67 (m, 1H), 5.92 (s, 2H). ¹⁹F NMR (377 MHz, DMSO-d₆) δ -66.92 (s, 3F), -111.52 (s, 2F). LCMS (ES⁺) *m*/*z* calculated for C₂₂H₁₅F₅N₆O₂ 490.39, found 491 (M+H)⁺. HPLC *t*_{R} 1.64 min.

### Example 12: (3-(4-(3-(Trifluoromethyl)pyridin-2-yl)benzyl)-1,2,3-oxadiazol-3-ium-5-yl)((2-(trifluoromethyl)pyridin-4-yl)carbamoyl)amide.

The title compound was obtained as a white powder (24.5 mg, 49%). ¹H NMR (400 MHz, DMSO-*d₆*) δ 10.19 (s, 1H), 8.92 (dd, *J =* 0.9, 4.8 Hz, 1H), 8.46 (d, *J* = 5.6 Hz, 1H), 8.37 (s, 1H), 8.34 (dd, *J* = 1.2, 8.1 Hz, 1H), 8.20 (d, *J =* 1.9 Hz, 1H), 7.72-7.66 (m, 4H), 7.55 (d, *J* = 8.1 Hz, 2H), 5.93 (s, 2H). ¹⁹F NMR (377 MHz, DMSO-d₆) δ -56.00 (s, 3F), -66.92 (s, 3F). LCMS (ES⁺) *m*/*z* calculated for C₂₂H₁₄F₆N₆O₂ 508.38, found 509 (M+H)⁺. HPLC *t*_{R} 1.69 min.

### Example 13: (3-(4-(4-Chloro-2-(trifluoromethyl)pyridin-3-yl)benzyl)-1,2,3-oxadiazol-3-ium-5-yl)((2-(trifluoromethyl)pyridin-4-yl)carbamoyl)amide (trifluoroacetate salt).

The title compound was obtained as a white powder (7.8 mg, 14%). ¹H NMR (400 MHz, DMSO-*d₆*) δ 10.20 (s, 1H), 8.76 (d, *J* = 5.3 Hz, 1H), 8.47 (d, *J* = 5.5 Hz, 1H), 8.41 (s, 1H), 8.21 (d, *J* = 1.9 Hz, 1H), 8.06 (d, *J* = 5.1 Hz, 1H), 7.69 (d, *J =* 8.4 Hz, 3H), 7.44 (d, *J =* 8.1 Hz, 2H), 5.93 (s, 2H). ¹⁹F NMR (377 MHz, DMSO-*d*₆) δ -60.37 (s, 3F), -66.92 (s, 3F), -74.54 (s, 3F). LCMS (ES⁺) *m*/*z* calculated for C₂₂H₁₃ClF₆N₆O₂ 542.82, found 543 (M+H)⁺. HPLC *t*_{R} 1.80 min.

### Example 15: (3-(4-(4-(Trifluoromethyl)pyridin-3-yl)benzyl)-1,2,3-oxadiazol-3-ium-5-yl)((2-(trifluoromethyl)pyridin-4-yl)carbamoyl)amide.

The title compound was obtained as a white powder (5.5 mg, 12%). ¹H NMR (400 MHz, DMSO-*d₆*) δ 10.19 (s, 1H), 8.90 (d, *J =* 5.1 Hz, 1H), 8.71 (s, 1H), 8.47 (d, *J=* 5.6 Hz, 1H), 8.39 (s, 1H), 8.21 (d, *J* = 1.8 Hz, 1H), 7.88 (d, *J* = 5.3 Hz, 1H), 7.70 (d, *J* = 8.1 Hz, 3H), 7.50 (d, *J* = 8.1 Hz, 2H), 5.92 (s, 2H). ¹⁹F NMR (377 MHz, DMSO-d₆) δ -57.95 (s, 3F), -66.92 (s, 3F). LCMS (ES⁺) *m*/*z* calculated for C₂₂H₁₄F₆N₆O₂ 508.38, found 509 (M+H)⁺. HPLC *t*_{R} 1.74 min.

### Example 18: (3-(4-(6-Oxo-2-(trifluoromethyl)-1,6-dihydropyridin-3-yl)benzyl)-1,2,3-oxadiazol-3-ium-5-yl)((2-(trifluoromethyl)pyridin-4-yl)carbamoyl)amide (trifluoroacetate salt).

The title compound was obtained as a white powder (5.5 mg, 12%). ¹H NMR (400 MHz, DMSO-*d₆*) δ 11.75 (br s, 1H), 10.18 (s, 1H), 8.46 (d, *J =* 5.6 Hz, 1H), 8.36 (s, 1H), 8.20 (d, *J =* 1.8 Hz, 1H), 7.75-7.67 (m, 2H), 7.65 (d, *J* = 8.4 Hz, 2H), 7.41 (d, *J* = 8.1 Hz, 2H), 6.99 (d, *J* = 8.4 Hz, 1H), 5.89 (s, 2H). ¹⁹F NMR (377 MHz, DMSO-*d*₆) δ -59.80 (s, 3F), -66.92 (s, 3F), -74.31 (s, 3F). LCMS (ES⁺) *m*/*z* calculated for C₂₂H₁₄F₆N₆O₃ 524.38, found 525 (M+H)⁺. HPLC *t*_{R} 1.52 min.

### Example 19: (3-(4-(2-Methoxy-4-(trifluoromethyl)pyridin-3-yl)benzyl)-1,2,3-oxadiazol-3-ium-5-yl)((2-(trifluoromethyl)pyridin-4-yl)carbamoyl)amide (trifluoroacetate salt).

The title compound was obtained as a white powder (1.6 mg, 4%). ¹H NMR (400 MHz, DMSO-*d₆*) δ 10.20 (s, 1H), 8.47 (t, *J* = 5.5 Hz, 2H), 8.40 (s, 1H), 8.22 (d, *J =* 1.8 Hz, 1H), 7.70 (dd, *J* = 2.0, 5.9 Hz, 1H), 7.65-7.61 (m, *J =* 8.3 Hz, 2H), 7.44 (d, *J* = 5.4 Hz, 1H), 7.39-7.34 (m, *J =* 8.1 Hz, 2H), 5.89 (s, 2H), 3.68 (s, 3H). ¹⁹F NMR (377 MHz, DMSO-d₆) δ -57.79 (s, 3F), -66.92 (s, 3F), -74.02 (s, 3F). LCMS (ES⁺) *m*/*z* calculated for C₂₃H₁₆F₆N₆O₃ 538.40, found 539 (M+H)⁺. HPLC *t*_{R} 1.93 min.

### Example 20: (3-(4-(4-(Trifluoromethyl)pyridin-2-yl)benzyl)-1,2,3-oxadiazol-3-ium-5-yl)((2-(trifluoromethyl)pyridin-4-yl)carbamoyl)amide (trifluoroacetate salt).

The title compound was obtained as a white powder (2.6 mg, 6%). ¹H NMR (400 MHz, DMSO-*d₆*) δ 10.18 (s, 1H), 8.97 (d, *J* = 5.1 Hz, 1H), 8.46 (d, *J* = 5.6 Hz, 1H), 8.35 (s, 2H), 8.29 (d, *J* = 8.3 Hz, 2H), 8.20 (d, *J =* 1.8 Hz, 1H), 7.78-7.72 (m, 3H), 7.69 (dd, *J =* 1.7, 5.6 Hz, 1H), 5.92 (s, 2H). ¹⁹F NMR (377 MHz, DMSO-d₆) δ -63.22 (s, 3F), -66.92 (s, 3F), -74.72 (s, 3F). LCMS (ES⁺) *m*/*z* calculated for C₂₂H₁₄F₆N₆O₂ 508.38, found 509 (M+H)⁺. HPLC *t*_{R} 1.79 min.

### Example 21: (3-((2'-(Trifluoromethyl)-[1,1'-biphenyl]-4-yl)methyl)-1,2,3-oxadiazol-3-ium-5-yl)((2-(trifluoromethyl)pyridin-4-yl)carbamoyl)amide (trifluoroacetate salt).

The title compound was obtained as a white powder (6.5 mg, 13%). ¹H NMR (400 MHz, DMSO-*d₆*) δ 10.19 (s, 1H), 8.46 (d, *J* = 5.6 Hz, 1H), 8.38 (s, 1H), 8.21 (d, *J* = 1.8 Hz, 1H), 7.85 (d, *J* = 7.5 Hz, 1H), 7.73 (d, *J* = 7.6 Hz, 1H), 7.70 (dd, *J =* 1.9, 5.6 Hz, 1H), 7.65 (d, *J* = 8.3 Hz, 3H), 7.45-7.39 (m, 3H), 5.90 (s, 2H). ¹⁹F NMR (377 MHz, DMSO-*d*₆) δ -55.29 (s, 3F), -66.92 (s, 3F), -74.58 (s, 3F). LCMS (ES⁺) *m*/*z* calculated for C₂₃H₁₅F₆N₅O₂ 509.38, found 510 (M+H)⁺. HPLC *t*_{R} 2.02 min.

### Example 23: (3-((2'-(Difluoromethyl)-[1,1'-biphenyl]-4-yl)methyl)-1,2,3-oxadiazol-3-ium-5-yl)((2-(trifluoromethyl)pyridin-4-yl)carbamoyl)amide (trifluoacetate salt).

The title compound was obtained as a white powder (23.7 mg, 47%). ¹H NMR (400 MHz, DMSO-*d₆*) δ 10.19 (s, 1H), 8.47 (d, *J* = 5.5 Hz, 1H), 8.39 (s, 1H), 8.21 (d, *J =* 1.8 Hz, 1H), 7.75 (d, *J* = 7.3 Hz, 1H), 7.70 (d, *J=* 8.0 Hz, 3H), 7.66-7.56 (m, 2H), 7.46 (d, *J =* 8.1 Hz, 2H), 7.41 (d, *J=* 7.6 Hz, 1H), 6.82 (t, *J =* 54.0 Hz, 1H), 5.91 (s, 2H). ¹⁹F NMR (377 MHz, DMSO-d₆) δ -66.92 (s, 3F), -74.58 (s, 3F), -107.20 (s, 2F). LCMS (ES⁺) *m*/*z* calculated for C₂₃H₁₆F₅N₅O₂ 489.40, found 490 (M+H)⁺. HPLC *t*_{R} 1.95 min.

### Example 24: (3-(4-(2-(Difluoromethyl)-4-methylpyridin-3-yl)benzyl)-1,2,3-oxadiazol-3-ium-5-yl)((2-(trifluoromethyl)pyridin-4-yl)carbamoyl)amide.

The title compound was obtained as a white powder (18.0 mg, 27%). ¹H NMR (400 MHz, DMSO-*d₆*) δ 10.19 (s, 1H), 8.59 (d, *J* = 4.9 Hz, 1H), 8.47 (d, *J* = 5.6 Hz, 1H), 8.42 (s, 1H), 8.21 (d, *J* = 1.9 Hz, 01H), 7.73-7.68 (m, 3H), 7.56 (d, *J* = 4.9 Hz, 1H), 7.37 (d, *J =* 8.1 Hz, 2H), 6.54 (d, *J* = 59 Hz, 1H), 5.92 (s, 2H), 2.05 (s, 3H). ¹⁹F NMR (377 MHz, DMSO-d₆) δ -66.92 (s, 3F), -112.07 (s, 2F). LCMS (ES⁺) *m*/*z* calculated for C₂₃H₁₇F₅N₆O₂ 504.41, found 505 (M+H)⁺. HPLC *t*_{R} 1.69 min.

### Example 29: (3-(4-(3-(Difluoromethyl)pyridin-2-yl)benzyl)-1,2,3-oxadiazol-3-ium-5-yl)((2-(trifluoromethyl)pyridin-4-yl)carbamoyl)amide.

The title compound was obtained as a white powder (25.0 mg, 62%). ¹H NMR (400 MHz, DMSO-*d₆*) δ 10.19 (s, 1H), 8.84 (d, *J* = 4.9 Hz, 1H), 8.46 (d, *J* = 5.6 Hz, 1H), 8.39 (s, 1H), 8.18-8.23 (m, 2H), 7.68-7.75 (m, 3H), 7.59-7.66 (m, 3H), 7.00 (t, *J =* 54.1 Hz, 1H), 5.93 ppm (s, 2H). ¹⁹F NMR (377 MHz, DMSO-*d*₆) δ -66.92 (s, 3F), -108.37 (s, 2F). LCMS (ES⁺) *m*/*z* calculated for C₂₂H₁₅F₅N₆O₂ 490.30, found 491 (M+H)⁺. HPLC *t*_{R} 1.68 min.

### Example 30: (3-(2-Chloro-4-(2-(difluoromethyl)pyridin-3-yl)benzyl)-1,2,3-oxadiazol-3-ium-5-yl)((2-(trifluoromethyl)pyridin-4-yl)carbamoyl)amide.

The title compound was obtained as a white powder (25.4 mg, 39%). ¹H NMR (400 MHz, DMSO-*d₆*) δ 10.20 (s, 1H), 8.78 (dd, *J =* 1.6, 4.7 Hz, 1H), 8.47 (d, *J* = 5.6 Hz, 1H), 8.31 (s, 1H), 8.19 (d, *J =* 1.8 Hz, 1H), 7.98-7.93 (m, 1H), 7.83 (d, J = 8.0 Hz, 1H), 7.72-7.66 (m, 3H), 7.51 (dd, *J =* 1.8, 8.0 Hz, 1H), 6.90 (t, *J =* 52.1 Hz, 1H), 6.05 (s, 2H). ¹⁹F NMR (377 MHz, DMSO-*d*₆) δ -66.91 (s, 3F), -111.48 (s, 2F). LCMS (ES⁺) *m*/*z* calculated for C₂₂H₁₄ClF₅N₆O₂ 524.83, found 525 (M+H)⁺. HPLC *t*_{R} 1.74 min.

### Example 34: (3-(4-(3-(Trifluoromethyl)pyridin-4-yl)benzyl)-1,2,3-oxadiazol-3-ium-5-yl)((2-(trifluoromethyl)pyridin-4-yl)carbamoyl)amide.

The title compound was obtained as a white powder (25.0 mg, 62%). ¹H NMR (400 MHz, DMSO-*d₆*) δ 10.20 (s, 1H), 8.78 (dd, *J =* 1.6, 4.7 Hz, 1H), 8.47 (d, *J* = 5.6 Hz, 1H), 8.31 (s, 1H), 8.19 (d, *J =* 1.8 Hz, 1H), 7.98-7.93 (m, 1H), 7.83 (d, *J* = 8.0 Hz, 1H), 7.72-7.66 (m, 3H), 7.51 (dd, *J =* 1.8, 8.0 Hz, 1H), 6.90 (t, *J =* 52.1 Hz, 1H), 6.05 (s, 2H). ¹⁹F NMR (377 MHz, DMSO-*d*₆) δ -55.43 (s, 3F), -66.93 (s, 3F). LCMS (ES⁺) *m*/*z* calculated for C₂₂H₁₄ClF₆N₆O₂ 508.38, found 509 (M+H)⁺. HPLC *t*_{R} 1.79 min.

### Example 38: (3-(4-(3-Hydroxypyridin-2-yl)benzyl)-1,2,3-oxadiazol-3-ium-5-yl)((2-(trifluoromethyl)pyridin-4-yl)carbamoyl)amide.

The title compound was obtained as a white powder (10.9 mg, 29%). ¹H NMR (400 MHz, DMSO-*d₆*) δ 10.68 (br s, 1H), 10.18 (s, 1H), 8.46 (d, *J =* 5.6 Hz, 1H), 8.33 (s, 1H), 8.24-8.18 (m, 2H), 8.04 (d, *J =* 8.3 Hz, 2H), 7.71-7.64 (m, 3H), 7.51 (br d, *J =* 7.8 Hz, 1H), 7.43-7.33 (m, 1H), 5.89 (s, 2H). ¹⁹F NMR (377 MHz, DMSO-*d*₆) δ -66.92 (s, 3F), -74.57 (s, 3F). LCMS (ES⁺) *m*/*z* calculated for C₂₁H₁₅F₃N₆O₂ 456.38, found 457 (M+H)⁺. HPLC *t*_{R} 1.15 min.

### Example 41: (3-(4-(4-Cyclopropyl-6-(trifluoromethyl)pyrimidin-5-yl)benzyl)-1,2,3-oxadiazol-3-ium-5-yl)((2-(trifluoromethyl)pyridin-4-yl)carbamoyl)amide.

The title compound was obtained as a white powder (36.9 mg, 47%). ¹H NMR (400 MHz, DMSO-*d₆*) δ 10.20 (s, 1H), 9.21 (s, 1H), 8.47 (d, *J =* 5.6 Hz, 1H), 8.41 (s, 1H), 8.21 (d, *J =* 1.6 Hz, 1H), 7.73 (d, *J* = 8.1 Hz, 2H), 7.69 (dd, *J* = 1.8, 5.5 Hz, 1H), 7.52 (d, *J* = 8.1 Hz, 2H), 5.93 (s, 2H), 1.66-1.59 (m, 1H), 1.17-1.09 (m, 2H), 1.09-1.01 (m, 2H). ¹⁹F NMR (377 MHz, DMSO-*d*₆) δ - 62.26 (s, 3F), -66.93 (s, 3F). LCMS (ES⁺) *m*/*z* calculated for C₂₄H₁₇F₆N₇O₂ 549.13, found 550 (M+H)⁺. HPLC *t*_{R} 1.96 min.

### Example 44: (3-(4-(3-(Trifluoromethyl)pyrazin-2-yl)benzyl)-1,2,3-oxadiazol-3-ium-5-yl)((2-(trifluoromethyl)pyridin-4-yl)carbamoyl)amide.

The title compound was obtained as a white powder (3.0 mg, 6%). ¹H NMR (400 MHz, DMSO-*d₆*): δ 10.19 (s, 1H), 9.07 (d, *J* = 2.0 Hz, 1H), 8.90 (d, *J* = 2.0 Hz, 1H), 8.46 (d, *J* = 5.5 Hz, 1H), 8.38 (s, 1H), 8.21 (s, 1H), 7.757.68 (m, 3H), 7.68-7.63 (m, 2H), 5.94 (s, 2H). ¹⁹F NMR (377 MHz, DMSO-*d*₆) δ -62.26 (s, 3F), -66.93 (s, 3F). LCMS (ES⁺) *m*/*z* calculated for C₂₁H₁₃F₆N₇O₂ 509.37, found 510 (M+H)⁺. HPLC *t*_{R} 1.71 min.

### Example 45: (3-(4-(2-(1,1-Difluoro-2-hydroxyethyl)pyridin-3-yl)benzyl)-1,2,3-oxadiazol-3-ium-5-yl)((2-(trifluoromethyl)pyridin-4-yl)carbamoyl)amide.

The title compound was obtained as a white powder (8.7 mg, 37%). ¹H NMR (400 MHz, DMSO-*d₆*) δ 10.19 (s, 1H), 8.70 (d, *J =* 4.5 Hz, 1H), 8.47 (d, *J* = 5.5 Hz, 1H), 8.37 (s, 1H), 8.21 (d, *J* = 1.5 Hz, 1H), 7.77 (dd, *J =* 1.5, 8.0 Hz, 1H), 7.70 (dd, *J =* 1.8, 5.5 Hz, 1H), 7.67-7.59 (m, 3H), 7.45 (d, *J* = 8.0 Hz, 2H), 5.90 (s, 2H), 5.43 (t, *J =* 6.4 Hz, 1H), 4.04 (dt, *J =* 6.5, 14.4 Hz, 2H). ¹⁹F NMR (377 MHz, DMSO-*d*₆) δ -66.93 (s, 3F), -98.64 (s, 2F). LCMS (ES⁺) *m*/*z* calculated for C₂₃H₁₇F₅N₆O₃ 520.42, found 521 (M+H)⁺. HPLC *t*_{R} 1.52 min.

### Example 46: (3-(4-(4-(Difluoromethyl)pyridin-3-yl)benzyl)-1,2,3-oxadiazol-3-ium-5-yl)((2-(trifluoromethyl)pyridin-4-yl)carbamoyl)amide.

The title compound was obtained as a white powder (10.4 mg, 58%). ¹H NMR (400 MHz, DMSO-*d₆*) δ 10.19 (s, 1H), 8.81 (d, *J =* 5.1 Hz, 1H), 8.66 (s, 1H), 8.47 (d, *J =* 5.5 Hz, 1H), 8.41 (s, 1H), 8.21 (d, *J =* 1.5 Hz, 1H), 7.76-7.68 (m, 4H), 7.55 (d, *J =* 8.1 Hz, 2H), 7.00 (t, *J =* 53.9 Hz, 1H), 5.92 (s, 2H). ¹⁹F NMR (377 MHz, DMSO-*d*₆) δ -66.92 (s, 3F), -108.36 (s, 2F). LCMS (ES⁺) m/z calculated for C₂₂H₁₅F₅N₆O₂ 490.39, found 491 (M+H)⁺. HPLC *t*_{R} 1.70 min.

### Example 48: (3-(4-(3-(Difluoromethyl)pyridin-4-yl)benzyl)-1,2,3-oxadiazol-3-ium-5-yl)((2-(trifluoromethyl)pyridin-4-yl)carbamoyl)amide.

The title compound was obtained as a white powder (4.5 mg, 47%). ¹H NMR (400 MHz, DMSO-*d₆*) δ 8.21 (d, *J =* 1.8 Hz, 1H), 7.80-7.63 (m, 3H), 7.57-7.47 (m, 3H), 7.04 (t, *J =* 53.9 Hz, 1H), 5.92 (s, 2H). ¹⁹F NMR (377 MHz, DMSO-*d*₆) δ -66.92 (s, 3F), -109.10 (s, 2F). LCMS (ES⁺) m/z calculated for C₂₂H₁₅F₅N₆O₂ 490.39, found 491 (M+H)⁺. HPLC *t*_{R} 1.66 min.

### Example 49: (3-(4-(6-(Methoxycarbonyl)-2-methylpyridin-3-yl)benzyl)-1,2,3-oxadiazol-3-ium-5-yl)((2-(trifluoromethyl)pyridin-4-yl)carbamoyl)amide.

The title compound was obtained as a white powder (30.0 mg, 29%). ¹H NMR (400 MHz, DMSO-*d₆*) δ 10.19 (s, 1H), 8.46 (d, *J =* 5.6 Hz, 1H), 8.38 (s, 1H), 8.21 (d, *J =* 1.8 Hz, 1H), 7.97 (d, *J* = 7.9 Hz, 1H), 7.83 (d, *J=* 8.0 Hz, 1H), 7.74-7.67 (m, 3H), 7.57 (d, *J=* 8.1 Hz, 2H), 5.91 (s, 2H), 3.90 (s, 3H), 2.50-2.49 (m, 3H). ¹⁹F NMR (377 MHz, DMSO-*d*₆) δ -66.92 (s, 3F). LCMS (ES⁺) *m*/*z* calculated for C₂₄H₁₉F₃N₆O₄ 512.40, found 513 (M+H)⁺. HPLC *t*_{R} 1.66 min.

### Example 35: (3-((6-(4,6-Dimethylpyrimidin-5-yl)pyridin-3-yl)methyl)-1,2,3-oxadiazol-3-ium-5-yl)((2-(trifluoromethyl)pyridin-4-yl)carbamoyl)amide.

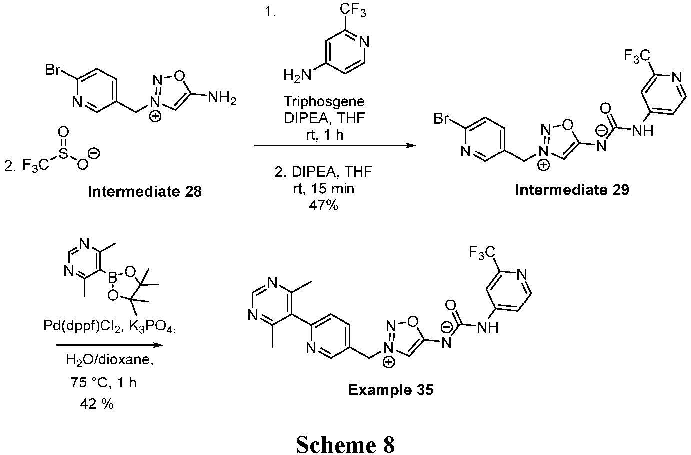

### Step 1: (3-((6-Bromopyridin-3yl)methyl)-1,2,3-oxadiazol-3-ium-5-yl) ((2-(trifluoromethyl)-pyridin-4-yl)carbamoyl)amide (Intermediate 29)

A solution of 2-(trifluoromethyl)pyridin-4-amine (155 mg, 1.0 mmol) and DIPEA (0.67 mL, 4.0 mmol) in dry THF (2.5 mL) was treated with triphosgene (200 mg, 0.67 mmol) and stirred at rt for 1 h. This solution was added dropwise to a stirring suspension of 5-amino-3-((6-bromopyridin-3-yl)methyl)-1,2,3-oxadiazol-3-ium trifluoromethanesulfinate (Intermediate **28,** 573 mg, 1.0 mmol, prepared as described in WO2023/227734 from appropriated starting materials) in dry THF (7.0 mL) followed by the addition of DIPEA (0.67 mL, 4.0 mmol) at 0 °C. The mixture was stirred at this temperature for 5 minutes before being diluted with H₂O and extracted with EtOAc (2x). The collected organic layers were washed with brine, dried over Na₂SO₄, filtered, and concentrated *in vacuo* to get a residue which was purified by flash chromatography on silica gel (eluting with 0-100% EtOAc in petroleum ether) to give the title compound as an orange solid (227 mg, 45%). ¹H NMR (400 MHz, DMSO-*d*₆) δ 10.18 (s, 1H), 8.64 (d, *J=* 2.3 Hz, 1H), 8.46 (d, *J* = 5.6 Hz, 1H), 8.39 (s, 1H), 8.21 (d, *J* = 1.8 Hz, 1H), 7.97 (dd, *J* = 8.3, 2.6 Hz, 1H), 7.77 (d, *J* = 8.3 Hz, 1H), 7.69 (dd, *J =* 5.6, 1.9 Hz, 1H), 5.87 ppm (s, 2H). LCMS (ES⁺) *m*/*z* calculated for C₁₅H₁₀BrF₃N₆O₂ 441.00-443.00, found 441 and 443 (M-H)⁻. HPLC *t*_{R} 1.52 min.

### Step 2: (3-((6-(4, 6-Dimethylpyrimidin-5-yl)pyridin-3-yl)methyl)-1,2,3-oxadiazol-3-ium-5-yl)((2-(trifluoromethyl)pyridin-4-yl)carbamoyl)amide (Example 35)

A suspension of (3-((6-bromopyridin-3-yl)methyl)-1,2,3-oxadiazol-3-ium-5-yl)((2-(trifluoromethyl)-pyridin-4-yl)carbamoyl)amide (Intermediate **29,** 50 mg, 0.1 mmol), 4,6-dimethyl-5-(4,4,5,5-tetramethyl-1,3,2-dioxaborolan-2-yl)pyrimidine (34 mg, 0.12 mmol), Pd(dppf)Cl₂ (7 mg, 0.01 mmol) and K₃PO₄ (62 mg, 0.3 mmol) in a mixture of 1,4-dioxane (0.8 mL) and H₂O (0.1 mL) was heated at 75 °C for 1 h before being diluted with EtOAc and washed with H₂O and brine. The organic layer was dried over Na₂SO₄, filtered, and concentrated *in vacuo* to get a residue which was purified by flash chromatography on C18 RP (eluting with 0-100% CH₃CN in H₂O) to give the title compound a white powder (19.0 mg, 42%). ¹H NMR (400 MHz, DMSO-*d*₆) δ 10.19 (s, 1H), 9.00-8.89 (m, 2H), 8.51-8.42 (m, 2H), 8.22 (d, *J=* 1.4 Hz, 1H), 8.17 (dd, *J* = 2.2, 8.1 Hz, 1H), 7.70 (dd, *J* = 1.5, 5.4 Hz, 1H), 7.65 (d, *J* = 8.0 Hz, 1H), 5.97 (s, 2H), 2.20 (s, 6H). ¹⁹F NMR (377 MHz, DMSO-*d*₆) δ -66.93 (s, 3F). LCMS (ES⁺) *m*/*z* calculated for C₂₁H₁₇F₃N₈O₂ 470.41, found 471 (M+H)⁺. HPLC *t*_{R} 1.24 min.

Examples **36** and **37** were prepared following the experimental protocol described in Scheme 8 using the appropriate starting materials.

### Example 36: (3-((6-(4-Chloro-1-methyl-1H-pyrazol-5-yl)pyridin-3-yl)methyl)-1,2,3-oxadiazol-3-ium-5-yl)((2-(trifluoromethyl)pyridin-4-yl)carbamoyl)amide.

The title compound was obtained as a white powder (4.1 mg, 9%). ¹H NMR (400 MHz, DMSO-*d₆*) δ 10.20 (s, 1H), 8.99 (d, *J* = 1.9 Hz, 1H), 8.49-8.44 (m, 2H), 8.23-8.19 (m, 2H), 7.86 (d, *J* = 8.1 Hz, 1H), 7.72 (s, 1H), 7.70 (dd, *J=* 1.6, 5.8 Hz, 1H), 5.97 (s, 2H), 3.97 (s, 3H). ¹⁹F NMR (377 MHz, DMSO-*d*₆) δ -66.93 (s, 3F). LCMS (ES⁺) *m*/*z* calculated for C₁₉H₁₄ClF₃N₈O₂ 478.82, found 479 (M+H)⁺. HPLC *t*_{R} 1.54 min.

### Example 37: (3-((2'-(Difluoromethyl)-[2,3'-bipyridin]-5-yl)methyl)-1,2,3-oxadiazol-3-ium-5-yl)((2-(trifluoromethyl)pyridin-4-yl)carbamoyl)amide.

The title compound was obtained as a white powder (7.2 mg, 19%). ¹H NMR (400 MHz, DMSO-*d₆*) δ 10.19 (s, 1H), 8.95 (d, J= 1.6 Hz, 1H), 8.83-8.78 (m, 1H), 8.48-8.44 (m, 2H), 8.24-8.18 (m, 2H), 8.13 (d, *J=* 7.9 Hz, 1H), 7.84 (d, *J=* 8.0 Hz, 1H), 7.75-7.68 (m, 2H), 7.39-7.02 (t, *J=* 70.0 Hz, 1H), 5.98 (s, 2H). ¹⁹F NMR (377 MHz, DMSO-*d*₆) δ -66.93 (s, 3F), -113.78 (s, 2F). LCMS (ES⁺) *m*/*z* calculated for C₂₁H₁₄F₅N₇O₂ 491.37, found 492 (M+H)⁺. HPLC *t*_{R} 1.40 min.

### Example 40: (3-(4-(4-Chloro-1-(2-hydroxy-2-methylpropyl)-1H-pyrazol-5-yl)benzyl)-1,2,3-oxadiazol-3-ium-5-yl)((2-(trifluoromethyl)pyridin-4-yl)carbamoyl)amide.

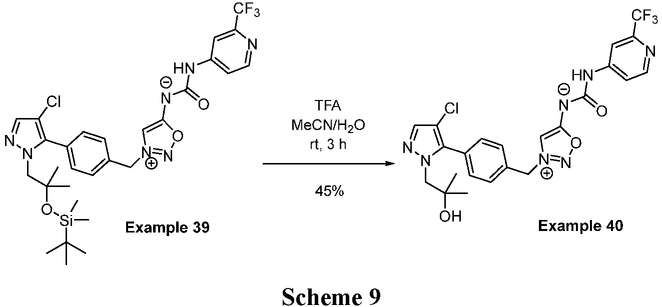

A solution of **Example 39** (31 mg, 0.048 mmol,) in a mixture of MeCN/H₂O (1:1, 0.3 M) at rt was treated with TFA (18 µL, 0.24 mmol). The resulting mixture reaction was then stirred for 3 h at rt. The mixture reaction was quenched with NaHCO₃ sat. aq. solution and diluted with EtOAc, the phases were separated and extracted with EtOAc (2x). The organic layer was washed with H₂O and brine, dried over Na₂SO₄, filtered and concentrated *in vacuo* to get a residue which was purified by flash chromatography on C18 RP (eluting with 0-100% CH₃CN in H₂O) to give the title compound as a white powder (11.5 mg, 45%). ¹H NMR (400 MHz, DMSO-*d*₆) δ 10.19 (s, 1H), 8.46 (d, *J =* 5.5 Hz, 1H), 8.41 (s, 1H), 8.21 (d, *J =* 1.5 Hz, 1H), 7.75-7.69 (m, 4H), 7.61 (d, *J* = 8.3 Hz, 2H), 5.91 (s, 2H), 4.64 (s, 1H), 3.98 (s, 2H), 0.97 (s, 6H). ¹⁹F NMR (377 MHz, DMSO-*d₆*) δ -66.94 (s, 3F). LCMS (ES⁺) m/z calculated for C₂₃H₂₁ClF₃N₇O₃ 535.13, found 536 (M+H)⁺. HPLC *t*_{R} 1.76 min.

### Example 47 & 51: (3-(4-(4-Chloro-1H-pyrazol-5-yl)benzyl)-1,2,3-oxadiazol-3-ium-5-yl)((2-(trifluoromethyl)pyridin-4-yl)carbamoyl)amide and (3-(4-(4-Chloro-1-(2,2,2-trifluoroethyl)-1H-pyrazol-3-yl)benzyl)-1,2,3-oxadiazol-3-ium-5-yl)((2-(trifluoromethyl)pyridin-4-yl)carbamoyl)amide

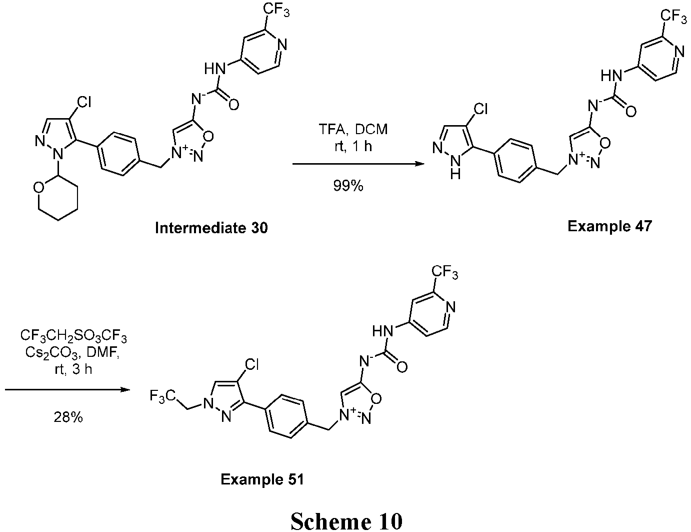

### Step 1: (3-(4-(4-Chloro-1H-pyrazol-5-yl)benzyl)-1,2, 3-oxadiazol-3-ium-5-yl) ((2-(trifluoromethyl)pyridin-4-yl)carbamoyl)amide (Example 47).

A solution of Intermediate **30** (156 mg, 0.28 mmol, synthesized following the synthetic protocol described in the Scheme 7 step 2A, using the appropriate Intermediate **25)** in DCM (5.0 mL, 0.05 M) at rt was treated with TFA (1.0 mL, 2.8 mmol). The resulting mixture reaction was then stirred for 1 h at rt. The excess of solvent was removed under vacuo and the resulting crude was then diluted with a EtOAc and treated with NaHCO₃ sat. aq. solution, then the phases were separated and extracted with EtOAc (2x). The organic layer was washed with brine, dried over Na₂SO₄, filtered and concentrated *in vacuo* to get a residue which was purified by flash chromatography on C18 RP (eluting with 0-100% CH₃CN in H₂O) to give the title compound as a white powder (110.0 mg, 99%). ¹H NMR (400 MHz, DMSO-*d*₆) δ 13.66-13.20 (m, 1H), 10.18 (s, 1H), 8.46 (d, *J=* 5.6 Hz, 1H), 8.35-8.31 (m, 1H), 8.20 (s, 1H), 8.12 (s, 1H), 7.92 (d, *J=* 8.1 Hz, 1H), 7.86-7.80 (m, 1H), 7.76-7.64 (m, 3H), 5.91-5.82 (m, 2H). ¹⁹F NMR (377 MHz, DMSO-*d*₆) δ -66.92 (s, 3F), -108.36 (s, 2F). LCMS (ES⁺) *m*/*z* calculated for C₁₉H₁₃ClF₃N₇O₂ 463.80, found 464 (M+H)⁺. HPLC *t*_{R} 1.65 min.

### Step 2: (3-(4-(4-Chloro-1-(2,2,2-trifluoroethyl)-1H-pyrazol-3-yl)benzyl)-1,2,3-oxadiazol-3-ium-5-yl)((2-(trifluoromethyl)pyridin-4-yl)carbamoyl)amide (Example 51)

A suspension of **Example 54** (20 mg, 0.04 mmol) and cesium carbonate (27 mg, 0.08 mmol) in DMF (100 µL, 0.4 M) at rt was treated with 2,2,2-trifluoroethyl trifluoromethanesulfonate (8.0 µL, 0.05 mmol). The resulting mixture reaction was then stirred for 3 h at rt. The resulting crude was then diluted with H₂O and EtOAc then the phases were separated and extracted with EtOAc (2x). The organic layer was washed with brine, dried over Na₂SO₄, filtered and concentrated *in vacuo* to get a residue which was purified by flash chromatography on C18 RP (eluting with 0-100% CH₃CN in H₂O) to give the title compound as a white powder (6.3 mg, 28%). ¹H NMR (400 MHz, DMSO-*d*₆) δ 10.18 (s, 1H), 8.46 (d, *J=* 5.6 Hz, 1H), 8.34 (s, 1H), 8.26 (s, 1H), 8.20 (d, *J=* 1.6 Hz, 1H), 7.90 (d, *J=* 8.4 Hz, 2H), 7.69 (d, *J=* 8.3 Hz, 3H), 5.88 (s, 2H), 5.22 (q, *J=* 9.1 Hz, 2H). ¹⁹F NMR (377 MHz, DMSO-*d*₆) δ -66.92 (s, 3F), -70.12 (s, 3F). LCMS (ES⁺) m/z calculated for C₂₁H₁₄Cl F₆N₇O₂ 545.82, found 547 (M+H)⁺. HPLC *t*_{R} 1.95 min.

### Example 52: (3-(4-(2-Methyl-6-(methylcarbamoyl)pyridin-3-yl)benzyl)-1,2,3-oxadiazol-3-ium-5-yl)((2-(trifluoromethyl)pyridin-4-yl)carbamoyl)amide

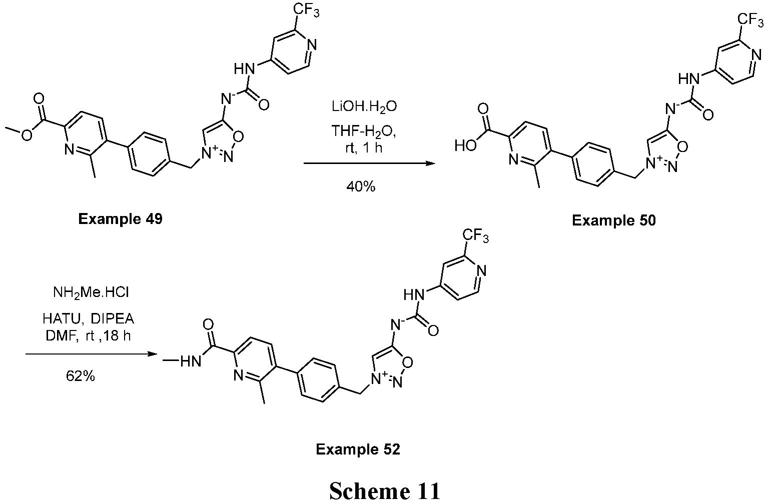

### Step 1: (3-(4-(6-Carboxy-2-methylpyridin-3-yl)benzyl)-1,2,3-oxadiazol-3-ium-5yl)((2-(trifluoromethyl)pyridin-4-yl)carbamoyl)amide (Example 50)

A solution of **Example 49** (28 mg, 0.05 mmol, synthesized following the synthetic protocol described in the Scheme 7 step 2B, using the appropriate starting materials) in a mixture of THF/H₂O (1:1, 0.5 M) at rt was treated with lithium hydroxyde monohydrate (4.5 mg, 0.06 mmol). The resulting reaction mixture was then stirred for 1 h at rt. The mixture reaction was quenched with HCl solution (1.0 M) up to acidic pH and diluted with EtOAc, the phases were separated and extracted with EtOAc. The organic layer was washed with H₂O and brine, dried over Na₂SO₄, filtered and concentrated *in vacuo* to get a residue which was purified by flash chromatography on C18 RP (eluting with 0-100% CH₃CN in H₂O) to give the title compound as a white powder (11.0 mg, 40%). ¹H NMR (400 MHz, DMSO-*d*₆) δ 10.19 (s, 1H), 8.46 (d, *J* = 5.5 Hz, 1H), 8.38 (s, 1H), 8.21 (d, *J* = 1.6 Hz, 1H), 7.95 (d, *J* = 7.9 Hz, 1H), 7.81 (d, *J* = 8.0 Hz, 1H), 7.74 - 7.67 (m, 3H), 7.56 (d, *J =* 8.3 Hz, 2H), 5.91 (s, 2H), 2.50-2.49 (m, 3H). ¹⁹F NMR (377 MHz, DMSO-*d*₆) δ -66.92 (s, 3F). LCMS (ES⁺) *m*/*z* calculated for C₂₃H₁₇F₃N₆O₄ 498.41, found 499 (M+H)⁺. HPLC *t*_{R} 1.28 min.

### Step 2: (3-(4-(2-Methyl-6-(methylcarbamoyl)pyridin-3-yl)benzyl)-1,2,3-oxadiazol-3-ium-5-yl)((2-(trifluoromethyl)pyridin-4-yl)carbamoyl)amide (Example 52)

A solution of (3-(4-(6-carboxy-2-methylpyridin-3-yl)benzyl)-1,2,3-oxadiazol-3-ium-5-yl)((2-(trifluoromethyl)pyridin-4-yl)carbamoyl)amide **(Example 50,** 8.0 mg, 0.02 mmol), methylamine hydrochloride (1.6 mg, 0.02 mmol), HATU (18.3 mg, 0.05 mmol) and DIPEA (16.7 µL, 0.10 mmol) in DMF (0.3 mL) was stirred at rt for 18 h before being diluted with EtOAc and NaHCO₃ (sat. aqueous solution). The organic phase was washed with brine, dried over Na₂SO₄, filtered and concentrated *in vacuo* to give a residue which was purified by automated RP-HPLC using H₂O (+ 0.1% TFA) and CH₃CN (+ 0.1% TFA) as eluents (C18 column). Fractions containing products were lyophilized to give the title compound as a white powder (5.1 mg, 62%). ¹H NMR (400 MHz, DMSO-*d*₆) δ 10.19 (s, 1H), 8.67 (br d, *J* = 4.9 Hz, 1H), 8.46 (d, *J* = 5.6 Hz, 1H), 8.38 (s, 1H), 8.25-8.17 (m, 1H), 7.92 (d, *J* = 7.9 Hz, 1H), 7.81 (d, *J* = 7.9 Hz, 1H), 7.71 (br d,*J* = 8.0 Hz, 3H), 7.56 (d, *J =* 8.1 Hz, 2H), 5.91 (s, 2H), 2.85 (d, *J* = 4.9 Hz, 3H), 2.51-2.48 (m, 3H). ¹⁹F NMR (377 MHz, DMSO-*d*₆) δ -66.92 (s, 3F). LCMS (ES⁺) *m*/*z* calculated for C₂₄H₂₀F₃N₇O₃ 511.46, found 512 (M+H)⁺. HPLC *t*_{R} 1.63 min.

### Example 57: (3-(4-(4,6-Dimethylpyrimidin-5-yl)benzyl)-1,2,3-oxadiazol-3-ium-5-yl)((2-(trifluoromethoxy)pyridin-4-yl)carbamoyl)amide (trifluoroacetate salt).

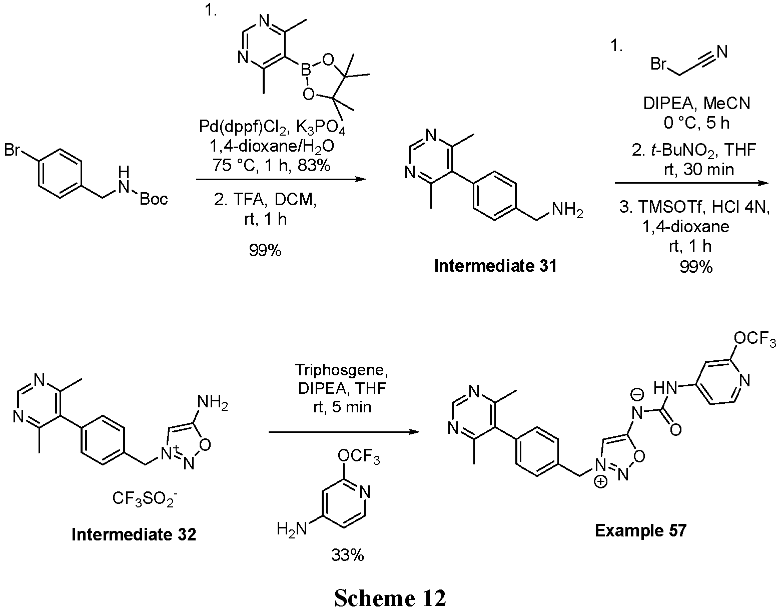

### Step 1: (4-(4,6-Dimethylpyrimidin-5-yl)phenyl)methanamine (trifluoroacetate salt) (Intermediate 31)

A degassed suspension of *tert*-butyl *N-*[(4-bromophenyl)methyl]carbamate (500 mg, 1.7 mmol), 4,6-dimethyl-5-(4,4,5,5-tetramethyl-1,3,2-dioxaborolan-2-yl)pyrimidine (613 mg, 1.5 mmol), Pd(dppf)Cl₂ (127 mg, 0.17 mmol) and K₃PO₄ (1.2 g, 5.2 mmol) in 1,4-dioxane (10 mL) and H₂O (1.0 mL) was stirred at 75 °C for 1 h before being concentrated *in vacuo* to get a residue which was treated with H₂O and EtOAc. The organic layer was washed with brine, dried over Na₂SO₄, filtered and concentrated under reduced pressure to get a residue which was purified by flash chromatography on silica gel (eluting with 5-100% EtOAc in petroleum ether) to give a white solid (455 mg) which was dissolved in DCM (5.8 mL) and treated with TFA (1.4 mL). The reaction mixture was stirred at rt for 1 h before being evaporated *in vacuo* to give the title compound as a yellow oil (570 mg, 82% over two steps). LCMS (ES⁺) *m*/*z* calculated for C₁₄H₁₈N₃ 213.13, found 214 (M+H)⁺. HPLC *t*_{R} 0.34 min.

### Step 2: 5-Amino-3-(4-(4,6-dimethylpyrimidin-5-yl)benzyl)-1,2,3-oxadiazol-3-ium (trifluomethanesulfonate salt) (Intermediate 32)

The title compound was prepared following the procedure reported for the synthesis of Intermediate **30** (prepared as described in WO2023/227734 from appropriated starting materials) obtained as a brown oil and used as such. LCMS (ES⁺) *m*/*z* calculated for C₁₅H₁₆N₅O⁺ 281.13, found 282 (M+H)⁺. HPLC *t*_{R} 0.62 min.

### Step 3: (3-(4-(4, 6-Dimethylpyrimidin-5-yl)benzyl)-1,2,3-oxadiazol-3-ium-5-yl)((2-(trifluoromethoxy)pyridin-4-yl)carbamoyl)amide (trifluoroacetate salt) (Example 57)

The title compound was prepared as reported for the synthesis of **Example 1** in Step 3, scheme 7 and obtained as a yellow powder (15.6 mg, 33%). ¹H NMR (400 MHz, DMSO-*d*₆) δ 10.14 (s, 1H), 8.90 (s, 1H), 8.36 (s, 1H), 8.08 (d, *J* = 5.6 Hz, 1H), 7.71 (d, *J* = 8.3 Hz, 2H), 7.55 (d, *J* = 1.6 Hz, 1H), 7.46-7.40 (m, 3H), 5.91 (s, 2H), 2.18 (s, 6H). ¹⁹F NMR (377 MHz, DMSO-*d*₆) δ -54.78 (s, 3F), -74.70 (s, 6F). LCMS (ES⁺) *m*/*z* calculated for C₂₂H₁₈F₃N₇O₃ 485.42, found 486 (M+H)⁺. HPLC *t*_{R} 1.45 min.

**Examples 53, 56** and **58-59** were prepared using the same synthetic procedure described for the synthesis of **Examples 57** Scheme 12 using the appropriate starting materials.

### Example 53: ((2-Cyclopropylpyridin-4-yl)carbamoyl)(3-(4-(4,6-dimethylpyrimidin-5-yl)benzyl)-1,2,3-oxadiazol-3-ium-5-yl)amide (trifluoracetate salt).

The title compound obtained as a yellow oil (10.5 mg, 45%). ¹H NMR (400 MHz, DMSO-*d*₆) δ 10.73 (s, 1H), 8.90 (s, 1H), 8.47 (s, 1H), 8.35 (d, *J* = 7.0 Hz, 1H), 7.76-7.72 (m, 2H), 7.71 (d, *J* = 8.3 Hz, 2H), 7.43 (d, *J=* 7.7 Hz, 2H), 5.95 (s, 2H), 2.23-2.19 (m, 1H), 2.18 (s, 6H), 1.33-1.25 (m, 2H), 1.03-0.95 (m, 2H). ¹⁹F NMR (377 MHz, DMSO-*d*₆) δ -74.33 (s, 3F). LCMS (ES⁺) m/z calculated for C₂₄H₂₃N₇O₂ 441.19, found 442 (M+H)⁺. HPLC *t*_{R} 0.99 min.

### Example 56: (3-(4-(4,6-Dimethylpyrimidin-5-yl)benzyl)-1,2,3-oxadiazol-3-ium-5-yl)((2-methoxy-6-(trifluoromethyl)pyridin-4-yl)carbamoyl)amide (trifluoracetate salt).

The title compound was obtained as a light-yellow powder (3.2 mg, 14%). ¹H NMR (400 MHz, DMSO-*d*₆) δ 10.10 (s, 1H), 8.90 (s, 1H), 8.39 (s, 1H), 7.74-7.69 (m, 3H), 7.42 (d, *J=* 8.3 Hz, 2H), 7.28 (s, 1H), 5.91 (s, 2H), 3.84 (s, 3H), 2.18 (s, 6H). ¹⁹F NMR (377 MHz, DMSO-*d*₆) δ -67.27 (s, 3F), -74.45 (s, 3F). LCMS (ES⁺) *m*/*z* calculated for C₂₃H₂₀F₃N₇O₃ 499.16, found 500 (M+H)⁺. HPLC *t*_{R} 1.64 min.

### Example 58: (3-(4-(4,6-Dimethylpyrimidin-5-yl)benzyl)-1,2,3-oxadiazol-3-ium-5-yl)((2-methyl-6-(trifluoromethyl)pyridin-4-yl)carbamoyl)amide.

The title compound was obtained as a white powder (9.6 mg, 22%). ¹H NMR (400 MHz, DMSO-*d₆*) δ 10.11 (s, 1H), 8.90 (s, 1H), 8.39 (s, 1H), 8.03 (d, *J =* 1.6 Hz, 1H), 7.73-7.69 (m, *J* = 8.4 Hz, 2H), 7.57 (s, 1H), 7.45-7.41 (m, 2H), 5.90 (s, 2H), 2.44 (s, 3H), 2.18 (s, 6H). ¹⁹F NMR (377 MHz, DMSO-*d*₆) δ -66.89 (s, 3F). LCMS (ES⁺) *m*/*z* calculated for C₂₃H₂₀F₃N₇O₂ 483.16, found 484 (M+H)⁺. HPLC *t*_{R} 1.41 min.

### Example 59: ((2-Chloro-6-(trifluoromethyl)pyridin-4-yl)carbamoyl)(3-(4-(4,6-dimethylpyrimidin-5-yl)benzyl)-1,2,3-oxadiazol-3-ium-5-yl)amide (trifluoracetate salt).

The title compound was obtained as a white powder (3.8 mg, 14%). ¹H NMR (400 MHz, DMSO-*d₆*) δ 10.42 (s, 1H), 8.90 (s, 1H), 8.44 (s, 1H), 8.12 (s, 1H), 7.88 (s, 1H), 7.73-7.69 (m, *J* = 8.3 Hz, 2H), 7.44-7.41 (m, *J =* 8.1 Hz, 2H), 5.92 (s, 2H), 2.18 (s, 6H). ¹⁹F NMR (377 MHz, DMSO-*d*₆) δ -66.26 (s, 3F), -74.75 (s, 3F). LCMS (ES⁺) *m*/*z* calculated for C₂₂H₁₇ClF₃N₇O₂ 503.86, found 504 (M+H)⁺. HPLC *t*_{R} 1.69 min.

### Example 54: (3-(4-(4-Chloro-1-methyl-1H-pyrazol-5-yl)benzyl)-1,2,3-oxadiazol-3-ium-5-yl)((2-cyclopropylpyridin-4-yl)carbamoyl)amide (trifluoroacetate salt).

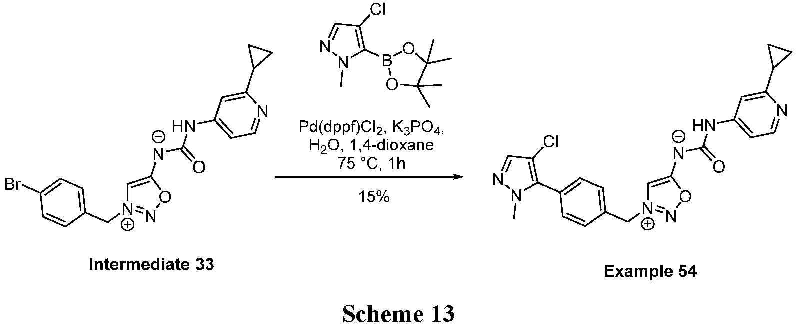

A solution of 4-chloro-1-methyl-5-(4,4,5,5-tetramethyl-1,3,2-dioxaborolan-2-yl)pyrazole (130.0 mg, 0.48 mmol), (3-(4-bromobenzyl)-1,2,3-oxadiazol-3-ium-5-yl)((2-cyclopropylpyridin-4-yl)carbamoyl)amide Intermediate **33** (prepared as reported in Scheme 7 from the corresponding commercially available starting material; 80 mg, 0.19 mmol) and K₃PO₄ (123.0 mg, 0.58 mmol) in 1,4-dioxane (1.3 mL) and H₂O (0.3 mL) was treated with Pd(dppf)Cl₂ (14.0 mg, 0.02 mmol), degassed and heated at 75 °C for 1 h. After cooling the mixture was treated with EtOAc and H₂O and the organic phase was washed with brine, dried over Na₂SO₄, filtered and concentrated *in vacuo* to give a residue which was purified by automated RP-HPLC using H₂O (+ 0.1% TFA) and CH₃CN (+ 0.1% TFA) as eluents (C18 column). Fractions containing product were lyophilized to give the title compound as a powder (3.5 mg, 4%). ¹H NMR (400 MHz, DMSO-*d*₆) δ 10.71 (s, 1H), 8.48 (s, 1H), 8.35 (d, *J =* 7.0 Hz, 1H), 7.78-7.76 (m, 3H), 7.69 (s, 1H), 7.67-7.60 (m, 2H), 5.96 (s, 2H), 3.83 (s, 3H), 2.56-2.54 (m, 1H), 2.28-2.17 (m, 1H), 1.33-1.21 (m, 2H), 1.07-0.91 (m, 2H). ¹⁹F NMR (377 MHz, DMSO-*d*₆) δ -77.39 (s, 3F). LCMS (ES⁺) *m*/*z* calculated for C₂₂H₂₀ClN₇O₂ 449.89, found 450 (M+H)⁺. HPLC *t*_{R} 1.12 min.

**Examples 55** and **60** were prepared using the same synthetic procedure described for the synthesis of **Examples 54** in Scheme 13 using the appropriate starting materials.

### Example 55: ((2-Cyclopropylpyridin-4-yl)carbamoyl)(3-(4-(2-(difluoromethyl)pyridin-3-yl)benzyl)-1,2,3-oxadiazol-3-ium-5-yl)amide.

The title compound was obtained as a white powder (15.0 mg, 27%). ¹H NMR (400 MHz, DMSO-*d₆*) δ 9.76 - 9.52 (m, 1H), 8.82-8.70 (m, 1H), 8.37-8.25 (m, 1H), 8.14-8.05 (m, 1H), 7.97-7.86 (m, 1H), 7.76-7.60 (m, 3H), 7.57-7.39 (m, 3H), 7.35-7.19 (m, 1H), 7.00-6.61 (m, 1H), 6.01-5.82 (m, 2H), 1.94-1.86 (m, 1H), 0.89-0.81 (m, 4H). ¹⁹F NMR (377 MHz, DMSO-*d*₆) δ -111.53 (s, 2F). LCMS (ES⁺) *m*/*z* calculated for C₂₄H₂₀F₂N₆O₂ 462.45, found 463 (M+H)⁺. HPLC *t*_{R} 1.12 min. **Example 60: (3-(4-(4,6-Dimethylpyrimidin-5-yl)benzyl)-1,2,3-oxadiazol-3-ium-5-yl)((2-(methylamino)-6-(trifluoromethyl)pyridin-4-yl)carbamoyl)amide (trifluoroacetate salt).** The title compound was obtained as a white powder (1.0 mg, 2%). ¹H NMR (400 MHz, DMSO-*d₆*) δ 9.76 (s, 1H), 8.90 (s, 1H), 8.32 (s, 1H), 7.72-7.68 (m, *J =* 8.1 Hz, 2H), 7.44-7.40 (m, *J=* 8.1 Hz, 2H), 7.20 (s, 1H), 7.01 (s, 1H), 5.89 (s, 2H), 2.76-2.69 (m, 3H), 2.18 (s, 6H). ¹⁹F NMR (377 MHz, DMSO-*d*₆) δ -59.71 (s, 3F), -74.52 (s, 3F). LCMS (ES⁺) *m*/*z* calculated for C₂₃H₂₁F₃N₈O₂ 498.46, found 499 (M+H)⁺. HPLC *t*_{R} 1.50 min.

Structural properties of exemplified compounds are included in the Table 1 below.

| Example | Molecule | IUPAC Name | Weight |
|---|---|---|---|
| 1 | 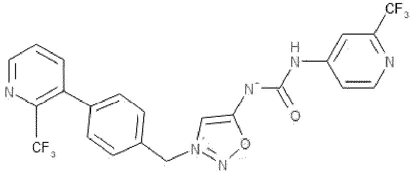 | (3-(4-(2-(trifluoromethyl)pyridin-3-yl)benzyl)-1,2,3-oxadiazol-3 -ium-5 -yl)((2-(trifluoromethyl)pyridin-4-yl)carbamoyl)amide | 508.37598 |
| 2 | 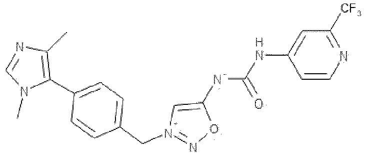 | (3-(4-(1,4-dimethyl-1H-imidazol-5-yl)benzyl)-1,2,3 -oxadiazol-3 -ium-5 - yl)((2-(trifluoromethyl)pyridin-4-yl)carbamoyl)amide | 457.40853 |
| 3 | 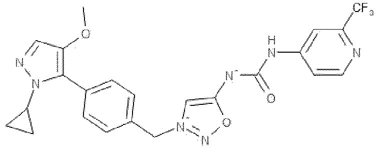 | (3-(4-(1-cyclopropyl-4-methoxy-1H-pyrazol-5-yl)benzyl)-1,2,3-oxadiazol-3 -ium-5 -yl)((2-(trifluoromethyl)pyridin-4-yl)carbamoyl)amide | 499.44521 |
| 4 | 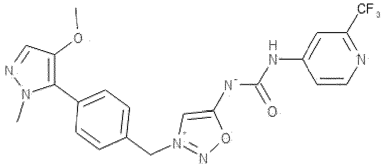 | (3-(4-(4-methoxy-1-methyl-1H-pyrazol-5-yl)benzyl)-1,2,3-oxadiazol-3 -ium-5 -yl)((2-(trifluoromethyl)pyridin-4-yl)carbamoyl)amide | 473.40793 |
| 5 | 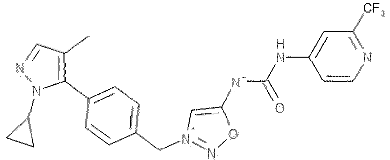 | (3-(4-(1-cyclopropyl-4-methyl-1H-pyrazol-5-yl)benzyl)-1,2,3-oxadiazol-3 -ium-5 -yl)((2-(trifluoromethyl)pyridin-4-yl)carbamoyl)amide | 483.44581 |
| 6 | 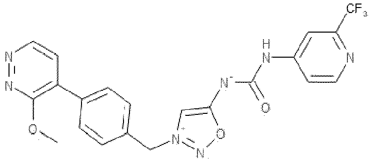 | (3-(4-(3-methoxypyridazin-4-yl)benzyl)-1,2,3-oxadiazol-3 -ium-5 -yl)((2-(trifluoromethyl)pyridin-4-yl)carbamoyl)amide | 471.39205 |
| 7 | 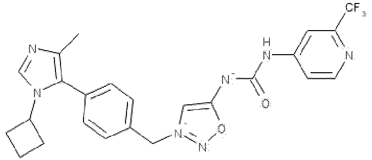 | (3-(4-(1-cyclobutyl-4-methyl-1H-imidazol-5-yl)benzyl)-1,2,3-oxadiazol-3 -ium-5 -yl)((2-(trifluoromethyl)pyridin-4-yl)carbamoyl)amide | 497.47239 |
| 8 | 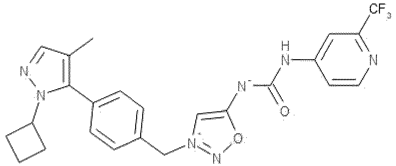 | (3-(4-(1-cyclobutyl-4-methyl-1H-pyrazol-5-yl)benzyl)-1,2,3-oxadiazol-3 -ium-5 -yl)((2-(trifluoromethyl)pyridin-4-yl)carbamoyl)amide | 497.47239 |
| 9 | 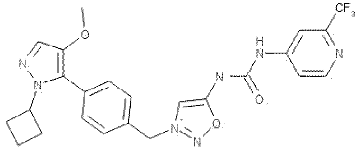 | (3-(4-(1-cyclobutyl-4-methoxy-1H-pyrazol-5-yl)benzyl)-1,2,3-oxadiazol-3 -ium-5 -yl)((2-(trifluoromethyl)pyridin-4-yl)carbamoyl)amide | 513.47179 |
| 10 | 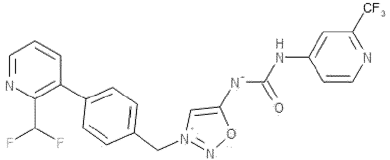 | (3-(4-(2-(difluoromethyl)pyridin-3-yl)benzyl)-1,2,3-oxadiazol-3 -ium-5 -yl)((2-(trifluoromethyl)pyridin-4-yl)carbamoyl)amide | 490.38552 |
| 11 | 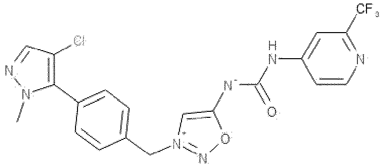 | (3-(4-(4-chloro-1 -methyl-1H-pyrazol-5-yl)benzyl)-1,2,3 -oxadiazol-3 -ium-5 - yl)((2-(trifluoromethyl)pyridin-4-yl)carbamoyl)amide | 477.82701 |
| 12 | 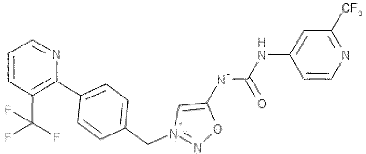 | (3-(4-(3-(trifluoromethyl)pyridin-2-yl)benzyl)-1,2,3-oxadiazol-3 -ium-5 -yl)((2-(trifluoromethyl)pyridin-4-yl)carbamoyl)amide | 508.37598 |
| 13 | 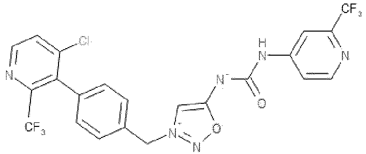 | (3-(4-(4-chloro-2-(trifluoromethyl)pyridin-3-yl)benzyl)-1,2,3-oxadiazol-3 -ium-5 -yl)((2-(trifluoromethyl)pyridin-4-yl)carbamoyl)amide | 542.82104 |
| 14 | 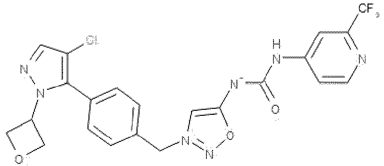 | (3-(4-(4-chloro-1 -(oxetan-3-yl)-1H-pyrazol-5-yl)benzyl)-1,2,3-oxadiazol-3 -ium-5 -yl)((2-(trifluoromethyl)pyridin-4-yl)carbamoyl)amide | 519.86369 |
| 15 | 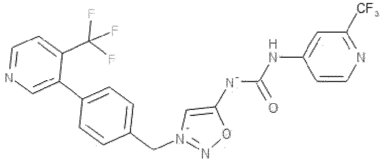 | (3-(4-(4-(trifluoromethyl)pyridin-3-yl)benzyl)-1,2,3-oxadiazol-3 -ium-5 -yl)((2-(trifluoromethyl)pyridin-4-yl)carbamoyl)amide | 508.37598 |
| 16 | 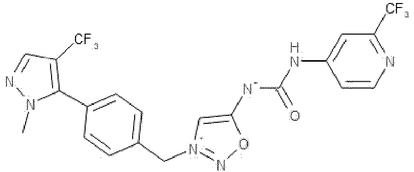 | (3-(4-(1-methyl-4-(trifluoromethyl)-1H-pyrazol-5-yl)benzyl)-1,2,3-oxadiazol-3 -ium-5 -yl)((2-(trifluoromethyl)pyridin-4-yl)carbamoyl)amide | 511.37992 |
| 17 | 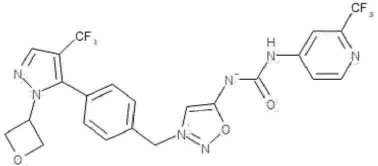 | (3-(4-(1-(oxetan-3-yl)-4-(trifluoromethyl)-1H-pyrazol-5-yl)benzyl)-1,2,3-oxadiazol-3 -ium-5 -yl)((2-(trifluoromethyl)pyridin-4-yl)carbamoyl)amide | 553.4166 |
| 18 | 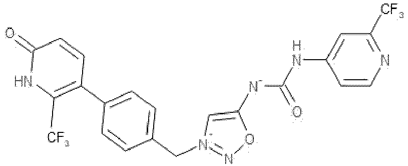 | (3-(4-(6-oxo-2-(trifluoromethyl)-1,6-dihydropyridin-3-yl)benzyl)-1,2,3-oxadiazol-3 -ium-5 -yl)((2-(trifluoromethyl)pyridin-4-yl)carbamoyl)amide | 524.37538 |
| 19 | 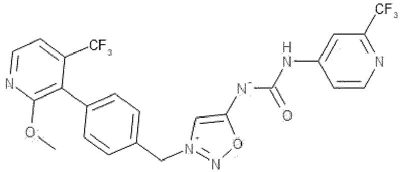 | (3-(4-(2-methoxy-4-(trifluoromethyl)pyridin-3-yl)benzyl)-1,2,3-oxadiazol-3 -ium-5 -yl)((2-(trifluoromethyl)pyridin-4-yl)carbamoyl)amide | 538.40196 |
| 20 | 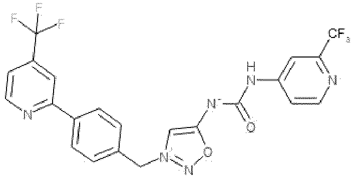 | (3-(4-(4-(trifluoromethyl)pyridin-2-yl)benzyl)-1,2,3-oxadiazol-3 -ium-5 -yl)((2-(trifluoromethyl)pyridin-4-yl)carbamoyl)amide | 508.37598 |
| 21 | 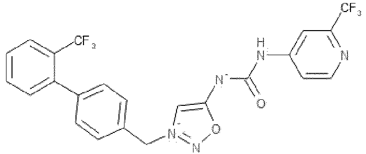 | (3-((2'-(trifluoromethyl)-[1,1'-biphenyl]-4-yl)methyl)-1,2,3-oxadiazol-3 -ium-5 -yl)((2-(trifluoromethyl)pyridin-4-yl)carbamoyl)amide | 507.38792 |
| 22 | 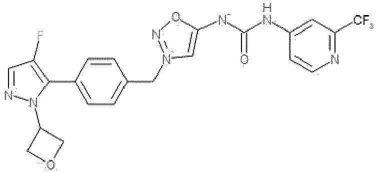 | (3-(4-(4-fluoro-1 -(oxetan-3-yl)-1H-pyrazol-5-yl)benzyl)-1,2,3-oxadiazol-3 -ium-5 -yl)((2-(trifluoromethyl)pyridin-4-yl)carbamoyl)amide | 503.40909 |
| 23 | 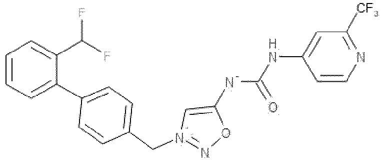 | (3-((2'-(difluoromethyl)-[1,1'-biphenyl]-4-yl)methyl)-1,2,3-oxadiazol-3 -ium-5 -yl)((2-(trifluoromethyl)pyridin-4-yl)carbamoyl)amide | 489.39746 |
| 24 | 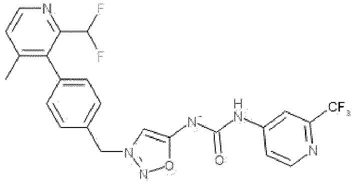 | (3-(4-(2-(difluoromethyl)-4-methylpyridin-3-yl)benzyl)-1,2,3-oxadiazol-3 -ium-5 -yl)((2-(trifluoromethyl)pyridin-4-yl)carbamoyl)amide | 504.4121 |
| 25 | 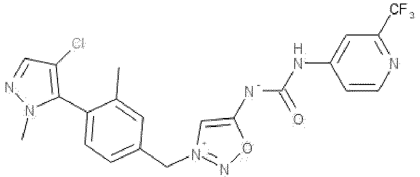 | (3-(4-(4-chloro-1 -methyl-1H-pyrazol-5-yl)-3-methylbenzyl)-1,2,3-oxadiazol-3 -ium-5 -yl)((2-(trifluoromethyl)pyridin-4-yl)carbamoyl)amide | 491.85359 |
| 26 | 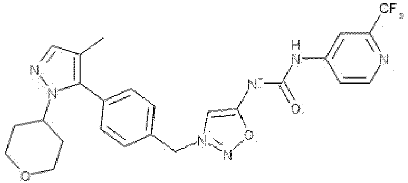 | (3-(4-(4-methyl-1-(tetrahydro-2H-pyran-4-yl)-1H-pyrazol-5-yl)benzyl)-1,2,3-oxadiazol-3 -ium-5 -yl)((2-(trifluoromethyl)pyridin-4-yl)carbamoyl)amide | 527.49837 |
| 27 | 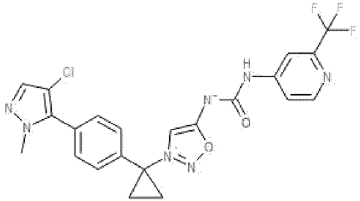 | (3-(1-(4-(4-chloro-1-methyl-1H-pyrazol-5-yl)phenyl)cyclopropyl)-1,2,3 -oxadiazol-3 -ium-5 - yl)((2-(trifluoromethyl)pyridin-4-yl)carbamoyl)amide | 503.86429 |
| 28 | 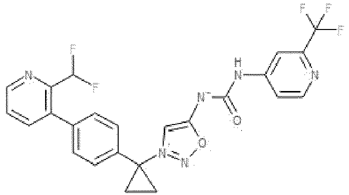 | (3-(1-(4-(2-(difluoromethyl)pyridin-3-yl)phenyl)cyclopropyl)-1,2,3 -oxadiazol-3 -ium-5 - yl)((2-(trifluoromethyl)pyridin-4-yl)carbamoyl)amide | 516.4228 |
| 29 | 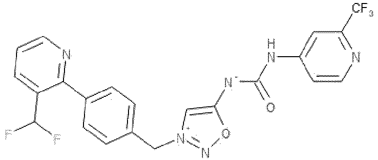 | (3-(4-(3-(difluoromethyl)pyridin-2-yl)benzyl)-1,2,3-oxadiazol-3 -ium-5 -yl)((2-(trifluoromethyl)pyridin-4-yl)carbamoyl)amide | 490.38552 |
| 30 | 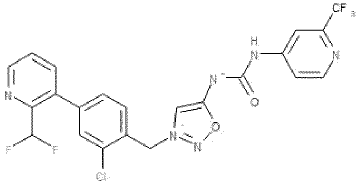 | (3-(2-chloro-4-(2-(difluoromethyl)pyridin-3-yl)benzyl)-1,2,3-oxadiazol-3 -ium-5 -yl)((2-(trifluoromethyl)pyridin-4-yl)carbamoyl)amide | 524.83058 |
| 31 | 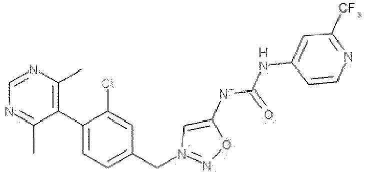 | (3-(3-chloro-4-(4,6-dimethylpyrimidin-5-yl)benzyl)-1,2,3-oxadiazol-3 -ium-5 -yl)((2-(trifluoromethyl)pyridin-4-yl)carbamoyl)amide | 503.86429 |
| 32 | 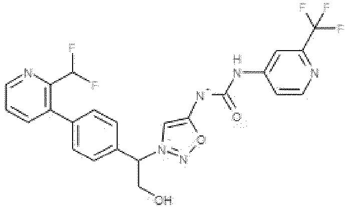 | (3-(1-(4-(2-(difluoromethyl)pyridin-3-yl)phenyl)-2-hydroxyethyl)-1,2,3-oxadiazol-3 -ium-5 -yl)((2-(trifluoromethyl)pyridin-4-yl)carbamoyl)amide | 520.4115 |
| 33 | 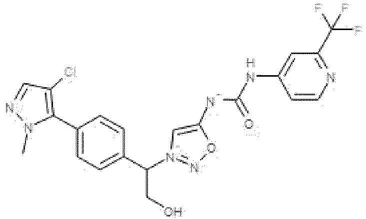 | (3-(1-(4-(4-chloro-1-methyl-1H-pyrazol-5-yl)phenyl)-2-hydroxyethyl)-1,2,3-oxadiazol-3 -ium-5 -yl)((2-(trifluoromethyl)pyridin-4-yl)carbamoyl)amide | 507.85299 |
| 34 | 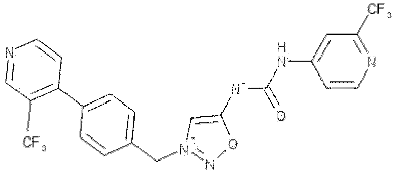 | (3-(4-(3-(trifluoromethyl)pyridin-4-yl)benzyl)-1,2,3-oxadiazol-3 -ium-5 -yl)((2-(trifluoromethyl)pyridin-4-yl)carbamoyl)amide | 508.37598 |
| 35 | 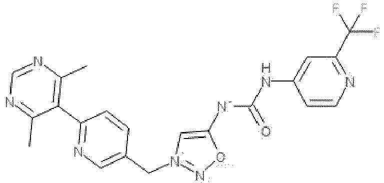 | (3-((6-(4,6-dimethylpyrimidin-5-yl)pyridin-3 -yl)methyl)-1,2,3 -oxadiazol-3 -ium-5 - yl)((2-(trifluoromethyl)pyridin-4-yl)carbamoyl)amide | 470.40729 |
| 36 | 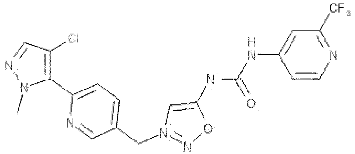 | (3-((6-(4-chloro-1-methyl-1H-pyrazol-5-yl)pyridin-3-yl)methyl)-1,2,3-oxadiazol-3 -ium-5 -yl)((2-(trifluoromethyl)pyridin-4-yl)carbamoyl)amide | 478.81507 |
| 37 | 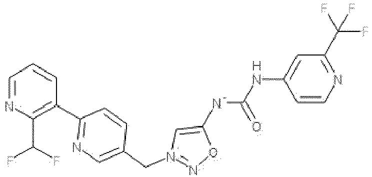 | (3-((2'-(difluoromethyl)-[2,3'-bipyridin]-5-yl)methyl)-1,2,3-oxadiazol-3 -ium-5 -yl)((2-(trifluoromethyl)pyridin-4-yl)carbamoyl)amide | 491.37358 |
| 38 | 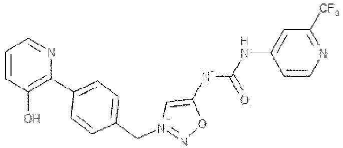 | (3-(4-(3-hydroxypyridin-2-yl)benzyl)-1,2,3-oxadiazol-3 -ium-5 -yl)((2-(trifluoromethyl)pyridin-4-yl)carbamoyl)amide | 456.37741 |
| 39 | 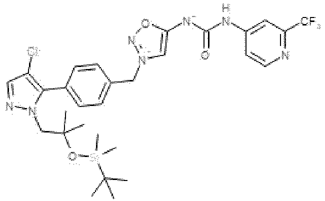 | (3-(4-(1-(2-((tertbutyldimethylsilyl)oxy)-2-methylpropyl)-4-chloro-1H-pyrazol-5-yl)benzyl)-1,2,3 -oxadiazol-3 -ium-5 - yl)((2-(trifluoromethyl)pyridin-4-yl)carbamoyl)amide | 650.16701 |
| 40 | 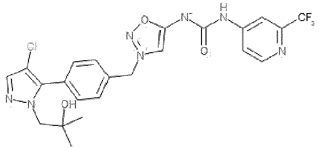 | (3-(4-(4-chloro-1-(2-hydroxy-2-methylpropyl)-1H-pyrazol-5-yl)benzyl)-1,2,3 -oxadiazol-3 -ium-5 - yl)((2-(trifluoromethyl)pyridin-4-yl)carbamoyl)amide | 535.90615 |
| 41 | 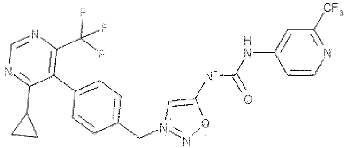 | (3-(4-(4-cyclopropyl-6-(trifluoromethyl)pyrimidin -5-yl)benzyl)-1,2,3-oxadiazol-3 -ium-5 -yl)((2-(trifluoromethyl)pyridin-4-yl)carbamoyl)amide | 549.4279 |
| 42 | 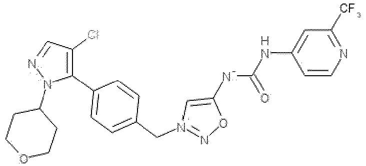 | (3-(4-(4-chloro-1-(tetrahydro-2H-pyran-4-yl)-1H-pyrazol-5-yl)benzyl)-1,2,3-oxadiazol-3 -ium-5 -yl)((2-(trifluoromethyl)pyridin-4-yl)carbamoyl)amide | 547.91685 |
| 43 | 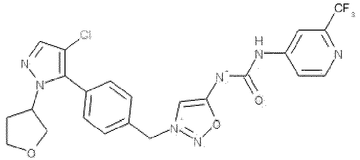 | (3-(4-(4-chloro-1-(tetrahydrofuran-3-yl)-1H-pyrazol-5-yl)benzyl)-1,2,3-oxadiazol-3 -ium-5 -yl)((2-(trifluoromethyl)pyridin-4-yl)carbamoyl)amide | 533.89027 |
| 44 | 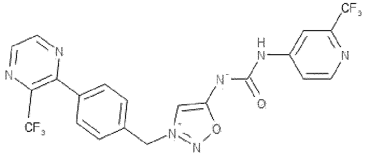 | (3-(4-(3-(trifluoromethyl)pyrazin-2-yl)benzyl)-1,2,3-oxadiazol-3 -ium-5 -yl)((2-(trifluoromethyl)pyridin-4-yl)carbamoyl)amide | 509.36404 |
| 45 | 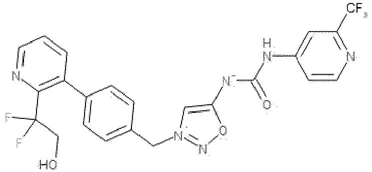 | (3-(4-(2-(1,1-difluoro-2-hydroxyethyl)pyridin-3 - yl)benzyl)-1,2,3-oxadiazol-3 -ium-5 -yl)((2-(trifluoromethyl)pyridin-4-yl)carbamoyl)amide | 520.4115 |
| 46 | 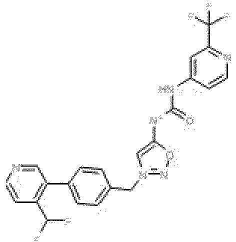 | (3-(4-(4-(difluoromethyl)pyridin-3-yl)benzyl)-1,2,3-oxadiazol-3 -ium-5 -yl)((2-(trifluoromethyl)pyridin-4-yl)carbamoyl)amide | 490,38552 |
| 47 | 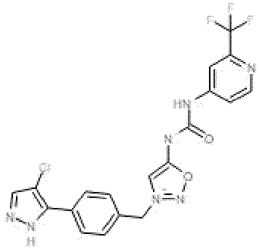 | (3-(4-(4-chloro-1H-pyrazol-5-yl)benzyl)-1,2,3-oxadiazol-3 -ium-5 -yl)((2-(trifluoromethyl)pyridin-4-yl)carbamoyl)amide | 463,80043 |
| 48 | 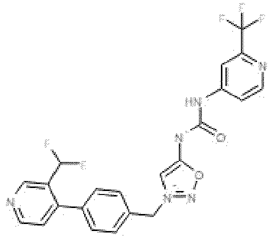 | (3-(4-(3-(difluoromethyl)pyridin-4-yl)benzyl)-1,2,3-oxadiazol-3 -ium-5 -yl)((2-(trifluoromethyl)pyridin-4-yl)carbamoyl)amide | 490,38552 |
| 49 | 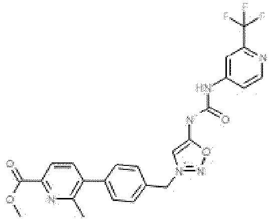 | (3-(4-(6-(methoxycarbonyl)-2-methylpyridin-3-yl)benzyl)-1,2,3-oxadiazol-3 -ium-5 -yl)((2-(trifluoromethyl)pyridin-4-yl)carbamoyl)amide | 512,44067 |
| 50 | 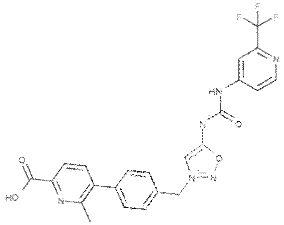 | (3-(4-(6-carboxy-2-methylpyridin-3-yl)benzyl)-1,2,3-oxadiazol-3 -ium-5 -yl)((2-(trifluoromethyl)pyridin-4-yl)carbamoyl)amide | 498,41409 |
| 51 | 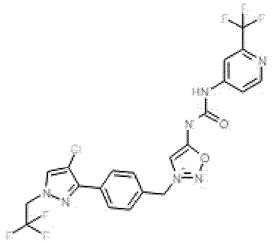 | (3-(4-(4-chloro-1-(2,2,2-trifluoroethyl)-1H-pyrazol-3-yl)benzyl)-1,2,3-oxadiazol-3 -ium-5 -yl)((2-(trifluoromethyl)pyridin-4-yl)carbamoyl)amide | 545,82498 |
| 52 | 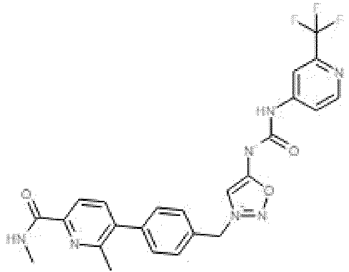 | (3-(4-(2-methyl-6-(methylcarbamoyl)pyridin-3-yl)benzyl)-1,2,3-oxadiazol-3 -ium-5 -yl)((2-(trifluoromethyl)pyridin-4-yl)carbamoyl)amide | 511,45591 |
| 53 | 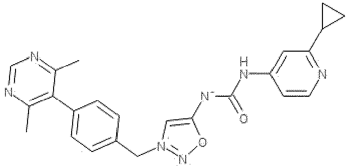 | ((2-cyclopropylpyridin-4-yl)carbamoyl)(3-(4-(4,6-dimethylpyrimidin-5-yl)benzyl)-1,2,3-oxadiazol-3 -ium-5 - yl)amide | 441.48512 |
| 54 | 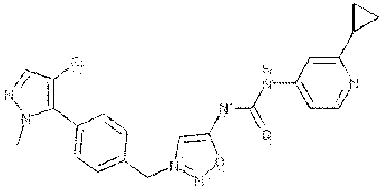 | (3-(4-(4-chloro-1 -methyl-1H-pyrazol-5-yl)benzyl)-1,2,3 -oxadiazol-3 -ium-5 - yl)((2-cyclopropylpyridin-4-yl)carbamoyl)amide | 449.8929 |
| 55 | 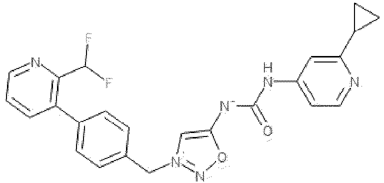 | ((2-cyclopropylpyridin-4-yl)carbamoyl)(3-(4-(2-(difluoromethyl)pyridin-3-yl)benzyl)-1,2,3-oxadiazol-3-ium-5-yl)amide | 462.45141 |
| 56 | 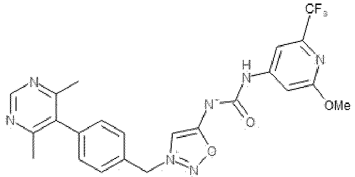 | (3-(4-(4,6-Dimethylpyrimidin-5-yl)benzyl)-1,2,3-oxadiazol-3 -ium-5 -yl)((2-methoxy-6-(trifluoromethyl)pyridin-4-yl)carbamoyl)amide | 499,44521 |
| 57 | 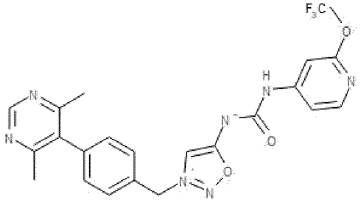 | (3-(4-(4,6-Dimethylpyrimidin-5-yl)benzyl)-1,2,3-oxadiazol-3 -ium-5 -yl)((2-(trifluoromethoxy)pyridin-4-yl)carbamoyl)amide | 485,41863 |
| 58 | 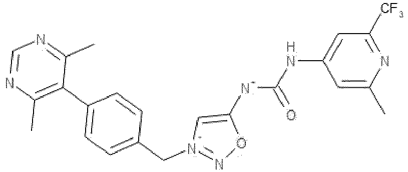 | (3-(4-(4,6-Dimethylpyrimidin-5-yl)benzyl)-1,2,3-oxadiazol-3 -ium-5 -yl)((2-methyl-6-(trifluoromethyl)pyridin-4-yl)carbamoyl)amide | 483,44581 |
| 59 | 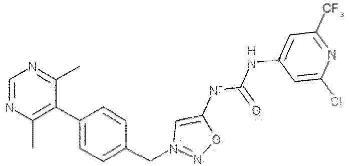 | ((2-Chloro-6-(trifluoromethyl)pyridin-4-yl)carbamoyl)(3-(4-(4,6-dimethylpyrimidin-5-yl)benzyl)-1,2,3-oxadiazol-3-ium-5-yl)amide | 503,86429 |
| 60 | 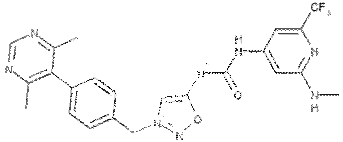 | (3-(4-(4,6-Dimethylpyrimidin-5-yl)benzyl)-1,2,3-oxadiazol-3 -ium-5 -yl)((2-(methylamino)-6-(trifluoromethyl)pyridin-4-yl)carbamoyl)amide | 498,46045 |

### Biology

### WNV protease inhibition assay

The enzyme inhibition assay was performed in 384-well flat bottom black polystyrene microplates (cat. N. 781900, Greiner,) in a reaction volume of 20 µl. West Nile Virus (WNV) NS2B-NS3 protease, MHHHHHH (SEQ. ID. No 2; NS2B₁₄₂₃₋₁₄₇₀) - GGGGSGGGG (SEQ. ID. No 3; NS3₁₅₀₆₋₁₆₈₉) Accession # YP_001527877.1, R&D, cat. N. 2907-SE-020, (2 nM) was incubated with increasing inhibitor concentrations (0.097-100 µM or 0.0009-1 µM for the most active compounds) in assay buffer (50 mM Tris-HCl, pH 9.0, 30% glycerol, 1% DMSO) for 10 minutes at 25 °C. 10 µM of pERTKR-AMC substrate (R&D, cat. N. ES013) was added and the reaction mixture incubated for additional 30 minutes at 25 °C. 10 µM of pERTKR-AMC substrate (R&D, cat. N. ES013) was added and the reaction mixture incubated for additional 30 minutes at 25 °C. Product formation was evaluated by fluorescence measurement (excitation 360 nm, emission 465 nM), using a SPARK TM10 Tecan instrument. Results were analyzed using Prism (GraphPad, San Diego, CA) and Vortex software (Dotmatics, Bioshops Stortford, UK). Dose-response curves were fitted by four-parameter logistic regression.

### WNV replicon generation

To construct the subgenomic replicon we used the U.S.A. isolate genome (Genbank Accession Number AY646354.1, total 11.030 bp). In the replicon, the coding sequences of structural genes were removed except for 31 amino acids at the N-terminus of the capsid protein fused with 30 amino acids at the C-terminus of the E protein. These sequences were retained to preserve the correct processing and translocation of NS1 and of the non-structural polyprotein across the endothelium reticulum membrane. An EMCV IRES was placed after the stop codon of the polyprotein. The IRES drives the translation of a downstream fusion protein consisting of a luciferase (Nano-Luc) gene followed by the ubiquitin gene (UBI), and the neomycin phosphotransferase resistance gene (NEO). This fusion protein is required for reporting purposes and to confer resistance to G-418. The sub-genomic replicon was transcribed as RNA via an upstream T7 promoter and transfected in Vero cells. The stable cell line used for the WNV replication assay was generated via selection with G-418.

### WNV replication inhibition assay and cell viability assay

A green monkey cell line (Vero) with a stable WNV replicon was grown and maintained in Dulbecco's modified eagle's medium (DMEM) with high glucose and pyruvate (GIBCO #41966) completed with 10% fetal bovine serum (FBS) (Gibco #10270), 1% Penicillin Streptomycin (10 mg/ml) (Gibco #14140). 0.700 mg/mL G418 solution (Sigma-Aldrich, Cat 4727894001) were used to maintain the WNV replication in the cells. The day of the experiment, compounds previously dissolved in 100% dimethyl sulfoxide (DMSO, Sigma-Aldrich, D5879-1L) were transferred to a 384 black plate (Greiner #781086) using an acoustic system (Echo650, Beckman). Compounds were tested in dose response. 50 µM of example 26 (WO2023/227734) was used in as positive control for the WNV replication assay while 32 µM of Gambogic Acid (Sigma-Aldrich, G8171) was used as positive control for the cell viability assay. In both cases, in the negative control wells were added with 0.5% DMSO. 6000 cells/well were plated on the compound containing plates in 40 µL growth medium. 72 h after the treatment at 37 °C, 5% CO₂ and 90% humidity, 20 µL Nano-Glo (Promega #N1150) were added to reveal the NanoLuc signal, while 30 µL CellTiter-Glo (Promega #G7573) were added to determine the cell viability (Multiplex assay). The luminescent signal for NanoLuc was detected reading the plate on the Envision plate reading 10 minutes post detection reagent addition (luminescence was measured at 0.5 sec/well), while for cell viability (CellTiter-Glo) reading the plate after 30 minutes post addition reagent detection. Data were normalized between 0 and 100% inhibition (negative control and positive control respectively). For EC₅₀ determinations, the dose-response was fitted with a 4-p logistic regression approach using the Dotmatics Studies software.

### ZIKV protease expression and purification

The sequence coding for NS2B (aa 49-95, R95A) and NS3 (aa 1-170, R29G) were synthesized (GenScript, Piscataway, New Jersey, United States), cloned in pET15b vector downstream the sequence for 6 x His tag and fused by a not cleavable linker (G₄SG₄). The protease was expressed in *E. coli* BL21 (DE3) cells: bacteria were grown in LB medium at 37 °C up to 0.8 O.D. 600, then the temperature was lowered to 20 °C and protein expression induced with 0.5 mM IPTG (cat. N. I5502, Sigma Aldrich) for 18 h. Cells were collected by centrifugation (4000 × *g*, 4 °C for 30 min), then re-suspended (7 ml/g of cell paste) in lysis buffer (25 mM Tris pH 8.0, 100 mM NaCl, 5% glycerol) supplemented with 0.7 mg/ml of lysozyme (cat. N. 16876, Sigma Aldrich) and 25 U/ml of benzonase (cat. N. E1014, Sigma Aldrich). After incubating at 25 °C for 30 min, cells were homogenized at 800 bars by PANDA homogenizer (GEA) then cell extract was cleared by centrifugation (32914 × g, 4 °C, 1h), brought to 500 mM NaCl then loaded on 1 ml HisTrap HP column (cat. N. 17-52407-01, Cytiva, Sweden) equilibrated in 25 mM Tris pH 8.5, 500 mMNaCl, 5% glycerol. After three wash steps at 20-, 30- and 50-mM imidazole, recombinant protein was eluted by a linear gradient up to 350 mM imidazole (cat. N. I202, Sigma Aldrich). Fractions containing ZIKV protease were pooled and treated with 2 mM TCEP (cat. N. C4706, Sigma Aldrich) at 4 °C for 40 min, then concentrated threefold on Vivaspin 20, 3 KDa cut off device (cat. N. Z614610-48EA, Sigma Aldrich) and loaded on HiLoad 16/60 Superdex 75 column (cat. N. 28989333, Cytiva, Sweden) using 25 mM Tris pH 8.5, 5% glycerol and 1 mM DTT (cat. N. A2948, PanReac AppliChem) as mobile phase. Monomer peak fractions were collected, divided in small aliquots and frozen in liquid nitrogen.

### ZIKV protease inhibition assay

The enzyme inhibition assay was performed in 384-well flat bottom black polystyrene microplates (cat. N. 781900, Greiner,) in a reaction volume of 20 µl. ZIKV NS2B-G₄SG₄-NS3 serine protease (MGS SHHHHHHS SGLVPRGSHMVDMYIERAGDITWEKDAEVTGNSPRLDVALDESGDF SLVEDDGPPMAGGGGSGGGGSGALWDVPAPKEVKKGETTDGVYRVMTRGLLGSTQV GVGVMQEGVFHTMWHVTKGSALRSGEGRLDPYWGDVKQDLVSYCGPWKLDAAWD GHSEVQLLAVPPGERARNIQTLPGIFKTKDGDIGAVALDYPAGTSGSPILDKCGRVIGLY GNGVVIKNGSYVSAITQGRR; SEQ. ID. No. 1) (1.25 nM) was incubated with increasing inhibitor concentrations (0.097-100 µM or 0.0009 -1 µM for the most active compounds) in assay buffer (50 mM Tris-HCl, pH 8.5, 1% glycerol, 1 mM CHAPS, 1% DMSO) for 10 minutes at 25 °C. 10 µM of Bz-Nle-KRR-AMC substrate (cat. N. 4055312, Bachem) was added and the reaction mixture incubated for additional 30 minutes at 25 °C. Product formation was evaluated by fluorescence measurement (excitation 360 nm, emission 465 nM), using a SPARK TM10 Tecan instrument. Results were analysed using Prism (GraphPad, San Diego, CA) and Vortex software (Dotmatics, Bioshops Stortford, UK). Dose-response curves were fitted by four-parameter logistic regression.

### ZIKV replicon generation

To construct the subgenomic replicon we used the Natal RGN isolate of Asian lineage (GenBank: KU527068.1). In the replicon, the coding sequences of structural genes were removed with the exception of 31 amino acids at the N-terminus of the capsid protein fused with 32 amino acids at the C-terminus of the E protein. These sequences were retained to preserve the correct processing and translocation of NS1 and of the non-structural polyprotein across the endothelium reticulum membrane. An EMCV IRES was placed after the stop codon of the polyprotein. The IRES drives the translation of a downstream fusion protein consisting of a luciferase (Nano-Luc) gene followed by the ubiquitin gene (UBI), and the neomycin phosphotransferase resistance gene (NEO). This fusion protein is required for reporting purposes and to confer resistance to G-418. The sub-genomic replicon was transcribed as RNA via an upstream T7 promoter and transfected in Vero cells. The stable cell line used for the ZIKV replication assay was generated via selection with G-418.

### ZIKV replication inhibition assay and cell viability assay

A green monkey cell line (Vero) with a stable ZIKV replicon was grown and maintained in Dulbecco's modified eagle's medium (DMEM) with high glucose and pyruvate (GIBCO #41966) completed with 10% fetal bovine serum (FBS) (Gibco #10270), 1% Penicillin Streptomycin (10 mg/ml) (Gibco #14140). 0.760 µg/mL G418 solution (Sigma-Aldrich, Cat 4727894001) were used to maintain the ZIKV replication in the cells. The day of the experiment, compounds previously dissolved in 100% dimethyl sulfoxide (DMSO, Sigma-Aldrich, D5879-1L) were transferred to a 384 black plate (Greiner #781086) using an acoustic system (ATS-100 EDC). Compounds were tested in dose response. 50 µM of mycophenolic acid (Sigma-Aldrich, M3536) was used in as positive control for the ZIKV replication assay while 32 µM of Gambogic Acid (Sigma-Aldrich, G8171) was used as positive control for the cell viability assay. In both cases, in the negative control wells were added with 0.5% DMSO. 8000 cells/well were plated on the compound containing plates in 40 µL growth medium. 72 h after the treatment at 37 °C, 5% CO₂ and 90% humidity, 20 µL Nano-Glo (Promega #N1150) were added to reveal the NanoLuc signal, while 20 µL CellTiter-Glo (Promega #G7573) were added to determine the cell viability. The luminescent signal for both assays was detected reading the plate on the Envision plate reading 10 minutes post detection reagent addition (luminescence was measured at 0.5 sec/well). Data were normalized between 0 and 100% inhibition (negative control and positive control respectively). For EC₅₀ determinations, the dose-response was fitted with a 4-p logistic regression approach using the Dotmatics Studies software. The activity of exemplified compounds is reported in the Table 2 below.

**Table 2. Activity values are expressed in nanomolars, wherein: A refers to IC₅₀ or EC₅₀ in the range >0 and ≤ 75 nM; B refers to IC₅₀ or EC₅₀ in the range >75 and ≤ 150 nM; C refers to IC₅₀ or EC₅₀ in the range >150 and ≤ 300 nM; D refers to IC₅₀ or EC₅₀ in the range >300 and ≤ 600 nM; E refers to IC₅₀ or EC₅₀ > 600 nM; N/A: not available.**

| Example | WNV NS3 Protease IC₅₀ | WNV Cell Nanoluc R474#12 EC₅₀ | Zika NS3 Protease IC₅₀ | Zika Cell Nanoluc EC₅₀ |
|---|---|---|---|---|
| 1 | A | A | A | A |
| 2 | A | B | A | C |
| 3 | A | A | A | A |
| 4 | B | A | A | B |
| 5 | A | A | A | A |
| 6 | A | B | B | D |
| 7 | A | B | A | B |
| 8 | B | A | A | A |
| 9 | C | A | A | B |
| 10 | A | A | A | A |
| 11 | A | A | A | A |
| 12 | A | A | A | A |
| 13 | A | A | A | A |
| 14 | A | A | A | A |
| 15 | A | A | A | A |
| 16 | A | A | A | A |
| 17 | A | A | A | A |
| 18 | A | C | A | C |
| 19 | B | A | A | A |
| 20 | E | C | A | C |
| 21 | C | B | A | B |
| 22 | B | B | B | A |
| 23 | D | C | A | B |
| 24 | A | A | A | A |
| 25 | C | B | A | A |
| 26 | A | A | A | A |
| 27 | B | A | A | A |
| 28 | B | A | B | B |
| 29 | A | A | A | A |
| 30 | C | B | A | B |
| 31 | A | A | A | A |
| 32 | B | B | B | C |
| 33 | C | C | A | B |
| 34 | A | A | A | A |
| 35 | A | C | C | B |
| 36 | A | A | B | A |
| 37 | A | B | C | B |
| 38 | C | C | C | D |
| 39 | E | A | C | A |
| 40 | A | A | A | A |
| 41 | A | A | A | A |
| 42 | A | A | A | A |
| 43 | A | A | A | A |
| 44 | A | A | A | A |
| 45 | A | A | A | A |
| 46 | A | A | A | A |
| 47 | C | A | A | B |
| 48 | A | A | A | A |
| 49 | A | E | A | E |
| 50 | B | E | C | E |
| 51 | D | A | A | B |
| 52 | B | A | A | A |
| 53 | A | B | A | B |
| 54 | B | C | A | B |
| 55 | B | C | B | C |
| 56 | E | E | D | D |
| 57 | D | N/A | D | C |
| 58 | B | C | C | B |
| 59 | D | C | C | N/A |
| 60 | D | C | C | C |

### Determination of brain penetration in CD1 Mice

Total brain levels, expressed as brain/plasma ratio (*Kₚ,brain*) were measured by *Cbrain@8h*/*Cplasma@8h* in CD1 mice after IV administration. Unbound fraction of each molecule in biological matrix was determined by in vitro plasma and brain binding assay. K*ₚᵤᵤ*, brain was calculated by the following equation:
*Kₚᵤᵤ,brain = Cbrain@8hlC plasma@8h* × (*fᵤ,brain*/f*ᵤ,plasma*)

In vitro plasma and brain binding assay was carried out on a RED (Rapid Equilibrium Dialysis) device.

Unbound fraction (*fu*) of each molecule in the brain homogenate and plasma were calculated by the ratio of the buffer side response to the brain homogenate/plasma side response. An IV study design was used as the in vivo screening model to identify brain penetration of each molecule. Male naive CD1 mice (n = 3) were intravenously dosed with each molecule at 0.2 mg/kg in 10% 2-hydroxypropyl-beta-cyclodextrin in 50 mM Citrate buffer pH 5.5 (w/v). At 0.083, 0.25, 0.5, 1, 2, 4, and 8 h post-dose plasma samples were collected via tail vein and brain tissue was harvested at the 8 h time point and homogenized in 3x volume of water. All samples were stored at ~-80 °C prior to LC/MS/MS analysis. At the 8 h time point, the unbound brain to plasma ratios were generated to identify molecules with K_{*p*,*uu*}, brain values > 0.3 which is indicative of good passive brain penetration.

**Table 3.**

| **Examples** | **K*****_{p,uu}**, brain* |
|---|---|
| **1** | >0.3 |
| **10** | >0.3 |
| **11** | >0.3 |
| **29** | <0.3 |
| **36** | >0.3 |
| **44** | >0.3 |

| | |
|---|---|
| **Cbrain: concentration measured in brain* **Cplasma: concentration measured in plasma* | |

## Claims

1. A compound of general formula (I): 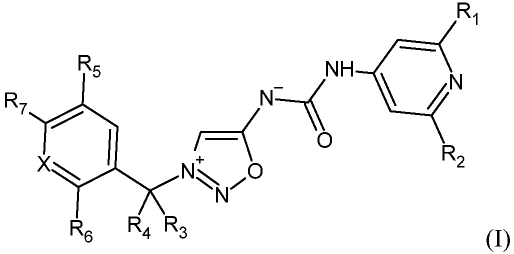 wherein
- XisCorN;
- R₁ is: CF₃, OCF₃ or cyclopropyl optionally substituted with fluorine;
- R₂ is: H, CH₃, OCH₃, Cl, NHCH₃, NHC(=O)R₁₂ wherein R₁₂ is a C₁₋₃alkyl optionally substituted with an aromatic ring ;
- R₃ is H or CH₃ and R₄ is H or CH₂OH; or R₃ and R₄ are linked together to form a spirocyclopropyl ring;
- Rs and R₆ are each independently selected from: H, CH₃ and Cl;
- R₇ is selected from:
a) pyridine, optionally substituted with one or more substitents independently selected from C₁₋₃alkyl, C₃₋₆cycloalkyl, C₁₋₃haloalkyl, C₁₋₃alkoxyl, halogen, hydroxy, C₁₋₃haloalkyl further substituted with an hydroxy group, C(O)OC₁₋₃alkyl, C(O)OH, C(O)NHC₁₋₃alkyl, NHC(=O)C₁₋₃alkyl, SO₂NHC₁₋₃alkyl, SO₂C₁₋₃alkyl, CN with the proviso that said optionally substituted pyridine is not 2-methylpyridin-3-yl and 2,4-dimethylpyridine-3-yl; or
b) pyrazole of formula (II) or (III): 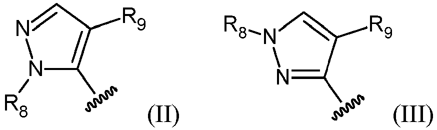 wherein:
R₈ is selected from H, C₁₋₄alkyl optionally substituted with OH, C₃₋₆cycloalkyl, C₃₋₆heterocycloalkyl and C₁₋₃haloalkyl optionally substituted with OH;
R₉ is selected from C₁₋₃alkyl, C₃₋₆cycloalkyl, C₁₋₃haloalkyl, C₁₋₃alkoxyl, CN and halogen;
and wherein if R₉ is CH₃ then Rs is not CH₃ or an oxetane ring; or
c) pyridazine optionally substituted with halogen, C₁₋₃alkoxyl, C₁₋₃haloalkyl, C₃₋₆cycloalkyl, CN; or
d) pyrimidine of formula (IV): 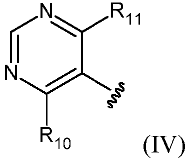 wherein R₁₀ and R₁₁ are each independently selected from C₁₋₃alkyl, C₁₋₃haloalkyl, C₃₋₆cycloalkyl, and wherein if R₁₀ and R₁₁ are both CH₃ then in general formula (I):
▪ X is N; and/or
▪ R₁ is cyclopropyl; and/or
▪ R₂ is CH₃, OCH₃, Cl, NHCH₃; and/orRs is Cl;
e) phenyl or 5 or 6 membered heteroaromatic ring selected from pyrazine, 6-oxo-1,6-dihydropyridine, imidazole and oxazole, each of said ring being optionally substituted with one or more substitents independently selected from C₁₋₃alkyl, C₁₋₃haloalkyl, C₁₋₃alkoxyl, halogen, hydroxy, C₁₋₃haloalkyl further substituted with an hydroxy group, C(O)OC₁₋₃alkyl, C(O)OH, C(O)NHC₁₋₃alkyl; or
or a pharmaceutically acceptable salt, tautomer, solvate, or stereoisomer thereof.

2. The compound of general formula (I) according to claim 1 wherein R₁ is CF₃ and R₂ is H.

3. The compound of general formula (I) according to claims 1 or 2 wherein R₃ and R₄ are H.

4. The compound of general formula (I) according to any one of previous claims wherein R₇ is a pyridine ring selected from: 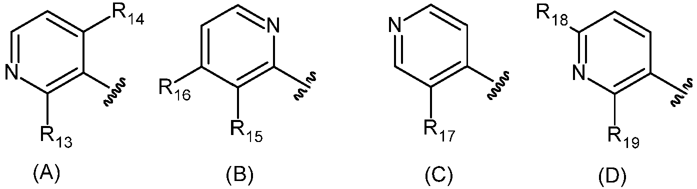 wherein:
- in structure (A) R₁₃ and R₁₄ are independently selected from H, C₁₋₃alkyl, C₁₋₃haloalkyl, C₁₋₃alkoxyl, halogen, and wherein one of R₁₃ and R₁₄ is C₁₋₃haloalkyl, preferably one of R₁₃ and R₁₄ is trifluoromethyl or difluoromethyl;
- in structure (B) R₁₅ and R₁₆ are independently selected from H, C₁₋₃haloalkyl and hydroxy, preferably the C₁₋₃haloalkyl is trifluoromethyl or difluoromethyl;
- in structure (C) R₁₇ is C₁₋₃haloalkyl, preferably trifluoromethyl or difluoromethyl;
- in structure (D) R₁₈ is selected from C₁₋₃alkyl and C(=O)OH, C(=O)=CH₃ and C(=O)NHCH₃ and R₁₉ is C₁₋₃alkyl or C₁₋₃haloalkyl.

5. The compound of general formula (I) according to any one of claims 1 to 3 wherein:
R₇ is a pyrazole of formula (II) wherein:
- Rs is H, methyl, hydroxy-2-methyl-propyl, 1,1-difluoro-2-hydroxyethyl, cyclopropyl, cyclobutyl, oxetane, tetrahydropyrane, tetrahydrofurane; and
- R₉ is methoxy, halogen, trifluoromethyl;
or wherein:
- Rs is H, hydroxy-2-methyl-propyl, cyclopropyl, cyclobutyl, tetrahydropyrane, tetrahydrofurane; and
- R₉ is methyl; or
R₇ is a pyrazole of formula (III) wherein Rs is C₁₋₃alkyl or C₁₋₃haloalkyl or and R₉ is halogen.

6. The compound of general formula (I) according to any one of claims 1 to 4 wherein R₇ is selected from: 2-(trifluoromethyl)pyridin-3-yl, 2-(difluoromethyl)pyridin-3-yl, 3-(trifluoromethyl)pyridin-2-yl, 4-chloro-2-(trifluoromethyl)pyridin-3-yl, 4-(trifluoromethyl)pyridin-3 -yl, 2-methoxy-4-(trifluoromethyl)pyridin-3-yl, 4-(trifluoromethyl)pyridin-2-yl, 2-trifluoromethylphenyl, 2-(difluoromethyl)-4-methylpyridin-3-yl, 3-(difluoromethyl)pyridin-2-yl, 2-(difluoromethyl)pyridin-3-yl, 3-(trifluoromethyl)pyridin-4-yl, 3-hydroxypyridin-2-yl, 1,1-difluoro-2-hydroxyethyl)pyridin-3-yl, 4-(difluoromethyl)pyridin-3-yl, 3-(difluoromethyl)pyridin-4-yl, 6-(methoxycarbonyl)-2-methylpyridin-3-yl, 6-carboxy-2-methylpyridin-3-yl, 2-methyl-6-(methylcarbamoyl)pyridin-3-yl.

7. The compound of general formula (I) according to any one of claims 1 to 3 and 5 wherein R₇ is selected from: 1-cyclopropyl-4-methoxy-1H-pyrazol-5-yl, 4-methoxy-1-methyl-1H-pyrazol-5-yl, 1-cyclopropyl-4-methyl-1H-pyrazol-5-yl, 1-cyclobutyl-4-methyl-1H-pyrazol-5-yl, 1-cyclobutyl-4-methoxy-1H-pyrazol-5-yl, 4-chloro-1-methyl-1H-pyrazol-5-yl, 4-(4-chloro-1-(oxetan-3-yl)-1H-pyrazol-5-yl, 1-methyl-4-(trifluoromethyl)-1H-pyrazol-5-yl, 1-(oxetan-3-yl)-4-(trifluoromethyl)-1H-pyrazol-5-yl, 4-fluoro-1-(oxetan-3-yl)-1H-pyrazol-5-yl, 4-chloro-1-methyl-1H-pyrazol-5-yl, 4-methyl-1-(tetrahydro-2H-pyran-4-yl)-1H-pyrazol-5-yl, 4-chloro-1-methyl-1H-pyrazol-5-yl, 4-chloro-1-methyl-1H-pyrazol-5-yl, 4-chloro-1-(2-hydroxy-2-methylpropyl)-1H-pyrazol-5-yl, 4-chloro-1-(tetrahydro-2H-pyran-4-yl)-1H-pyrazol-5-yl, 4-chloro-1-(tetrahydrofuran-3-yl)-1H-pyrazol-5-yl, 4-chloro-1H-pyrazol-5-yl, 4-chloro-1-(2,2,2-trifluoroethyl)-1H-pyrazol-3-yl.

8. The compound of general formula (I) according to any one of claims 1 to 3 wherein R₇ is selected from: 1,4-dimethyl-1H-imidazol-5-yl, 3-methoxypyridazin-4-yl, 1-cyclobutyl-4-methyl-1H-imidazol-5-yl, 2-trifluoromethylphenyl, 2-difluoromethylphenyl, 4,6-dimethylpyrimidin-5-yl, 4-cyclopropyl-6-(trifluoromethyl)pyrimidin-5-yl, 3-(trifluoromethyl)pyrazin-2-yl.

9. The compound of general formula (I) according to claim 1 being selected from:
- (3-(4-(2-(trifluoromethyl)pyridin-3-yl)benzyl)-1,2,3-oxadiazol-3-ium-5-yl)((2-(trifluoromethyl)pyridin-4-yl)carbamoyl)amide
- (3-(4-(1,4-dimethyl-1H-imidazol-5-yl)benzyl)-1,2,3-oxadiazol-3-ium-5-yl)((2-(trifluoromethyl)pyridin-4-yl)carbamoyl)amide
- (3-(4-(1-cyclopropyl-4-methoxy-1H-pyrazol-5-yl)benzyl)-1,2,3-oxadiazol-3-ium-5-yl)((2-(trifluoromethyl)pyridin-4-yl)carbamoyl)amide
- (3-(4-(4-methoxy-1-methyl-1H-pyrazol-5-yl)benzyl)-1,2,3-oxadiazol-3-ium-5-yl)((2-(trifluoromethyl)pyridin-4-yl)carbamoyl)amide
- (3-(4-(1-cyclopropyl-4-methyl-1H-pyrazol-5-yl)benzyl)-1,2,3-oxadiazol-3-ium-5-yl)((2-(trifluoromethyl)pyridin-4-yl)carbamoyl)amide
- (3-(4-(3-methoxypyridazin-4-yl)benzyl)-1,2,3-oxadiazol-3-ium-5-yl)((2-(trifluoromethyl)pyridin-4-yl)carbamoyl)amide
- (3-(4-(1-cyclobutyl-4-methyl-1H-imidazol-5-yl)benzyl)-1,2,3-oxadiazol-3-ium-5-yl)((2-(trifluoromethyl)pyridin-4-yl)carbamoyl)amide
- (3-(4-(1-cyclobutyl-4-methyl-1H-pyrazol-5-yl)benzyl)-1,2,3-oxadiazol-3-ium-5-yl)((2-(trifluoromethyl)pyridin-4-yl)carbamoyl)amide
- (3-(4-(1-cyclobutyl-4-methoxy-1H-pyrazol-5-yl)benzyl)-1,2,3-oxadiazol-3-ium-5-yl)((2-(trifluoromethyl)pyridin-4-yl)carbamoyl)amide
- (3-(4-(2-(difluoromethyl)pyridin-3-yl)benzyl)-1,2,3-oxadiazol-3-ium-5-yl)((2-(trifluoromethyl)pyridin-4-yl)carbamoyl)amide
- (3-(4-(4-chloro-1-methyl-1H-pyrazol-5-yl)benzyl)-1,2,3-oxadiazol-3-ium-5-yl)((2-(trifluoromethyl)pyridin-4-yl)carbamoyl)amide
- (3-(4-(3-(trifluoromethyl)pyridin-2-yl)benzyl)-1,2,3-oxadiazol-3-ium-5-yl)((2-(trifluoromethyl)pyridin-4-yl)carbamoyl)amide
- (3-(4-(4-chloro-2-(trifluoromethyl)pyridin-3-yl)benzyl)-1,2,3-oxadiazol-3-ium-5-yl)((2-(trifluoromethyl)pyridin-4-yl)carbamoyl)amide
- (3-(4-(4-chloro-1-(oxetan-3-yl)-1H-pyrazol-5-yl)benzyl)-1,2,3-oxadiazol-3-ium-5-yl)((2-(trifluoromethyl)pyridin-4-yl)carbamoyl)amide
- (3-(4-(4-(trifluoromethyl)pyridin-3-yl)benzyl)-1,2,3-oxadiazol-3-ium-5-yl)((2-(trifluoromethyl)pyridin-4-yl)carbamoyl)amide
- (3-(4-(1-methyl-4-(trifluoromethyl)-1H-pyrazol-5-yl)benzyl)-1,2,3-oxadiazol-3-ium-5-yl)((2-(trifluoromethyl)pyridin-4-yl)carbamoyl)amide
- (3-(4-(1-(oxetan-3-yl)-4-(trifluoromethyl)-1H-pyrazol-5-yl)benzyl)-1,2,3-oxadiazol-3-ium-5-yl)((2-(trifluoromethyl)pyridin-4-yl)carbamoyl)amide
- (3-(4-(6-oxo-2-(trifluoromethyl)-1,6-dihydropyridin-3-yl)benzyl)-1,2,3-oxadiazol-3-ium-5-yl)((2-(trifluoromethyl)pyridin-4-yl)carbamoyl)amide
- (3-(4-(2-methoxy-4-(trifluoromethyl)pyridin-3-yl)benzyl)-1,2,3-oxadiazol-3-ium-5-yl)((2-(trifluoromethyl)pyridin-4-yl)carbamoyl)amide
- (3-(4-(4-(trifluoromethyl)pyridin-2-yl)benzyl)-1,2,3-oxadiazol-3-ium-5-yl)((2-(trifluoromethyl)pyridin-4-yl)carbamoyl)amide
- (3-((2'-(trifluoromethyl)-[1,1'-biphenyl]-4-yl)methyl)-1,2,3-oxadiazol-3-ium-5-yl)((2-(trifluoromethyl)pyridin-4-yl)carbamoyl)amide
- (3-(4-(4-fluoro-1-(oxetan-3-yl)-1H-pyrazol-5-yl)benzyl)-1,2,3-oxadiazol-3-ium-5-yl)((2-(trifluoromethyl)pyridin-4-yl)carbamoyl)amide
- (3-((2'-(difluoromethyl)-[1,1'-biphenyl]-4-yl)methyl)-1,2,3-oxadiazol-3 -ium-5-yl)((2-(trifluoromethyl)pyridin-4-yl)carbamoyl)amide
- (3-(4-(2-(difluoromethyl)-4-methylpyridin-3-yl)benzyl)-1,2,3-oxadiazol-3-ium-5-yl)((2-(trifluoromethyl)pyridin-4-yl)carbamoyl)amide
- (3-(4-(4-chloro-1-methyl-1H-pyrazol-5-yl)-3-methylbenzyl)-1,2,3-oxadiazol-3-ium-5-yl)((2-(trifluoromethyl)pyridin-4-yl)carbamoyl)amide
- (3-(4-(4-methyl-1-(tetrahydro-2H-pyran-4-yl)-1H-pyrazol-5-yl)benzyl)-1,2,3-oxadiazol-3-ium-5-yl)((2-(trifluoromethyl)pyridin-4-yl)carbamoyl)amide
- (3-(1-(4-(4-chloro-1-methyl-1H-pyrazol-5-yl)phenyl)cyclopropyl)-1,2,3-oxadiazol-3-ium-5-yl)((2-(trifluoromethyl)pyridin-4-yl)carbamoyl)amide
- (3-(1-(4-(2-(difluoromethyl)pyridin-3-yl)phenyl)cyclopropyl)-1,2,3-oxadiazol-3-ium-5-yl)((2-(trifluoromethyl)pyridin-4-yl)carbamoyl)amide
- (3-(4-(3-(difluoromethyl)pyridin-2-yl)benzyl)-1,2,3-oxadiazol-3-ium-5-yl)((2-(trifluoromethyl)pyridin-4-yl)carbamoyl)amide
- (3-(2-chloro-4-(2-(difluoromethyl)pyridin-3-yl)benzyl)-1,2,3-oxadiazol-3-ium-5-yl)((2-(trifluoromethyl)pyridin-4-yl)carbamoyl)amide
- (3-(3-chloro-4-(4,6-dimethylpyrimidin-5-yl)benzyl)-1,2,3-oxadiazol-3-ium-5-yl)((2-(trifluoromethyl)pyridin-4-yl)carbamoyl)amide
- (3-(1-(4-(2-(difluoromethyl)pyridin-3-yl)phenyl)-2-hydroxyethyl)-1,2,3-oxadiazol-3-ium-5-yl)((2-(trifluoromethyl)pyridin-4-yl)carbamoyl)amide
- (3-(1-(4-(4-chloro-1-methyl-1H-pyrazol-5-yl)phenyl)-2-hydroxyethyl)-1,2,3-oxadiazol-3-ium-5-yl)((2-(trifluoromethyl)pyridin-4-yl)carbamoyl)amide
- (3-(4-(3-(trifluoromethyl)pyridin-4-yl)benzyl)-1,2,3-oxadiazol-3-ium-5-yl)((2-(trifluoromethyl)pyridin-4-yl)carbamoyl)amide
- (3-((6-(4,6-dimethylpyrimidin-5-yl)pyridin-3-yl)methyl)-1,2,3-oxadiazol-3-ium-5-yl)((2-(trifluoromethyl)pyridin-4-yl)carbamoyl)amide
- (3-((6-(4-chloro-1-methyl-1H-pyrazol-5-yl)pyridin-3-yl)methyl)-1,2,3-oxadiazol-3-ium-5-yl)((2-(trifluoromethyl)pyridin-4-yl)carbamoyl)amide
- (3-((2'-(difluoromethyl)-[2,3'-bipyridin]-5-yl)methyl)-1,2,3-oxadiazol-3-ium-5-yl)((2-(trifluoromethyl)pyridin-4-yl)carbamoyl)amide
- (3-(4-(3-hydroxypyridin-2-yl)benzyl)-1,2,3-oxadiazol-3-ium-5-yl)((2-(trifluoromethyl)pyridin-4-yl)carbamoyl)amide
- (3-(4-(4-chloro-1-(2-hydroxy-2-methylpropyl)-1H-pyrazol-5-yl)benzyl)-1,2,3-oxadiazol-3-ium-5-yl)((2-(trifluoromethyl)pyridin-4-yl)carbamoyl)amide
- (3-(4-(4-cyclopropyl-6-(trifluoromethyl)pyrimidin-5-yl)benzyl)-1,2,3-oxadiazol-3-ium-5-yl)((2-(trifluoromethyl)pyridin-4-yl)carbamoyl)amide
- (3-(4-(4-chloro-1-(tetrahydro-2H-pyran-4-yl)-1H-pyrazol-5-yl)benzyl)-1,2,3-oxadiazol-3-ium-5-yl)((2-(trifluoromethyl)pyridin-4-yl)carbamoyl)amide
- (3-(4-(4-chloro-1-(tetrahydrofuran-3-yl)-1H-pyrazol-5-yl)benzyl)-1,2,3-oxadiazol-3-ium-5-yl)((2-(trifluoromethyl)pyridin-4-yl)carbamoyl)amide
- (3-(4-(3-(trifluoromethyl)pyrazin-2-yl)benzyl)-1,2,3-oxadiazol-3-ium-5-yl)((2-(trifluoromethyl)pyridin-4-yl)carbamoyl)amide
- (3-(4-(2-(1,1-difluoro-2-hydroxyethyl)pyridin-3-yl)benzyl)-1,2,3-oxadiazol-3-ium-5-yl)((2-(trifluoromethyl)pyridin-4-yl)carbamoyl)amide
- (3-(4-(4-(difluoromethyl)pyridin-3-yl)benzyl)-1,2,3-oxadiazol-3-ium-5-yl)((2-(trifluoromethyl)pyridin-4-yl)carbamoyl)amide
- (3-(4-(4-chloro-1H-pyrazol-5-yl)benzyl)-1,2,3-oxadiazol-3-ium-5-yl)((2-(trifluoromethyl)pyridin-4-yl)carbamoyl)amide
- (3-(4-(3-(difluoromethyl)pyridin-4-yl)benzyl)-1,2,3-oxadiazol-3-ium-5-yl)((2-(trifluoromethyl)pyridin-4-yl)carbamoyl)amide
- (3-(4-(6-(methoxycarbonyl)-2-methylpyridin-3-yl)benzyl)-1,2,3-oxadiazol-3-ium-5-yl)((2-(trifluoromethyl)pyridin-4-yl)carbamoyl)amide
- (3-(4-(6-carboxy-2-methylpyridin-3-yl)benzyl)-1,2,3-oxadiazol-3-ium-5-yl)((2-(trifluoromethyl)pyridin-4-yl)carbamoyl)amide
- (3-(4-(4-chloro-1-(2,2,2-trifluoroethyl)-1H-pyrazol-3-yl)benzyl)-1,2,3-oxadiazol-3-ium-5-yl)((2-(trifluoromethyl)pyridin-4-yl)carbamoyl)amide
- (3-(4-(2-methyl-6-(methylcarbamoyl)pyridin-3-yl)benzyl)-1,2,3-oxadiazol-3-ium-5-yl)((2-(trifluoromethyl)pyridin-4-yl)carbamoyl)amide
- (3-(4-(3-Methoxypyridazin-4-yl)benzyl)-1,2,3-oxadiazol-3-ium-5-yl)((2-(trifluoromethyl)pyridin-4-yl)carbamoyl)amide
- ((2-cyclopropylpyridin-4-yl)carbamoyl)(3-(4-(4,6-dimethylpyrimidin-5-yl)benzyl)-1,2,3-oxadiazol-3-ium-5-yl)amide
- (3-(4-(4-chloro-1-methyl-1H-pyrazol-5-yl)benzyl)-1,2,3-oxadiazol-3-ium-5-yl)((2-cyclopropylpyridin-4-yl)carbamoyl)amide
- ((2-cyclopropylpyridin-4-yl)carbamoyl)(3-(4-(2-(difluoromethyl)pyridin-3-yl)benzyl)-1,2,3-oxadiazol-3-ium-5-yl)amide
- (3-(4-(4,6-Dimethylpyrimidin-5-yl)benzyl)-1,2,3-oxadiazol-3-ium-5-yl)((2-methoxy-6-(trifluoromethyl)pyridin-4-yl)carbamoyl)amide
- (3-(4-(4,6-Dimethylpyrimidin-5-yl)benzyl)-1,2,3-oxadiazol-3-ium-5-yl)((2-(trifluoromethoxy)pyridin-4-yl)carbamoyl)amide
- (3-(4-(4,6-Dimethylpyrimidin-5-yl)benzyl)-1,2,3-oxadiazol-3-ium-5-yl)((2-methyl-6-(trifluoromethyl)pyridin-4-yl)carbamoyl)amide
- ((2-Chloro-6-(trifluoromethyl)pyridin-4-yl)carbamoyl)(3-(4-(4,6-dimethylpyrimidin-5-yl)benzyl)-1,2,3-oxadiazol-3-ium-5-yl)amide
- (3-(4-(4,6-Dimethylpyrimidin-5-yl)benzyl)-1,2,3-oxadiazol-3-ium-5-yl)((2-(methylamino)-6-(trifluoromethyl)pyridin-4-yl)carbamoyl)amide 10. The compound according to any one of previous claims being an inhibitor of NS2B-NS3 protease of a Flavivirus, preferably an inhibitor of the NS2B-NS3 protease of West Nile and/or Zika virus.

11. The compound according to any one of previous claims for medical use.

12. The compound according to claim 11 for use in treatment and/or prevention of a Flavivirus infection

13. The compound according to claims 11 and 12 for use in the treatment and/or prevention of an infection from West Nile and Zika virus.

14. A pharmaceutical composition comprising the compound according to any one of claims 1 to 9, either alone or in combination with one further therapeutic agent, and at least one pharmaceutically acceptable excipient.

15. The pharmaceutical composition of claim 14 for use in the treatment and/or prevention of a Flavivirus infection, preferably wherein the Flavivirus is selected from West Nile and Zika and virus.
